# EUROPEAN PATENT APPLICATION

(11) **EP 4 803 514 A1**
(43) Date of publication of application: **09.09.2026**
(21) Application number: 24885754.2
(22) Date of filing: 30.10.2024
(51) Int. Cl.: C07D 401/14, A01N 43/56, A01N 43/76, A01N 43/78, A01N 43/653, A01P 13/00, C07D 409/14

(54) **NITROGEN-CONTAINING HETEROCYCLIC COMPOUND HAVING SULFONYL GROUP, AGRICULTURAL/HORTICULTURAL HERBICIDE CONTAINING SAME, AND USE THEREOF**

(30) Priority: 31.10.2023 JP 2023186165
(71) Applicant: Nihon Nohyaku Co., Ltd., Tokyo 104-8386 (JP)
(72) Inventor: INOUE Hayato, Kawachinagano-shi, Osaka 586-0094 (JP); MATSUMOTO Seitaro, Kawachinagano-shi, Osaka 586-0094 (JP)
(74) Representative: Kraus & Lederer PartGmbB
(86) International application number: PCT/JP2024/038670
(87) International publication number: WO 2025/094987

(57) **Abstract**

Problem to be solved is to provide a novel herbicide having both high safety for crops and excellent herbicidal activity against weeds in order to resolve the food crisis that is anticipated to come in the near future due to global population growth.

The problem is solved by a compound represented by the following general formula (I): 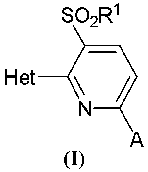 or a salt thereof, an agricultural or horticultural herbicide comprising the compound or the salt thereof as an active ingredient, and a method for using the herbicide.

## Description

### TECHNICAL FIELD

The present invention relates to a nitrogen-containing heterocyclic compound having a sulfonyl group and a salt thereof, an agricultural or horticultural herbicide comprising the compound or the salt thereof as an active ingredient, and a method for using the compound or the salt thereof or the herbicide.

### BACKGROUND ART

Patent literature 1 to 5 describes that certain kinds of arylazole compounds have insecticidal activity. However, the literature does not describe the specific structure of the compound of the present invention, nor does it disclose or suggest any compounds useful as herbicides.

### CITATION LIST

### Patent Literature

Patent Literature 1: WO 2015/144826
Patent Literature 2: WO 2017/104741
Patent Literature 3: WO 2018/052035
Patent Literature 4: WO 2018/116945
Patent Literature 5: WO 2021/085370

### SUMMARY OF INVENTION

### TECHNICAL PROBLEM

A stable and secure food supply is essential to resolve the food crisis that is anticipated to come in the near future due to global population growth. The stable and secure food supply requires economical and efficient elimination or control of weeds that interfere with crop cultivation and harvest. Therefore, it is becoming increasingly important to develop new herbicides and plant growth regulators that can provide solutions to this problem. In order to respond to such social demands, the present invention is intended to provide a novel herbicide having both high safety for crops and excellent herbicidal activity against weeds.

### SOLUTION TO PROBLEM

After conducting extensive research to develop a novel agricultural or horticultural herbicide, the present inventors found that a compound represented by the general formula (I) of the present invention or a salt thereof is useful as an agricultural or horticultural herbicide. Based on this finding, the present inventors completed the present invention.

That is, the present invention includes the following.
[1] A compound represented by the general formula (I): 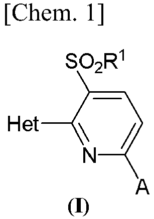 {wherein
   R¹ represents
      (a1) a (C₁-C₆) alkyl group;
      (a2) a halo (C₁-C₆) alkyl group; or
      (a3) an N(R^{2a})R^{2b} group
      (wherein R^{2a} and R^{2b} represent
      (b1) a hydrogen atom,
      (b2) a (C₁-C₆) alkyl group,
      (b3) a (C₂-C₆) alkenyl group,
      (b4) a (C₂-C₆) alkynyl group,
      (b5) a (C₃-C₆) cycloalkyl group,
      (b6) a halo (C₁-C₆) alkyl group,
      (b7) a (C₁-C₇) alkylcarbonyl group,
      (b8) a (C₁-C₆) alkoxycarbonyl group,
      (b9) a halo (C₁-C₇) alkylcarbonyl group, or
      (b10) a halo (C₁-C₆) alkoxycarbonyl group, and
   R^{2a} and R²⁶ may be the same or different), and
   Het represents the formula (Het-1) or the formula (Het-2): 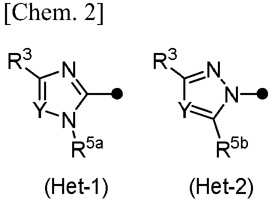 (wherein
   R³ represents
      (c1) a halogen atom;
      (c2) a cyano atom;
      (c3) a (C₁-C₆) alkyl group;
      (c4) a (C₂-C₆) alkenyl group;
      (c5) a (C₂-C₆) alkynyl group;
      (c6) a (C₃-C₆) cycloalkyl group;
      (c7) a (C₁-C₆) alkoxy group;
      (c8) a halo (C₁-C₆) alkyl group;
      (c9) a halo (C₂-C₆) alkenyl group;
      (c10) a halo (C₂-C₆) alkynyl group;
      (c11) a halo (C₃-C₆) cycloalkyl group;
      (c12) a halo (C₁-C₆) alkoxy group;
      (c13) a substituted (C₁-C₆) alkyl group having 1 to 3 substituents, each independently selected from a set S of substituents;
      (c14) a substituted (C₃-C₆) cycloalkyl group having 1 to 3 substituents, each independently selected from a set T of substituents;
      (c15) a (C₁-C₆) alkylsulfanyl group;
      (c16) a (C₁-C₆) alkylsulfinyl group;
      (c17) a (C₁-C₆) alkylsulfonyl group;
      (c18) a (C₁-C₆) alkoxycarbonyl group;
      (c19) a furanyl group;
      (c20) a substituted furanyl group having 1 to 3 substituents on the ring, each independently selected from a set U of substituents;
      (c21) an oxazolyl group;
      (c22) a substituted oxazolyl group having 1 to 3 substituents on the ring, each independently selected from the set U of substituents;
      (c23) a thienyl group;
      (c24) a substituted thienyl group having 1 to 3 substituents on the ring, each independently selected from the set U of substituents;
      (c25) a thiazolyl group;
      (c26) a substituted thiazolyl group having 1 or 2 substituents on the ring, each independently selected from the set U of substituents;
      (c27) a naphthyl group;
      (c28) a substituted naphthyl group having 1 to 7 substituents on the ring, each independently selected from the set U of substituents;
      (c29) a phenyl group;
      (c30) a substituted phenyl group having 1 to 5 substituents on the ring, each independently selected from the set U of substituents;
      (c31) a pyridyl group;
      (c32) a substituted pyridyl group having 1 to 4 substituents on the ring, each independently selected from the set U of substituents;
      (c33) a pyridazinyl group;
      (c34) a substituted pyridazinyl group having 1 to 3 substituents on the ring, each independently selected from the set U of substituents;
      (c35) a pyrimidinyl group;
      (c36) a substituted pyrimidinyl group having 1 to 3 substituents on the ring, each independently selected from the set U of substituents;
      (c37) a pyrazinyl group;
      (c38) a substituted pyrazinyl group having 1 to 3 substituents on the ring, each independently selected from the set U of substituents;
      (c39) a phenyl (C₁-C₆) alkyl group; or
      (c40) a substituted phenyl (C₁-C₆) alkyl group having 1 to 5 substituents on the ring, each independently selected from the set U of substituents,
   Y represents a nitrogen atom or CR⁴(wherein R⁴ represents a hydrogen atom, a halogen atom or a (C₁-C₆) alkyl group),
   R^{5a} represents
      (d1) a hydrogen atom;
      (d2) a (C₁-C₆) alkyl group; or
      (d3) a halo (C₁-C₆) alkyl group,
   R^{5b} represents
      (e1) a hydrogen atom;
      (e2) a halogen atom;
      (e3) a (C₁-C₆) alkyl group; or
      (e4) a halo (C₁-C₆) alkyl group,
   the set S of substituents consists of
      (f1) a cyano group;
      (f2) a (C₃-C₆) cycloalkyl group;
      (f3) a (C₁-C₆) alkoxy group;
      (f4) a (C₁-C₆) alkylsulfanyl group;
      (f5) a (C₁-C₆) alkylsulfinyl group;
      (f6) a (C₁-C₆) alkylsulfonyl group;
      (f7) a halo (C₃-C₆) cycloalkyl group;
      (f8) a halo (C₁-C₆) alkoxy group;
      (f9) a halo (C₁-C₆) alkylsulfanyl group;
      (f10) a halo (C₁-C₆) alkylsulfinyl group; and
      (f11) a halo (C₁-C₆) alkylsulfonyl group,
   the set T of substituents consists of
      (g1) a cyano group;
      (g2) a (C₁-C₆) alkyl group;
      (g3) a (C₃-C₆) cycloalkyl group;
      (g4) a (C₁-C₆) alkoxy group;
      (g5) a (C₁-C₆) alkylsulfanyl group;
      (g6) a (C₁-C₆) alkylsulfinyl group;
      (g7) a (C₁-C₆) alkylsulfonyl group;
      (g8) a halo (C₁-C₆) alkyl group;
      (g9) a halo (C₃-C₆) cycloalkyl group;
      (g10) a halo (C₁-C₆) alkoxy group;
      (g11) a halo (C₁-C₆) alkylsulfanyl group;
      (g12) a halo (C₁-C₆) alkylsulfinyl group;
      (g13) a halo (C₁-C₆) alkylsulfonyl group; and
      (g14) a phenyl group,
   the set U of substituents consists of
      (h1) a halogen atom;
      (h2) a cyano group;
      (h3) a nitro group;
      (h4) an amino group;
      (h5) a hydroxy group;
      (h6) a hydroxy (C₁-C₆) alkyl group;
      (h7) a (C₁-C₆) alkyl group;
      (h8) a (C₂-C₆) alkenyl group;
      (h9) a (C₂-C₆) alkynyl group;
      (h10) a (C₃-C₆) cycloalkyl group;
      (h11) a halo (C₁-C₆) alkyl group;
      (h12) a halo (C₂-C₆) alkenyl group;
      (h13) a halo (C₂-C₆) alkynyl group;
      (h14) a halo (C₃-C₆) cycloalkyl group;
      (h15) a (C₁-C₆) alkoxy group;
      (h16) a (C₁-C₆) alkylsulfanyl group;
      (h17) a (C₁-C₆) alkylsulfinyl group;
      (h18) a (C₁-C₆) alkylsulfonyl group;
      (h19) a halo (C₁-C₆) alkoxy group;
      (h20) a halo (C₁-C₆) alkylsulfanyl group;
      (h21) a halo (C₁-C₆) alkylsulfinyl group;
      (h22) a halo (C₁-C₆) alkylsulfonyl group;
      (h23) an N-((C₁-C₆) alkylcarbonyl) amino group; and
      (h24) an N-((C₁-C₆) alkylsulfonyl) amino group, and
      each solid circle represents the position of binding),
   A represents
      (i1) an oxazolinyl group;
      (i2) a substituted oxazolinyl group having 1 to 4 substituents on the ring, each independently selected from a set V of substituents;
      (i3) an imidazolinyl group;
      (i4) a substituted imidazolinyl group having 1 to 4 substituents on the ring, each independently selected from the set V of substituents;
      (i5) an imidazolidinonyl group;
      (i6) a substituted imidazolidinonyl group having 1 to 4 substituents on the ring, each independently selected from the set V of substituents;
      (i7) a triazolinonyl group;
      (i8) a substituted triazolinonyl group having 1 or 2 substituents on the ring, each independently selected from the set V of substituents;
      (i9) a pyrazolinonyl group;
      (i10) a substituted pyrazolinonyl group having 1 to 3 substituents on the ring, each independently selected from the set V of substituents;
      (i11) a pyrazolyl group;
      (i12) a substituted pyrazolyl group having 1 to 3 substituents on the ring, each independently selected from the set V of substituents;
      (i13) a triazolyl group;
      (i14) a substituted triazolyl group having 1 or 2 substituents on the ring, each independently selected from the set V of substituents;
      (i15) a tetrazolyl group;
      (i16) a substituted tetrazolyl group having 1 substituent on the ring, selected from the set V of substituents;
      (i17) an imidazolyl group;
      (i18) a substituted imidazolyl group having 1 to 3 substituents on the ring, each independently selected from the set V of substituents;
      (i19) a pyrrolyl group;
      (i20) a substituted pyrrolyl group having 1 to 4 substituents on the ring, each independently selected from the set V of substituents;
      (i21) a thiazolyl group;
      (i22) a substituted thiazolyl group having 1 or 2 substituents on the ring, each independently selected from the set V of substituents;
      (i23) an oxazolyl group;
      (i24) a substituted oxazolyl group having 1 or 2 substituents on the ring, each independently selected from the set V of substituents;
      (i25) a tetrahydrofuranyl (C₁-C₆) alkoxy group;
      (i26) a substituted tetrahydrofuranyl (C₁-C₆) alkoxy group having 1 to 4 substituents on the ring, each independently selected from the set V of substituents;
      (i27) a dioxolanyl (C₁-C₆) alkoxy group;
      (i28) a substituted dioxolanyl (C₁-C₆) alkoxy group having 1 to 4 substituents on the ring, each independently selected from the set V of substituents;
      (i29) an isoxazolinyl (C₁-C₆) alkoxy group;
      (i30) a substituted isoxazolinyl (C₁-C₆) alkoxy group having 1 to 4 substituents on the ring, each independently selected from the set V of substituents;
      (i31) a pyrazolyl (C₁-C₆) alkoxy group;
      (i32) a substituted pyrazolyl (C₁-C₆) alkoxy group having 1 to 3 substituents on the ring, each independently selected from the set V of substituents;
      (i33) a triazolyl (C₁-C₆) alkoxy group;
      (i34) a substituted triazolyl (C₁-C₆) alkoxy group having 1 or 2 substituents on the ring, each independently selected from the set V of substituents;
      (i35) a tetrazolyl (C₁-C₆) alkoxy group;
      (i36) a substituted tetrazolyl (C₁-C₆) alkoxy group having 1 substituent on the ring, selected from the set V of substituents;
      (i37) an imidazolyl (C₁-C₆) alkoxy group;
      (i38) a substituted imidazolyl (C₁-C₆) alkoxy group having 1 to 3 substituents on the ring, each independently selected from the set V of substituents;
      (i39) a pyrrolyl (C₁-C₆) alkoxy group;
      (i40) a substituted pyrrolyl (C₁-C₆) alkoxy group having 1 to 4 substituents on the ring, each independently selected from the set V of substituents;
      (i41) a thiazolyl (C₁-C₆) alkoxy group;
      (i42) a substituted thiazolyl (C₁-C₆) alkoxy group having 1 or 2 substituents on the ring, each independently selected from the set V of substituents;
      (i43) an oxazolyl (C₁-C₆) alkoxy group; or
      (i44) a substituted oxazolyl (C₁-C₆) alkoxy group having 1 or 2 substituents on the ring, each independently selected from the set V of substituents, and
   the set V of substituents consists of
      (j1) a halogen atom;
      (j2) a cyano group;
      (j3) a (C₁-C₆) alkyl group;
      (j4) a (C₂-C₆) alkenyl group;
      (j5) a (C₂-C₆) alkynyl group;
      (j6) a (C₃-C₆) cycloalkyl group;
      (j7) a halo (C₁-C₆) alkyl group;
      (j8) a halo (C₂-C₆) alkenyl group;
      (j9) a halo (C₂-C₆) alkynyl group;
      (j10) a halo (C₃-C₆) cycloalkyl group;
      (j11) a (C₁-C₆) alkoxy group;
      (j12) a (C₁-C₆) alkylsulfanyl group;
      (j13) a (C₁-C₆) alkylsulfinyl group;
      (j14) a (C₁-C₆) alkylsulfonyl group;
      (j15) a halo (C₁-C₆) alkoxy group;
      (j16) a halo (C₁-C₆) alkylsulfanyl group;
      (j17) a halo (C₁-C₆) alkylsulfinyl group;
      (j18) a halo (C₁-C₆) alkylsulfonyl group;
      (j19) a (C₁-C₆) alkoxy (C₁-C₆) alkyl group;
      (j20) a (C₁-C₆) alkylsulfanyl (C₁-C₆) alkyl group;
      (j21) a (C₁-C₆) alkoxycarbonyl group;
      (j22) a phenyl group;
      (j23) a substituted phenyl group having 1 to 5 substituents on the ring, each independently selected from the group consisting of a halogen atom, a (C₁-C₆) alkyl, and a halo (C₁-C₆) alkyl;
      (j24) a thienyl group; and
      (j25) a substituted thienyl group having 1 to 3 substituents on the ring, each independently selected from the group consisting of a halogen atom, a (C₁-C₆) alkyl, and a halo (C₁-C₆) alkyl}, or
      a salt thereof.
[2] The compound or the salt thereof according to the above [1], wherein
   R¹ is
      (a1) a (C₁-C₆) alkyl group; or
      (a3') an N(R^{2a})R^{2b} group
      (wherein R^{2a} and R^{2b} represent
      (b1) a hydrogen atom,
      (b2) a (C₁-C₆) alkyl group,
      (b5) a (C₃-C₆) cycloalkyl group,
      (b6) a halo (C₁-C₆) alkyl group,
      (b7) a (C₁-C₇) alkylcarbonyl group, or
      (b8) a (C₁-C₆) alkoxycarbonyl group, and
   R^{2a} and R^{2b} may be the same or different),
   Het is the formula (Het-1) or the formula (Het-2),
   R³ is
      (c1) a halogen atom;
      (c3) a (C₁-C₆) alkyl group;
      (c4) a (C₂-C₆) alkenyl group;
      (c6) a (C₃-C₆) cycloalkyl group;
      (c7) a (C₁-C₆) alkoxy group;
      (c8) a halo (C₁-C₆) alkyl group;
      (c9) a halo (C₂-C₆) alkenyl group;
      (c11) a halo (C₃-C₆) cycloalkyl group;
      (c13) a substituted (C₁-C₆) alkyl group having 1 to 3 substituents, each independently selected from a set S of substituents;
      (c14) a substituted (C₃-C₆) cycloalkyl group having 1 to 3 substituents, each independently selected from a set T of substituents;
      (c15) a (C₁-C₆) alkylsulfanyl group;
      (c16) a (C₁-C₆) alkylsulfinyl group;
      (c17) a (C₁-C₆) alkylsulfonyl group;
      (c18) a (C₁-C₆) alkoxycarbonyl group;
      (c19) a furanyl group;
      (c20) a substituted furanyl group having 1 to 3 substituents on the ring, each independently selected from a set U of substituents;
      (c23) a thienyl group;
      (c24) a substituted thienyl group having 1 to 3 substituents on the ring, each independently selected from the set U of substituents;
      (c25) a thiazolyl group;
      (c26) a substituted thiazolyl group having 1 or 2 substituents on the ring, each independently selected from the set U of substituents;
      (c27) a naphthyl group;
      (c28) a substituted naphthyl group having 1 to 7 substituents on the ring, each independently selected from the set U of substituents;
      (c29) a phenyl group;
      (c30) a substituted phenyl group having 1 to 5 substituents on the ring, each independently selected from the set U of substituents;
      (c31) a pyridyl group;
      (c32) a substituted pyridyl group having 1 to 4 substituents on the ring, each independently selected from the set U of substituents;
      (c39) a phenyl (C₁-C₆) alkyl group; or
      (c40) a substituted phenyl (C₁-C₆) alkyl group having 1 to 5 substituents on the ring, each independently selected from the set U of substituents,
   Y is a nitrogen atom or CR⁴ (wherein R⁴ represents a hydrogen atom),
   R^{5a} is
      (d1) a hydrogen atom; or
      (d2) a (C₁-C₆) alkyl group,
   R^{5b} is
      (e1) a hydrogen atom; or
      (e3) a (C₁-C₆) alkyl group,
   the set S of substituents consists of
      (f1) a cyano group;
      (f2) a (C₃-C₆) cycloalkyl group;
      (f3) a (C₁-C₆) alkoxy group;
      (f4) a (C₁-C₆) alkylsulfanyl group;
      (f5) a (C₁-C₆) alkylsulfinyl group; and
      (f6) a (C₁-C₆) alkylsulfonyl group,
   the set T of substituents consists of
      (g1) a cyano group;
      (g2) a (C₁-C₆) alkyl group;
      (g8) a halo (C₁-C₆) alkyl group; and
      (g14) a phenyl group,
   the set U of substituents consists of
      (h1) a halogen atom;
      (h2) a cyano group;
      (h5) a hydroxy group;
      (h6) a hydroxy (C₁-C₆) alkyl group;
      (h7) a (C₁-C₆) alkyl group;
      (h10) a (C₃-C₆) cycloalkyl group;
      (h11) a halo (C₁-C₆) alkyl group;
      (h14) a halo (C₃-C₆) cycloalkyl group;
      (h15) a (C₁-C₆) alkoxy group;
      (h16) a (C₁-C₆) alkylsulfanyl group;
      (h17) a (C₁-C₆) alkylsulfinyl group;
      (h18) a (C₁-C₆) alkylsulfonyl group;
      (h19) a halo (C₁-C₆) alkoxy group;
      (h20) a halo (C₁-C₆) alkylsulfanyl group;
      (h21) a halo (C₁-C₆) alkylsulfinyl group;
      (h22) a halo (C₁-C₆) alkylsulfonyl group; and
      (h23) an N-((C₁-C₆) alkylcarbonyl) amino group,
   A is
      (i1) an oxazolinyl group;
      (i2) a substituted oxazolinyl group having 1 to 4 substituents on the ring, each independently selected from a set V of substituents;
      (i3) an imidazolinyl group;
      (i4) a substituted imidazolinyl group having 1 to 4 substituents on the ring, each independently selected from the set V of substituents;
      (i5) an imidazolidinonyl group;
      (i6) a substituted imidazolidinonyl group having 1 to 4 substituents on the ring, each independently selected from the set V of substituents;
      (i7) a triazolinonyl group;
      (i8) a substituted triazolinonyl group having 1 or 2 substituents on the ring, each independently selected from the set V of substituents;
      (i9) a pyrazolinonyl group;
      (i10) a substituted pyrazolinonyl group having 1 to 3 substituents on the ring, each independently selected from the set V of substituents;
      (i11) a pyrazolyl group;
      (i12) a substituted pyrazolyl group having 1 to 3 substituents on the ring, each independently selected from the set V of substituents;
      (i13) a triazolyl group;
      (i14) a substituted triazolyl group having 1 or 2 substituents on the ring, each independently selected from the set V of substituents;
      (i21) a thiazolyl group;
      (i22) a substituted thiazolyl group having 1 or 2 substituents on the ring, each independently selected from the set V of substituents;
      (i23) an oxazolyl group;
      (i24) a substituted oxazolyl group having 1 or 2 substituents on the ring, each independently selected from the set V of substituents;
      (i25) a tetrahydrofuranyl (C₁-C₆) alkoxy group;
      (i26) a substituted tetrahydrofuranyl (C₁-C₆) alkoxy group having 1 to 4 substituents on the ring, each independently selected from the set V of substituents;
      (i27) a dioxolanyl (C₁-C₆) alkoxy group;
      (i28) a substituted dioxolanyl (C₁-C₆) alkoxy group having 1 to 4 substituents on the ring, each independently selected from the set V of substituents;
      (i29) an isoxazolinyl (C₁-C₆) alkoxy group;
      (i30) a substituted isoxazolinyl (C₁-C₆) alkoxy group having 1 to 4 substituents on the ring, each independently selected from the set V of substituents;
      (i31) a pyrazolyl (C₁-C₆) alkoxy group;
      (i32) a substituted pyrazolyl (C₁-C₆) alkoxy group having 1 to 3 substituents on the ring, each independently selected from the set V of substituents;
      (i33) a triazolyl (C₁-C₆) alkoxy group;
      (i34) a substituted triazolyl (C₁-C₆) alkoxy group having 1 or 2 substituents on the ring, each independently selected from the set V of substituents;
      (i41) a thiazolyl (C₁-C₆) alkoxy group;
      (i42) a substituted thiazolyl (C₁-C₆) alkoxy group having 1 or 2 substituents on the ring, each independently selected from the set V of substituents;
      (i43) an oxazolyl (C₁-C₆) alkoxy group; or
      (i44) a substituted oxazolyl (C₁-C₆) alkoxy group having 1 or 2 substituents on the ring, each independently selected from the set V of substituents, and
   the set V of substituents consists of
      (j1) a halogen atom;
      (j2) a cyano group;
      (j3) a (C₁-C₆) alkyl group;
      (j4) a (C₂-C₆) alkenyl group;
      (j6) a (C₃-C₆) cycloalkyl group;
      (j7) a halo (C₁-C₆) alkyl group;
      (j8) a halo (C₂-C₆) alkenyl group;
      (j10) a halo (C₃-C₆) cycloalkyl group;
      (j11) a (C₁-C₆) alkoxy group;
      (j12) a (C₁-C₆) alkylsulfanyl group;
      (j13) a (C₁-C₆) alkylsulfinyl group;
      (j14) a (C₁-C₆) alkylsulfonyl group;
      (j15) a halo (C₁-C₆) alkoxy group;
      (j16) a halo (C₁-C₆) alkylsulfanyl group;
      (j20) a (C₁-C₆) alkylsulfanyl (C₁-C₆) alkyl group;
      (j21) a (C₁-C₆) alkoxycarbonyl group;
      (j22) a phenyl group;
      (j23) a substituted phenyl group having 1 to 5 substituents on the ring, each independently selected from the group consisting of a halogen atom, a (C₁-C₆) alkyl, and a halo (C₁-C₆) alkyl;
      (j24) a thienyl group; and
      (j25) a substituted thienyl group having 1 to 3 substituents on the ring, each independently selected from the group consisting of a halogen atom, a (C₁-C₆) alkyl, and a halo (C₁-C₆) alkyl.
[3] The compound or the salt thereof according to the above [1], wherein
   R¹ is
      (a1) a (C₁-C₆) alkyl group; or
      (a3'') an N(R^{2a})R^{2b} group
      (wherein R^{2a} and R^{2b} represent
      (b1) a hydrogen atom,
      (b2) a (C₁-C₆) alkyl group,
      (b7) a (C₁-C₇) alkylcarbonyl group, or
      (b8) a (C₁-C₆) alkoxycarbonyl group, and
   R^{2a} and R^{2b} may be the same or different),
   Het is the formula (Het-1) or the formula (Het-2),
   R³ is
      (c1) a halogen atom;
      (c3) a (C₁-C₆) alkyl group;
      (c4) a (C₂-C₆) alkenyl group;
      (c6) a (C₃-C₆) cycloalkyl group;
      (c8) a halo (C₁-C₆) alkyl group;
      (c13) a substituted (C₁-C₆) alkyl group having 1 to 3 substituents, each independently selected from a set S of substituents;
      (c14) a substituted (C₃-C₆) cycloalkyl group having 1 to 3 substituents, each independently selected from a set T of substituents;
      (c18) a (C₁-C₆) alkoxycarbonyl group;
      (c19) a furanyl group;
      (c23) a thienyl group;
      (c25) a thiazolyl group;
      (c26) a substituted thiazolyl group having 1 or 2 substituents on the ring, each independently selected from a set U of substituents;
      (c27) a naphthyl group;
      (c29) a phenyl group;
      (c30) a substituted phenyl group having 1 to 5 substituents on the ring, each independently selected from the set U of substituents;
      (c31) a pyridyl group;
      (c32) a substituted pyridyl group having 1 to 4 substituents on the ring, each independently selected from the set U of substituents; or
      (c40) a substituted phenyl (C₁-C₆) alkyl group having 1 to 5 substituents on the ring, each independently selected from the set U of substituents,
   Y is a nitrogen atom or CR⁴ (wherein R⁴ represents a hydrogen atom),
   R^{5a} is
      (d2) a (C₁-C₆) alkyl group,
   R^{5b} is
      (e1) a hydrogen atom; or
      (e3) a (C₁-C₆) alkyl group,
   the set S of substituents is
      (f3) a (C₁-C₆) alkoxy group,
   the set T of substituents is
      (g14) a phenyl group,
   the set U of substituents consists of
      (h1) a halogen atom;
      (h5) a hydroxy group;
      (h6) a hydroxy (C₁-C₆) alkyl group;
      (h7) a (C₁-C₆) alkyl group;
      (h11) a halo (C₁-C₆) alkyl group; and
      (h15) a (C₁-C₆) alkoxy group,
   A is
      (i2) a substituted oxazolinyl group having 1 to 4 substituents on the ring, each independently selected from a set V of substituents;
      (i4) a substituted imidazolinyl group having 1 to 4 substituents on the ring, each independently selected from the set V of substituents;
      (i6) a substituted imidazolidinonyl group having 1 to 4 substituents on the ring, each independently selected from the set V of substituents;
      (i8) a substituted triazolinonyl group having 1 or 2 substituents on the ring, each independently selected from the set V of substituents;
      (i10) a substituted pyrazolinonyl group having 1 to 3 substituents on the ring, each independently selected from the set V of substituents;
      (i11) a pyrazolyl group;
      (i12) a substituted pyrazolyl group having 1 to 3 substituents on the ring, each independently selected from the set V of substituents;
      (i13) a triazolyl group;
      (i14) a substituted triazolyl group having 1 or 2 substituents on the ring, each independently selected from the set V of substituents;
      (i22) a substituted thiazolyl group having 1 or 2 substituents on the ring, each independently selected from the set V of substituents;
      (i26) a substituted tetrahydrofuranyl (C₁-C₆) alkoxy group having 1 to 4 substituents on the ring, each independently selected from the set V of substituents;
      (i28) a substituted dioxolanyl (C₁-C₆) alkoxy group having 1 to 4 substituents on the ring, each independently selected from the set V of substituents;
      (i30) a substituted isoxazolinyl (C₁-C₆) alkoxy group having 1 to 4 substituents on the ring, each independently selected from the set V of substituents;
      (i31) a pyrazolyl (C₁-C₆) alkoxy group;
      (i32) a substituted pyrazolyl (C₁-C₆) alkoxy group having 1 to 3 substituents on the ring, each independently selected from the set V of substituents;
      (i34) a substituted triazolyl (C₁-C₆) alkoxy group having 1 or 2 substituents on the ring, each independently selected from the set V of substituents; or
      (i42) a substituted thiazolyl (C₁-C₆) alkoxy group having 1 or 2 substituents on the ring, each independently selected from the set V of substituents, and
   the set V of substituents consists of
      (j1) a halogen atom;
      (j3) a (C₁-C₆) alkyl group;
      (j4) a (C₂-C₆) alkenyl group;
      (j6) a (C₃-C₆) cycloalkyl group;
      (j7) a halo (C₁-C₆) alkyl group;
      (j11) a (C₁-C₆) alkoxy group;
      (j12) a (C₁-C₆) alkylsulfanyl group;
      (j20) a (C₁-C₆) alkylsulfanyl (C₁-C₆) alkyl group;
      (j21) a (C₁-C₆) alkoxycarbonyl group;
      (j23') a substituted phenyl group having 1 to 5 substituents on the ring, selected independently from a halogen atom; and
      (j24) a thienyl group.
[4] An agricultural or horticultural herbicide comprising the compound or the salt thereof according to any one of the above [1] to [3] as an active ingredient.
[5] A method for using an agricultural or horticultural herbicide, comprising treating weeds, soil, paddy fields, or growing media with an effective amount of the agricultural or horticultural herbicide according to the above [4].
[6] A method for controlling weeds, comprising treating weeds, soil, paddy fields, or growing media with an effective amount of the agricultural or horticultural herbicide according to the above [4].

### ADVANTAGEOUS EFFECTS OF INVENTION

The compound represented by the general formula (I) of the present invention or a salt thereof is a highly effective agricultural or horticultural herbicide.

### DESCRIPTION OF EMBODIMENTS

In the definitions of the general formula (I) representing the compound of the present invention, "halo" means "halogen atom" and is usually exemplified by a chlorine atom, a bromine atom, an iodine atom, or a fluorine atom.

The "(C₁-C₆) alkyl group" refers to a straight-chain or branched-chain alkyl group of 1 to 6 carbon atoms, for example, a methyl group, an ethyl group, a n-propyl group, an isopropyl group, a n-butyl group, an isobutyl group, a sec-butyl group, a tert-butyl group, a n-pentyl group, an isopentyl group, a tert-pentyl group, a neopentyl group, a 2,3-dimethylpropyl group, an 1-ethylpropyl group, a 1-methylbutyl group, a 2-methylbutyl group, a n-hexyl group, an isohexyl group, a 2-hexyl group, a 3-hexyl group, a 2-methylpentyl group, a 3-methylpentyl group, a 1,1,2-trimethyl propyl group, a 3,3-dimethylbutyl group or the like.

The "(C₂-C₆) alkenyl group" refers to a straight-chain or branched-chain alkenyl group of 2 to 6 carbon atoms, for example, a vinyl group, an allyl group, an isopropenyl group, a 1-butenyl group, a 2-butenyl group, a 2-methyl-2-propenyl group, a 1-methyl-2-propenyl group, a 2-methyl-1-propenyl group, a pentenyl group, a 1-hexenyl group, a 3,3-dimethyl-1-butenyl group or the like. The "(C₂-C₆) alkynyl group" refers to a straight-chain or branched-chain alkynyl group of 2 to 6 carbon atoms, for example, an ethynyl group, a 1-propynyl group, a 2-propynyl group, a 1-butynyl group, a 2-butynyl group, a 3-butynyl group, a 3-methyl-1-propynyl group, a 2-methyl-3-propynyl group, a pentynyl group, a 1-hexynyl group, a 3-methyl-1-butynyl group, a 3,3-dimethyl-1-butynyl group or the like.

The "(C₃-C₆) cycloalkyl group" refers to a cyclic alkyl group of 3 to 6 carbon atoms, for example, a cyclopropyl group, a cyclobutyl group, a cyclopentyl group, a cyclohexyl group or the like.

The "(C₁-C₆) alkoxy group" refers to a straight-chain or branched-chain alkoxy group of 1 to 6 carbon atoms, for example, a methoxy group, an ethoxy group, a n-propoxy group, an isopropoxy group, a n-butoxy group, a sec-butoxy group, a tert-butoxy group, a n-pentyloxy group, an isopentyloxy group, a tert-pentyloxy group, a neopentyloxy group, a 2,3-dimethylpropyloxy group, an 1-ethylpropyloxy group, a 1-methylbutyloxy group, a n-hexyloxy group, an isohexyloxy group, a 1,1,2-trimethylpropyloxy group or the like.

The "(C₁-C₆) alkylsulfanyl group" refers to a straight-chain or branched-chain alkylsulfanyl group of 1 to 6 carbon atoms, for example, a methylthio group, an ethylthio group, a n-propylthio group, an isopropylthio group, a n-butylthio group, a sec-butylthio group, a tert-butylthio group, a n-pentylthio group, an isopentylthio group, a tert-pentylthio group, a neopentylthio group, a 2,3-dimethylpropylthio group, an 1-ethylpropylthio group, a 1-methylbutylthio group, a n-hexylthio group, an isohexylthio group, a 1,1,2-trimethylpropylthio group or the like.

The "(C₁-C₆) alkylsulfinyl group" refers to a straight-chain or branched-chain alkylsulfinyl group of 1 to 6 carbon atoms, for example, a methylsulfinyl group, an ethylsulfinyl group, a n-propylsulfinyl group, an isopropylsulfinyl group, a n-butylsulfinyl group, a sec-butylsulfinyl group, a tert-butylsulfinyl group, a n-pentylsulfinyl group, an isopentylsulfinyl group, a tert-pentylsulfinyl group, a neopentylsulfinyl group, a 2,3-dimethylpropylsulfinyl group, an 1-ethylpropylsulfinyl group, a 1-methylbutylsulfinyl group, a n-hexylsulfinyl group, an isohexylsulfinyl group, a 1,1,2-trimethylpropylsulfinyl group or the like.

The "(C₁-C₆) alkylsulfonyl group" refers to a straight-chain or branched-chain alkylsulfonyl group of 1 to 6 carbon atoms, for example, a methylsulfonyl group, an ethylsulfonyl group, a n-propylsulfonyl group, an isopropylsulfonyl group, a n-butylsulfonyl group, a sec-butylsulfonyl group, a tert-butylsulfonyl group, a n-pentylsulfonyl group, an isopentylsulfonyl group, a tert-pentylsulfonyl group, a neopentylsulfonyl group, a 2,3-dimethylpropylsulfonyl group, an 1-ethylpropylsulfonyl group, a 1-methylbutylsulfonyl group, a n-hexylsulfonyl group, an isohexylsulfonyl group, a 1,1,2-trimethylpropylsulfonyl group or the like.

The "(C₁-C₇) alkylcarbonyl group" refers to an alkylcarbonyl group of 2 to 8 carbon atoms, for example, an alkylcarbonyl group in which the alkyl group is a (C₁-C₇) alkyl group, such as an acetyl group, a propanoyl group, a butanoyl group, a 2-methylpropanoyl group, a pentanoyl group, a 2-methylbutanoyl group, a 3-methylbutanoyl group, a pivaloyl group, a hexanoyl group, a heptanoyl group, an octanoyl group or the like.

The "(C₁-C₆) alkoxycarbonyl group" refers to an alkoxycarbonyl group of 2 to 7 carbon atoms, for example, an alkoxycarbonyl group in which the alkoxy group is a (C₁-C₆) alkoxy group as defined above, such as a methoxycarbonyl group, an ethoxycarbonyl group, a n-propoxycarbonyl group, an isopropoxycarbonyl group, a n-butoxycarbonyl group, an isobutoxycarbonyl group, a sec-butoxycarbonyl group, a tert-butoxycarbonyl group, a pentyloxycarbonyl group, or the like.

The above-mentioned "(C₁-C₆) alkyl group", "(C₂-C₆) alkenyl group", "(C₂-C₆) alkynyl group", "(C₁-C₆) alkoxy group", "(C₁-C₆) alkylsulfanyl group", "(C₁-C₆) alkylsulfinyl group", "(C₁-C₆) alkylsulfonyl group", "(C₃-C₆) cycloalkyl group", "(C₁-C₆) alkylcarbonyl group", "(C₁-C₆) alkoxycarbonyl group", etc. may be substituted with one or more halogen atoms at a substitutable position(s), and in the case where any of the above-listed groups is substituted with two or more halogen atoms, the halogen atoms may be the same or different.

The above-mentioned groups substituted with one or more halogen atoms are expressed as a "halo (C₁-C₆) alkyl group", a "halo (C₂-C₆) alkenyl group", a "halo (C₂-C₆) alkynyl group", a "halo (C₁-C₆) alkoxy group", a "halo (C₁-C₆) alkylsulfanyl group", a "halo (C₁-C₆) alkylsulfinyl group", a "halo (C₁-C₆) alkylsulfonyl group", a "halo (C₃-C₆) cycloalkyl group", a "halo (C₁-C₆) alkylcarbonyl group", a "halo (C₁-C₆) alkoxycarbonyl group", etc.

In the present invention, the expressions "(C₁-C₆)", "(C₂-C₆)", "(C₃-C₆)", etc. each represent the range of the number of carbon atoms in each group. The same definition holds true for groups in which two or more of the above-mentioned groups are coupled together, and for example, the "(C₁-C₆) alkoxy (C₁-C₆) alkyl group" means that a straight-chain or branched-chain alkoxy group of 1 to 6 carbon atoms is bound to a straight-chain or branched-chain alkyl group of 1 to 6 carbon atoms.

Examples of the salt of the compound represented by the general formula (I) of the present invention include inorganic acid salts, such as hydrochlorides, sulfates, nitrates and phosphates; organic acid salts, such as acetates, fumarates, maleates, oxalates, methanesulfonates, benzenesulfonates and p-toluenesulfonates; and salts with an inorganic or organic base such as a sodium ion, a potassium ion, a calcium ion and a trimethylammonium ion.

The compound represented by the general formula (I) of the present invention and a salt thereof can have one or more chiral centers in the structural formula and can exist as two or more kinds of optical isomers or diastereomers. All the optical isomers and mixtures of the isomers at any ratio are also included in the present invention. Further, the compound represented by the general formula (I) of the present invention and a salt thereof can exist as two kinds of geometric isomers due to a carbon-carbon double bond or a carbon-nitrogen double bond in the structural formula. All the geometric isomers and mixtures of the isomers at any ratio are also included in the present invention. Further, the compound represented by the general formula (I) of the present invention and a salt thereof can also exist as different tautomers. All of the tautomers and mixtures of the tautomers at any ratio are also included in the present invention.

Preferable embodiments of the compound represented by the general formula (I) of the present invention are shown below.

R¹ is
preferably (a1) a (C₁-C₆) alkyl group; or (a3') an N(R^{2a})R^{2b} group (wherein R^{2a} and R^{2b} represent (b1) a hydrogen atom, (b2) a (C₁-C₆) alkyl group, (b5) a (C₃-C₆) cycloalkyl group, (b6) a halo (C₁-C₆) alkyl group, (b7) a (C₁-C₇) alkylcarbonyl group, or (b8) a (C₁-C₆) alkoxycarbonyl group, and R^{2a} and R^{2b} may be the same or different), and more preferably (a1) a (C₁-C₆) alkyl group; or (a3") an N(R^{2a})R^{2b} group (wherein R^{2a} and R^{2b} represent (b1) a hydrogen atom, (b2) a (C₁-C₆) alkyl group, (b7) a (C₁-C₇) alkylcarbonyl group, or (b8) a (C₁-C₆) alkoxycarbonyl group, and R^{2a} and R^{2b} may be the same or different).

Het is preferably the formula (Het-1) or the formula (Het-2).

R³ is
preferably (c1) a halogen atom, (c3) a (C₁-C₆) alkyl group, (c4) a (C₂-C₆) alkenyl group, (c6) a (C₃-C₆) cycloalkyl group, (c7) a (C₁-C₆) alkoxy group, (c8) a halo (C₁-C₆) alkyl group, (c9) a halo (C₂-C₆) alkenyl group, (c11) a halo (C₃-C₆) cycloalkyl group, (c13) a substituted (C₁-C₆) alkyl group having 1 to 3 substituents, each independently selected from a set S of substituents, (c14) a substituted (C₃-C₆) cycloalkyl group having 1 to 3 substituents, each independently selected from a set T of substituents, (c15) a (C₁-C₆) alkylsulfanyl group, (c16) a (C₁-C₆) alkylsulfinyl group, (c17) a (C₁-C₆) alkylsulfonyl group, (c18) a (C₁-C₆) alkoxycarbonyl group, (c19) a furanyl group, (c20) a substituted furanyl group having 1 to 3 substituents on the ring, each independently selected from a set U of substituents, (c23) a thienyl group, (c24) a substituted thienyl group having 1 to 3 substituents on the ring, each independently selected from the set U of substituents, (c25) a thiazolyl group, (c26) a substituted thiazolyl group having 1 or 2 substituents on the ring, each independently selected from the set U of substituents, (c27) a naphthyl group, (c28) a substituted naphthyl group having 1 to 7 substituents on the ring, each independently selected from the set U of substituents, (c29) a phenyl group, (c30) a substituted phenyl group having 1 to 5 substituents on the ring, each independently selected from the set U of substituents, (c31) a pyridyl group, (c32) a substituted pyridyl group having 1 to 4 substituents on the ring, each independently selected from the set U of substituents, (c39) a phenyl (C₁-C₆) alkyl group, or (c40) a substituted phenyl (C₁-C₆) alkyl group having 1 to 5 substituents on the ring, each independently selected from the set U of substituents, and
more preferably (c1) a halogen atom, (c3) a (C₁-C₆) alkyl group, (c4) a (C₂-C₆) alkenyl group, (c6) a (C₃-C₆) cycloalkyl group, (c8) a halo (C₁-C₆) alkyl group, (c13) a substituted (C₁-C₆) alkyl group having 1 to 3 substituents, each independently selected from the set S of substituents, (c14) a substituted (C₃-C₆) cycloalkyl group having 1 to 3 substituents, each independently selected from the set T of substituents, (c18) a (C₁-C₆) alkoxycarbonyl group, (c19) a furanyl group, (c23) a thienyl group, (c25) a thiazolyl group, (c26) a substituted thiazolyl group having 1 or 2 substituents on the ring, each independently selected from the set U of substituents, (c27) a naphthyl group, (c29) a phenyl group, (c30) a substituted phenyl group having 1 to 5 substituents on the ring, each independently selected from the set U of substituents, (c31) a pyridyl group, (c32) a substituted pyridyl group having 1 to 4 substituents on the ring, each independently selected from the set U of substituents, or (c40) a substituted phenyl (C₁-C₆) alkyl group having 1 to 5 substituents on the ring, each independently selected from the set U of substituents.

Y is preferably a nitrogen atom or CH.

R^{5a} is
preferably (d1) a hydrogen atom or (d2) a (C₁-C₆) alkyl group, and
more preferably (d2) a (C₁-C₆) alkyl group.

R^{5b} is
preferably (e1) a hydrogen atom or (e3) a (C₁-C₆) alkyl group.

The set S of substituents
preferably consists of (f1) a cyano group, (f2) a (C₃-C₆) cycloalkyl group, (f3) a (C₁-C₆) alkoxy group, (f4) a (C₁-C₆) alkylsulfanyl group, (f5) a (C₁-C₆) alkylsulfinyl group, and (f6) a (C₁-C₆) alkylsulfonyl group, and
is more preferably (f3) a (C₁-C₆) alkoxy group.

The set T of substituents
preferably consists of (g1) a cyano group, (g2) a (C₁-C₆) alkyl group, (g8) a halo (C₁-C₆) alkyl group, and (g14) a phenyl group, and
is more preferably (g14) a phenyl group.

The set U of substituents
preferably consists of (h1) a halogen atom, (h2) a cyano group, (h5) a hydroxy group, (h6) a hydroxy (C₁-C₆) alkyl group, (h7) a (C₁-C₆) alkyl group, (h10) a (C₃-C₆) cycloalkyl group, (h11) a halo (C₁-C₆) alkyl group, (h14) a halo (C₃-C₆) cycloalkyl group, (h15) a (C₁-C₆) alkoxy group, (h16) a (C₁-C₆) alkylsulfanyl group, (h17) a (C₁-C₆) alkylsulfinyl group, (h18) a (C₁-C₆) alkylsulfonyl group, (h19) a halo (C₁-C₆) alkoxy group, (h20) a halo (C₁-C₆) alkylsulfanyl group, (h21) a halo (C₁-C₆) alkylsulfinyl group, (h22) a halo (C₁-C₆) alkylsulfonyl group, and (h23) an N-((C₁-C₆) alkylcarbonyl) amino group, and
more preferably consists of (h1) a halogen atom, (h5) a hydroxy group, (h6) a hydroxy (C₁-C₆) alkyl group, (h7) a (C₁-C₆) alkyl group, (h11) a halo (C₁-C₆) alkyl group, and (h15) a (C₁-C₆) alkoxy group.

A is
preferably (i1) an oxazolinyl group, (i2) a substituted oxazolinyl group having 1 to 4 substituents on the ring, each independently selected from a set V of substituents, (i3) an imidazolinyl group, (i4) a substituted imidazolinyl group having 1 to 4 substituents on the ring, each independently selected from the set V of substituents, (i5) an imidazolidinonyl group, (i6) a substituted imidazolidinonyl group having 1 to 4 substituents on the ring, each independently selected from the set V of substituents, (i7) a triazolinonyl group, (i8) a substituted triazolinonyl group having 1 or 2 substituents on the ring, each independently selected from the set V of substituents, (i9) a pyrazolinonyl group, (i10) a substituted pyrazolinonyl group having 1 to 3 substituents on the ring, each independently selected from the set V of substituents, (i11) a pyrazolyl group, (i12) a substituted pyrazolyl group having 1 to 3 substituents on the ring, each independently selected from the set V of substituents, (i13) a triazolyl group, (i14) a substituted triazolyl group having 1 or 2 substituents on the ring, each independently selected from the set V of substituents, (i21) a thiazolyl group, (i22) a substituted thiazolyl group having 1 or 2 substituents on the ring, each independently selected from the set V of substituents, (i23) an oxazolyl group, (i24) a substituted oxazolyl group having 1 or 2 substituents on the ring, each independently selected from the set V of substituents, (i25) a tetrahydrofuranyl (C₁-C₆) alkoxy group, (i26) a substituted tetrahydrofuranyl (C₁-C₆) alkoxy group having 1 to 4 substituents on the ring, each independently selected from the set V of substituents, (i27) a dioxolanyl (C₁-C₆) alkoxy group, (i28) a substituted dioxolanyl (C₁-C₆) alkoxy group having 1 to 4 substituents on the ring, each independently selected from the set V of substituents, (i29) an isoxazolinyl (C₁-C₆) alkoxy group, (i30) a substituted isoxazolinyl (C₁-C₆) alkoxy group having 1 to 4 substituents on the ring, each independently selected from the set V of substituents, (i31) a pyrazolyl (C₁-C₆) alkoxy group, (i32) a substituted pyrazolyl (C₁-C₆) alkoxy group having 1 to 3 substituents on the ring, each independently selected from the set V of substituents, (i33) a triazolyl (C₁-C₆) alkoxy group, (i34) a substituted triazolyl (C₁-C₆) alkoxy group having 1 or 2 substituents on the ring, each independently selected from the set V of substituents, (i41) a thiazolyl (C₁-C₆) alkoxy group, (i42) a substituted thiazolyl (C₁-C₆) alkoxy group having 1 or 2 substituents on the ring, each independently selected from the set V of substituents, (i43) an oxazolyl (C₁-C₆) alkoxy group, or (i44) a substituted oxazolyl (C₁-C₆) alkoxy group having 1 or 2 substituents on the ring, each independently selected from the set V of substituents, and more preferably (i2) a substituted oxazolinyl group having 1 to 4 substituents on the ring, each independently selected from the set V of substituents, (i4) a substituted imidazolinyl group having 1 to 4 substituents on the ring, each independently selected from the set V of substituents, (i6) a substituted imidazolidinonyl group having 1 to 4 substituents on the ring, each independently selected from the set V of substituents, (i8) a substituted triazolinonyl group having 1 or 2 substituents on the ring, each independently selected from the set V of substituents, (i10) a substituted pyrazolinonyl group having 1 to 3 substituents on the ring, each independently selected from the set V of substituents, (i11) a pyrazolyl group, (i12) a substituted pyrazolyl group having 1 to 3 substituents on the ring, each independently selected from the set V of substituents, (i13) a triazolyl group, (i14) a substituted triazolyl group having 1 or 2 substituents on the ring, each independently selected from the set V of substituents, (i22) a substituted thiazolyl group having 1 or 2 substituents on the ring, each independently selected from the set V of substituents, (i26) a substituted tetrahydrofuranyl (C₁-C₆) alkoxy group having 1 to 4 substituents on the ring, each independently selected from the set V of substituents, (i28) a substituted dioxolanyl (C₁-C₆) alkoxy group having 1 to 4 substituents on the ring, each independently selected from the set V of substituents, (i30) a substituted isoxazolinyl (C₁-C₆) alkoxy group having 1 to 4 substituents on the ring, each independently selected from the set V of substituents, (i31) a pyrazolyl (C₁-C₆) alkoxy group, (i32) a substituted pyrazolyl (C₁-C₆) alkoxy group having 1 to 3 substituents on the ring, each independently selected from the set V of substituents, (i34) a substituted triazolyl (C₁-C₆) alkoxy group having 1 or 2 substituents on the ring, each independently selected from the set V of substituents, or (i42) a substituted thiazolyl (C₁-C₆) alkoxy group having 1 or 2 substituents on the ring, each independently selected from the set V of substituents.

The set V of substituents preferably consists of (j 1) a halogen atom, (j2) a cyano group, (j3) a (C₁-C₆) alkyl group, (j4) a (C₂-C₆) alkenyl group, (j6) a (C₃-C₆) cycloalkyl group, (j7) a halo (C₁-C₆) alkyl group, (j8) a halo (C₂-C₆) alkenyl group, (j10) a halo (C₃-C₆) cycloalkyl group, (j11) a (C₁-C₆) alkoxy group, (j12) a (C₁-C₆) alkylsulfanyl group, (j13) a (C₁-C₆) alkylsulfinyl group, (j14) a (C₁-C₆) alkylsulfonyl group, (j15) a halo (C₁-C₆) alkoxy group, (j16) a halo (C₁-C₆) alkylsulfanyl group, (j20) a (C₁-C₆) alkylsulfanyl (C₁-C₆) alkyl group, (j21) a (C₁-C₆) alkoxycarbonyl group, (j22) a phenyl group, (j23) a substituted phenyl group having 1 to 5 substituents on the ring, each independently selected from the group consisting of a halogen atom, a (C₁-C₆) alkyl, and a halo (C₁-C₆) alkyl, (j24) a thienyl group, and (j25) a substituted thienyl group having 1 to 3 substituents on the ring, each independently selected from the group consisting of a halogen atom, a (C₁-C₆) alkyl, and a halo (C₁-C₆) alkyl, and
more preferably consists of (j1) a halogen atom, (j3) a (C₁-C₆) alkyl group, (j4) a (C₂-C₆) alkenyl group, (j6) a (C₃-C₆) cycloalkyl group, (j7) a halo (C₁-C₆) alkyl group, (j11) a (C₁-C₆) alkoxy group, (j12) a (C₁-C₆) alkylsulfanyl group, (j20) a (C₁-C₆) alkylsulfanyl (C₁-C₆) alkyl group, (j21) a (C₁-C₆) alkoxycarbonyl group, (j23') a substituted phenyl group having 1 to 5 substituents on the ring, selected independently from a halogen atom, and (j24) a thienyl group.

The compounds of the present invention can be produced according to, for example, the production methods described below, which are non-limiting examples.

### Production Method 1

The compound represented by the general formula (I) of the present invention can be produced from the compound represented by the general formula (II-1) through the step [a-1] described below.

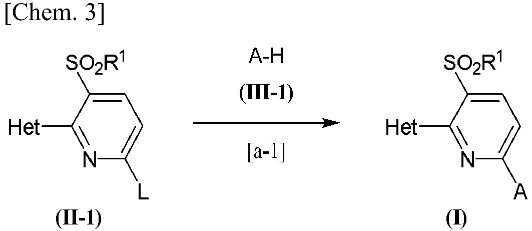

In the formula, Het, R¹ and A are the same as above; and L represents a leaving group such as fluorine, chlorine, bromine, iodine, a (C₁-C₄) alkylsulfonyl group, a (C₁-C₄) alkylsulfonyloxy group, or a halo (C₁-C₄) alkylsulfonyloxy group.

### Production method at step [a-1]

The compound represented by the general formula (I) can be produced by reacting the compound represented by the general formula (II-1) with the compound represented by the general formula (III-1) in the presence of an inert solvent with or without a base.

Examples of the base that can be used in this reaction include inorganic bases such as sodium hydroxide, potassium hydroxide, sodium carbonate, potassium carbonate, cesium carbonate, sodium hydrogen carbonate, potassium hydrogen carbonate, and sodium hydride; acetates such as sodium acetate and potassium acetate; as well as organic bases including alkali metal alkoxides such as potassium tert-butoxide, sodium methoxide and sodium ethoxide, tertiary amines such as triethylamine, N,N-diisopropylethylamine and DBU, nitrogen-containing aromatic compounds such as pyridine and N,N-dimethyl-4-aminopyridine; alkyllithiums such as methyllithium, n-butyllithium, sec-butyllithium and tert-butyllithium; and organometallic compounds such as lithium hexamethyldisilazane and sodium hexamethyldisilazane. The amount of the base used is usually in the range of a 1- to 10-fold molar amount relative to the compound represented by the general formula (II-1).

The inert solvent used in this reaction may be any solvent that does not markedly inhibit the progress of the reaction, and examples include chain or cyclic saturated hydrocarbons such as pentane, hexane, and cyclohexane; aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; nitriles such as acetonitrile and propionitrile; esters such as methyl acetate; ketones such as acetone and methyl ethyl ketone; aprotic polar solvents such as N,N-dimethylformamide, N,N-dimethylacetamide, dimethyl sulfoxide, and 1,3-dimethyl-2-imidazolidinone; and alcohols such as methanol, ethanol, propanol, butanol, and 2-propanol. One of these inert solvents may be used alone, and also two or more of them may be used as a mixture. The amount of the inert solvent used is usually selected as appropriate from the range of 0.1 L to 100 L relative to 1 mol of the compound represented by the general formula (II-1).

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production Method 2

The compound represented by the general formula (I') of the present invention can be produced from the compound represented by the general formula (II-1) through the step [a-2] described below.

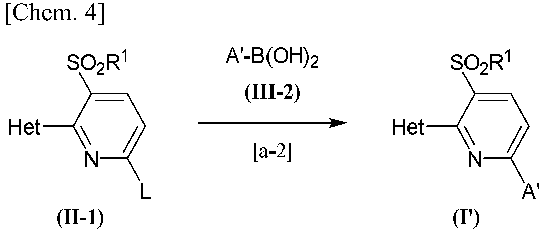

In the formula, Het and R¹ are the same as above; A' represents (i11) a pyrazolyl group, (i12) a substituted pyrazolyl group having 1 to 3 substituents on the ring, each independently selected from the set V of substituents, (i13) a triazolyl group, (i14) a substituted triazolyl group having 1 or 2 substituents on the ring, each independently selected from the set V of substituents, (i15) a tetrazolyl group, (i16) a substituted tetrazolyl group having 1 substituent on the ring selected from the set V of substituents, (i17) an imidazolyl group, (i18) a substituted imidazolyl group having 1 to 3 substituents on the ring, each independently selected from the set V of substituents, (i19) a pyrrolyl group, (i20) a substituted pyrrolyl group having 1 to 4 substituents on the ring, each independently selected from the set V of substituents, (i21) a thiazolyl group, (i22) a substituted thiazolyl group having 1 or 2 substituents on the ring, each independently selected from the set V of substituents, (i23) an oxazolyl group, or (i24) a substituted oxazolyl group having 1 or 2 substituents on the ring, each independently selected from the set V of substituents; and L represents a leaving group such as fluorine, chlorine, bromine, iodine, a (C₁-C₄) alkylsulfonyl group, a (C₁-C₄) alkylsulfonyloxy group, or a halo (C₁-C₄) alkylsulfonyloxy group.

### Production method at step [a-2]

The compound represented by the general formula (I') of the present invention can be produced by reacting the compound represented by the general formula (II-1) with the boronic acid represented by the general formula (III-2) or its pinacol ester in the presence of a metal catalyst, a base, and an inert solvent.

Examples of the base that can be used in this reaction include carbonates such as lithium carbonate, lithium hydrogen carbonate, sodium carbonate, potassium carbonate, cesium carbonate, potassium hydrogen carbonate, calcium carbonate, and magnesium carbonate; acetates such as lithium acetate, sodium acetate, and potassium acetate; and organic bases such as pyridine, picoline, lutidine, triethylamine, tributylamine, N,N-diisopropylethylamine, and 1,4-diazabicyclo[2.2.2]octane. The amount of the base used is usually in the range of a 1-to 5-fold molar amount relative to the compound represented by the general formula (II-1).

Examples of the metal catalyst that can be used in this reaction include a palladium catalyst, a nickel catalyst, an iron catalyst, a ruthenium catalyst, a platinum catalyst, a rhodium catalyst and an iridium catalyst. Such a metal catalyst can be used in the form of a metal; a supported metal; a metal salt such as a metal chloride, a metal bromide, a metal iodide, a metal nitrate, a metal sulfate, a metal carbonate, a metal oxalate, a metal acetate and a metal oxide; or a complex compound such as an olefin complex, a phosphine complex, an amine complex, an ammine complex and an acetylacetonate complex.

Among these metal catalysts, palladium catalysts are particularly preferred. Examples of the palladium catalyst include palladium metals such as palladium black and palladium sponge; supported palladium metals such as palladium/alumina, palladium/carbon, palladium/silica and palladium/type Y zeolite; palladium metal salts such as palladium chloride, palladium bromide, palladium iodide and palladium acetate; and palladium complex compounds such as π-allylpalladium chloride dimer, palladium acetylacetonate, dichlorobis(acetonitrile)palladium, dichlorobis(benzonitrile)palladium, bis(dibenzylideneacetone)palladium, tris(dibenzylideneacetone)dipalladium, tris(dibenzylideneacetone)dipalladium (chloroform adduct), dichlorodiamine palladium, dichlorobis(triphenylphosphine)palladium, dichlorobis(tricyclohexylphosphine)palladium, tetrakis(triphenylphosphine)palladium, dichloro[1,2-bis(diphenylphosphino)ethane]palladium, dichloro[1,3-bis(diphenylphosphino)propane]palladium, dichloro[1,4-bis(diphenylphosphino)butane]palladium, dichloro[1,1'-bis(diphenylphosphino)ferrocene]palladium, a [(diphenylphosphino)ferrocene]dichloropalladium-dichloromethane complex, etc. The amount of the metal catalyst used is usually selected as appropriate from the range of a 0.001-to 0.5-fold molar amount relative to the compound represented by the general formula (II-1).

These palladium catalysts may be used alone or in combination with a tertiary phosphine. Examples of the tertiary phosphine that can be used in combination with the palladium catalyst include triphenylphosphine, trimethylphosphine, triethylphosphine, tributylphosphine, tri(tert-butyl)phosphine, tricyclohexylphosphine, tri-o-tolylphosphine, trioctylphosphine, 9,9-dimethyl-4,5-bis(diphenylphosphino)xanthene, 2-(di-tert-butylphosphino)biphenyl, 2-(dicyclohexylphosphino)biphenyl, 1,2-bis(diphenylphosphino)ethane, 1,3-bis(diphenylphosphino)propane, 1,4-bis(diphenylphosphino)butane, 1,1'-bis(diphenylphosphino)ferrocene, (R)-(+)-2,2'-bis(diphenylphosphino)-1,1'-binaphthyl, (S)-(-)-2,2'-bis(diphenylphosphino)-1,1'-binaphthyl and (±)-2,2'-bis(diphenylphosphino)-1,1'-binaphthyl. The amount of the tertiary phosphine used is usually selected as appropriate from the range of a 0.5- to 10-fold molar amount relative to the metal catalyst.

The inert solvent used in this reaction may be any solvent that does not markedly inhibit the reaction, and examples include alcohols such as methanol, ethanol, propanol, butanol and 2-propanol; chain or cyclic ethers such as diethyl ether, tetrahydrofuran, dioxane and 1,2-dimethoxyethane; aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; nitriles such as acetonitrile; esters such as ethyl acetate; polar solvents such as N,N-dimethylformamide, N,N-dimethylacetamide, dimethyl sulfoxide and 1,3-dimethyl-2-imidazolidinone; and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (II-1).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like, but is basically selected as appropriate from the range of a few minutes to 48 hours. This reaction may be conducted under the atmosphere of an inert gas such as nitrogen gas and argon gas. After the reaction is completed, the compound of interest is isolated from the post-reaction mixture by the usual method. As needed, recrystallization, column chromatography, etc. can be employed for the purification of the compound of interest.

### Production Method 3

The compound represented by the general formula (I-1) of the present invention can be produced from the compound represented by the general formula (II-2) and the compound represented by the general formula (Het-2-H) through the step [b-1] described below. The reaction conditions for the step [b-1] are the same as those of the step [a-1].

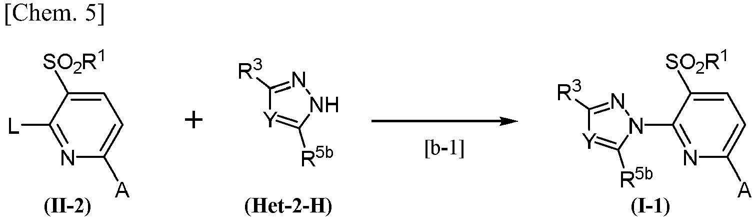

In the formula, R¹, R³, R^{5b}, A and Y are the same as above, and A may be the above-defined A'; and L represents a leaving group such as fluorine, chlorine, bromine, iodine, a (C₁-C₄) alkylsulfonyl group, a (C₁-C₄) alkylsulfonyloxy group, or a halo (C₁-C₄) alkylsulfonyloxy group.

### Production method 1 for starting compound

Among examples of the compound represented by the general formula (II-1) as the starting compound for Production Method 1, the compound represented by the general formula (II-1-1) wherein R¹ is the above-defined N(R^{2a})R^{2b}, Het is the formula (Het-2), L is a (C₁-C₄) alkylsulfonyl group can be produced from the compound represented by the general formula (IV-1) through the steps [c-1], [c-2], [c-3], and [c-4] described below. The reaction conditions for the step [c-3] are the same as those of the step [a-1].

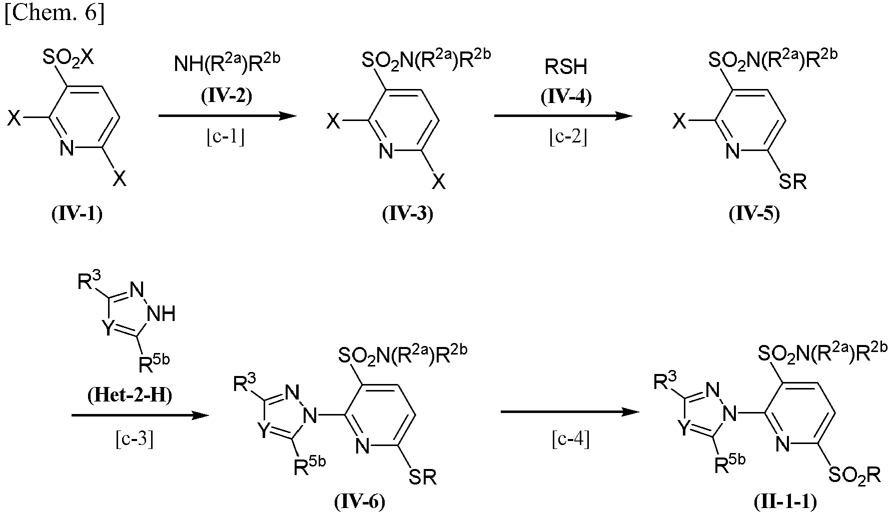

In the formula, R^{2a}, R^{2b}, R³, R^{5b} and Y are the same as above; R represents a (C₁-C₄) alkyl group such as a methyl group or an ethyl group; and X represents a leaving group of a halogen atom such as fluorine, chlorine, bromine, or iodine.

### Production method at step [c-1]

The compound represented by the general formula (IV-3) can be produced by reacting the compound represented by the general formula (IV-1) with the compound represented by the general formula (IV-2) in the presence of an inert solvent with or without a base.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (IV-1).

The inert solvent used in the reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; amides such as N,N-dimethylformamide and N,N-dimethylacetamide; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (IV-1).

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [c-2]

The compound represented by the general formula (IV-5) can be produced by reacting the compound represented by the general formula (IV-3) with the compound represented by the general formula (IV-4) in the presence of a base and an inert solvent.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (IV-3). In the case where an alkali salt of the compound represented by the general formula (IV-4) is used, it is not necessary to use a base.

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (IV-3).

Since this reaction is an equimolar reaction of the compounds, they are basically used in equimolar amounts, but either of them may be used in an excess amount. The reaction temperature in this reaction is usually in the range of -20°C to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [c-4]

The compound represented by the general formula (II-1-1) can be produced by reacting the compound represented by the general formula (IV-6) with an oxidizing agent in the presence of an inert solvent.

Examples of the oxidizing agent that can be used in this reaction include peroxides such as a hydrogen peroxide solution, peroxybenzoic acid and m-chloroperoxybenzoic acid. The amount of the oxidizing agent used is usually in the range of a 1- to 5-fold molar amount relative to the compound represented by the general formula (IV-6).

The inert solvent that can be used in this reaction may be any solvent that does not markedly inhibit the reaction, and examples include chain or cyclic ethers such as diethyl ether, tetrahydrofuran and dioxane; aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; nitriles such as acetonitrile; esters such as ethyl acetate; organic acids such as formic acid and acetic acid; and polar solvents such as N,N-dimethylformamide, N,N-dimethylacetamide, 1,3-dimethyl-2-imidazolidinone and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (IV-6).

The reaction temperature in this reaction is usually in the range of -10°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method 2 for starting compound

Among examples of the compound represented by the general formula (II-1) as the starting compound for Production Methods 1 and 2, the compound represented by the general formula (II-1-2) wherein R¹ is the above-defined N(R^{2a})R^{2b}, Het is the formula (Het-1), Y is a nitrogen atom, and L is a halogen atom such as fluorine, chlorine, bromine, iodine etc., can be produced from the compound represented by the general formula (V-1) through the steps [d-1], [d-2], [d-3], [d-4], and [d-5] described below. The reaction conditions for the step [d-1] are the same as those of the step [c-2].

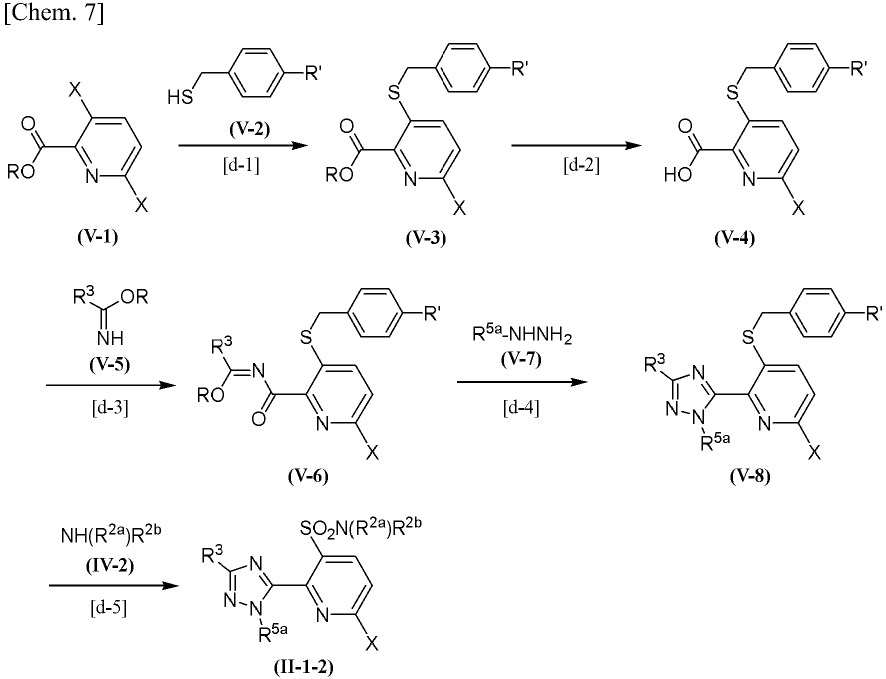

In the formula, R^{2a}, R^{2b}, R³, and R^{5a} are the same as above; R represents a (C₁-C₄) alkyl group such as a methyl group or an ethyl group; R' represents a (C₁-C₄) alkyl group such as a tert-butyl group or a hydrogen atom; and X represents a leaving group of a halogen atom such as fluorine, chlorine, bromine, or iodine.

### Production method at step [d-2]

The compound represented by the general formula (V-4) can be produced by hydrolyzing the compound represented by the general formula (V-3) in the presence of a base, water and an inert solvent.

Examples of the base that can be used in this reaction include organic bases such as lithium hydroxide, sodium hydroxide, potassium hydroxide, calcium hydroxide and other hydroxides. The amount of the base used is usually in the range of a 1- to 10-fold molar amount relative to the compound represented by the general formula (V-3).

The inert solvent used in this reaction may be any solvent that does not markedly inhibit the reaction, and examples include chain or cyclic saturated hydrocarbons such as pentane, hexane, and cyclohexane; chain or cyclic ethers such as diethyl ether, tetrahydrofuran, and dioxane; and nonpolar solvents including aromatic hydrocarbons such as benzene, toluene, and xylene. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (V-3).

The reaction temperature in this reaction is usually in the range of 0°C to the boiling point of the solvent used. The reaction time varies with the reaction scale, the reaction temperature and the like and is not the same in every case, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [d-3]

The compound represented by the general formula (V-6) can be produced by reacting the compound represented by the general formula (V-4) with the compound represented by the general formula (V-5) in the presence of a base, a condensing agent, and an inert solvent.

Examples of the condensing agent that can be used in this condensation reaction include acid-activating reagents such as phosgene, phosphorus trichloride, phosphorus oxychloride, oxalyl chloride, and thionyl chloride; carbodiimides such as N,N'-dicyclohexylcarbodiimide (DCC) and 1-(3-dimethylaminopropyl)-3-ethylcarbodiimide (EDCI); and other reagents such as phosphorus pentoxide, polyphosphoric acid, N,N'-carbonyldiimidazole, 2-chloropyridine-1-methoiodide (Mukaiyama reagent), 2-ethoxy-N-ethoxycarbonyl-1,2-dihydroquinoline (EEDQ), triphenylphosphine/carbon tetrachloride, bromotripyrrolidinophosphonium hexafluorophosphate (BROP), O-(1H-benzotriazol-1-yloxy)tris(dimethylamino)phosphonium hexafluorophosphate (BOP), N,N,N',N'-bis(tetramethylene)chlorouronium tetrafluoroborate, O-(1H-benzotriazol-1-yl)-N,N,N',N'-tetramethyluronium hexafluorophosphate (HBTU), O-(1H-benzotriazol-1-yl)-N,N,N',N'-bis(tetramethylene)uronium hexafluorophosphate, O-(1H-benzotriazol-1-yl)-N,N,N',N'-tetramethyluronium tetrafluoroborate (TBTU), O-(1H-benzotriazol-1-yl)-N,N,N',N'-bis(tetramethylene)uronium tetrafluoroborate, O-(7-azabenzotriazol-1-yl)-N,N,N',N'-tetramethyluronium hexafluorophosphate (HATU), 1-hydroxybenzotriazole (HOBt), propylphosphonic anhydride (T₃P), 4-(4,6-dimethoxy-1,3,5-triazin-2-yl)-4-methylmorpholinium salt (DMT-MM), etc. One of these condensing agents may be used alone, and also two or more of them may be used as a mixture. The amount of the condensing agent used is usually selected as appropriate from the range of a 0.5- to 5-fold molar amount relative to the compound represented by the general formula (V-4).

Examples of the base that can be used in the condensation include carbonates such as lithium carbonate, lithium hydrogen carbonate, sodium carbonate, sodium hydrogen carbonate, potassium carbonate, cesium carbonate, potassium hydrogen carbonate, calcium carbonate and magnesium carbonate; acetates such as lithium acetate, sodium acetate and potassium acetate; and organic bases such as pyridine, picoline, lutidine, triethylamine, tributylamine and N,N-diisopropylethylamine. The amount of the base used is usually selected as appropriate from the range of a 0.5- to 5-fold molar amount relative to the compound represented by the general formula (V-4). In some cases, the base can be used as the solvent as well.

The inert solvent used in the condensation may be any solvent that does not markedly inhibit the reaction, and examples include chain or cyclic saturated hydrocarbons such as pentane, hexane, and cyclohexane; chain or cyclic ethers such as diethyl ether, tetrahydrofuran, and dioxane; aromatic hydrocarbons such as benzene, toluene, and xylene; halogenated hydrocarbons such as methylene chloride, chloroform, and carbon tetrachloride; nitriles such as acetonitrile and isopropylnitrile; and polar solvents such as N,N-dimethylformamide, N,N-dimethylacetamide, dimethyl sulfoxide, and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (V-4). In the case where the base is used also as the solvent, it is not necessary to use another solvent.

Since the condensation is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [d-4]

The compound represented by the general formula (V-8) can be produced by reacting the compound represented by the general formula (V-6) with the compound represented by the general formula (V-7) or a salt thereof in the presence of an inert solvent with or without a base.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (V-6).

The inert solvent used in this reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (V-6).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [d-5]

The compound represented by the general formula (II-1-2) can be produced by chlorosulfonylation of the compound represented by the general formula (V-8) with a halogenating agent in the presence of an inert solvent, followed by amination with the compound represented by the general formula (IV-2) in the presence of an inert solvent with or without a base.

Examples of the halogenating agent that can be used in the chlorosulfonylation include thionyl chloride, chlorine, sulfuryl chloride, 1,3-dichloro-5,5-dimethylhydantoin, and N-chlorosuccinimide. The amount of the halogenating agent used is usually selected as appropriate from the range of a 0.5- to 5-fold molar amount relative to the compound represented by the general formula (V-8).

The inert solvent used in the chlorosulfonylation may be any solvent that does not markedly inhibit the progress of the reaction, and examples include halogenated hydrocarbons such as methylene chloride, chloroform and carbon tetrachloride; halogenated aromatic hydrocarbons such as chlorobenzene and dichlorobenzene; nitriles such as acetonitrile and propionitrile; organic acids such as acetic acid and propionic acid; and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (V-8).

Since the chlorosulfonylation is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours.

After the reaction is completed, when the chlorosulfonylated product is stable, the product is isolated from the post-reaction mixture by the usual method. As needed, recrystallization, column chromatography, etc. can be employed for the purification of the product. When the chlorosulfonylated product is unstable, the post-reaction mixture is directly used in the next reaction without purification.

Examples of the base that can be used in the amination include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (V-8).

The inert solvent used in the amination may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; amides such as N,N-dimethylformamide and N,N-dimethylacetamide; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (V-8).

Since the amination is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest.

### Production method 3 for starting compound

Among examples of the compound represented by the general formula (II-1) as the starting compound for Production Methods 1 and 2, the compound represented by the general formula (II-1-3) wherein R¹ is the above-defined N(R^{2a})R^{2b}, Het is the formula (Het-1), Y is the above-defined CR⁴, L is a halogen atom such as fluorine, chlorine, bromine, or iodine, can be produced from the compound represented by the general formula (V-4) through the steps [e-1], [e-2], [e-3], and [e-4] described below. The reaction conditions for the step [e-4] are the same as those of the step [d-5].

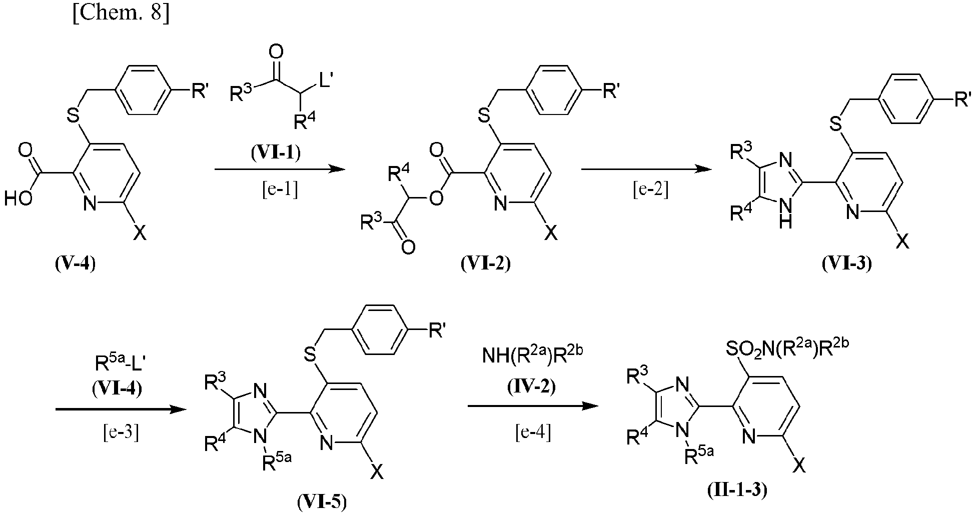

In the formula, R^{2a}, R^{2b}, R³, R⁴ and R^{5a} are the same as above; R' represents a (C₁-C₄) alkyl group such as a tert-butyl group or a hydrogen atom; L' and L may be the same or different, and each represent a leaving group such as fluorine, chlorine, bromine, iodine, or a (C₁-C₄) alkylcarbonyloxy group; and X represents a leaving group of a halogen atom such as fluorine, chlorine, bromine, or iodine.

### Production method at step [e-1]

The compound represented by the general formula (VI-2) can be produced by reacting the compound represented by the general formula (V-4) with the compound represented by the general formula (VI-1) in the presence of a base and an inert solvent.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (V-4).

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (V-4).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [e-2]

The compound represented by the general formula (VI-3) can be produced by reacting the compound represented by the general formula (VI-2) with a nitrogen source in the presence of an acid and an inert solvent.

Examples of the nitrogen source that can be used in this reaction include ammonia, ammonium carbamate, ammonium acetate, and ammonium carbonate. The amount of the nitrogen source used is usually selected as appropriate from the range of a 1- to 20-fold molar amount relative to the compound represented by the general formula (VI-2).

Examples of the acid that can be used in this reaction include inorganic acids such as hydrochloric acid, sulfuric acid and nitric acid; organic acids such as formic acid, acetic acid, propionic acid, trifluoroacetic acid and benzoic acid; sulfonic acids such as methanesulfonic acid, trifluoromethanesulfonic acid and p-toluenesulfonic acid; and phosphoric acid. The amount of the acid used is usually selected as appropriate from the range of a 0.01- to 10-fold molar amount relative to the compound represented by the general formula (VI-2). In some cases, the acid can be used as the solvent as well.

The inert solvent used in the dehydration may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (VI-2) obtained by the condensation. In the case where the acid is used also as the solvent, it is not necessary to use another solvent.

The reaction temperature in the dehydration is usually in the range of room temperature to the boiling point of the solvent used. The reaction time varies with the reaction scale and the reaction temperature, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [e-3]

The compound represented by the general formula (VI-5) can be produced by reacting the compound represented by the general formula (VI-3) with the compound represented by the general formula (VI-4) in the presence of an inert solvent and a base.

Examples of the base that can be used in this reaction include inorganic bases such as sodium hydroxide, potassium hydroxide, sodium carbonate, potassium carbonate, cesium carbonate, sodium hydrogen carbonate, potassium hydrogen carbonate and sodium hydride; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (VI-3).

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (VI-3).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method 4 for starting compound

Among examples of the compound represented by the general formula (II-2) as the starting compound for Production Method 3, the compound represented by the general formula (II-2-1) where L is a halogen atom such as fluorine, chlorine, bromine, or iodine can be produced from the compound represented by the general formula (VII-1) through the step [f-1] described below. The reaction conditions for the step [f-1] are the same as those of the step [a-1].

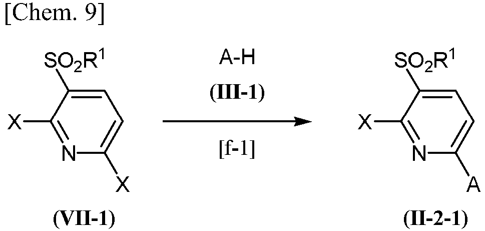

In the formula, R¹ and A are the same as above; and X represents a leaving group of a halogen atom such as fluorine, chlorine, bromine, or iodine.

### Production method 5 for starting compound

Among examples of the compound represented by the general formula (II-2) as the starting compound for Production Method 3, the compound represented by the general formula (II-2-1') where A is the above-defined A', L is a halogen atom such as fluorine, chlorine, bromine, or iodine can be produced from the compound represented by the general formula (VII-1) through the step [f-2] described below. The reaction conditions for the step [f-2] are the same as those of the step [a-2].

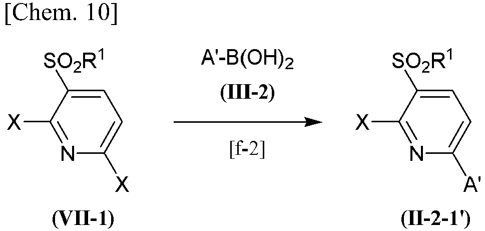

In the formula, R¹ and A' are the same as above; and X represents a leaving group of a halogen atom such as fluorine, chlorine, bromine, or iodine.

### Production method 6 for starting compound

The compound represented by the general formula (Het-2-H) of Production Method 3 can be produced by any of the methods described in, for example, Heterocycles, 1983, 20, 1243-1246, or Organic Process Research & Development, 2004, 8, 28-32, or their modified methods.

### Production method 7 for starting compound

Among examples of the compound represented by the general formula (II-1-2), the compound represented by the general formula (II-1-2-1) where R^{2a} is a hydrogen atom can be produced from the compound represented by the general formula (VIII-1) through the steps [g-1], [g-2], [g-3] and [g-4] described below. The reaction conditions for the step [g-1] are the same as those of the step [c-1].

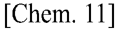

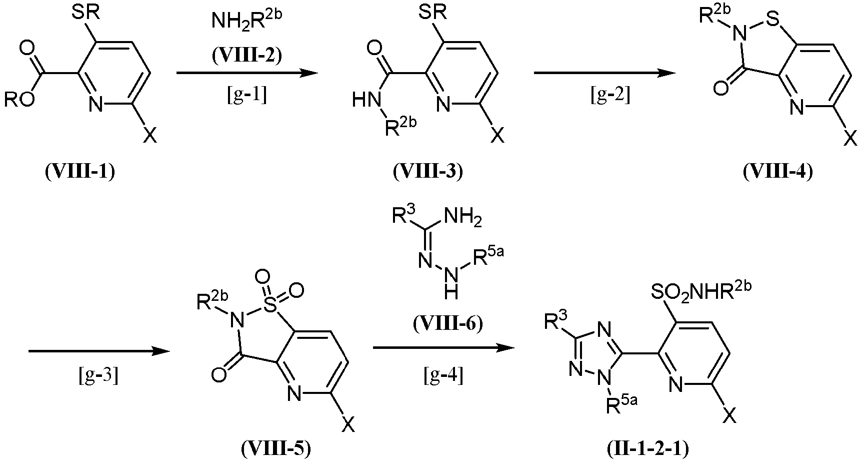

In the formula, R^{2b}, R³ and R^{5a} are the same as above; R represents a (C₁-C₄) alkyl group such as a methyl group or an ethyl group; and X represents a leaving group of a halogen atom such as fluorine, chlorine, bromine, or iodine.

### Production method at step [g-2]

The compound represented by the general formula (VIII-4) can be produced by reacting the compound represented by the general formula (VIII-3) with a halogenating agent in the presence of an inert solvent.

Examples of the halogenating agent that can be used in the reaction include thionyl chloride, chlorine, bromine, sulfuryl chloride, 1,3-dichloro-5,5-dimethylhydantoin, N-chlorosuccinimide, 1-chloromethyl-4-fluoro-1,4-diazoniabicyclo[2.2.2]octanebis(tetrafluoroborate), etc. The amount of the halogenating agent used is usually selected as appropriate from the range of a 0.5- to 5-fold molar amount relative to the compound represented by the general formula (VIII-3).

The inert solvent used in the reaction may be any solvent that does not markedly inhibit the progress of the reaction, and examples include halogenated hydrocarbons such as methylene chloride, chloroform and carbon tetrachloride; halogenated aromatic hydrocarbons such as chlorobenzene and dichlorobenzene; nitriles such as acetonitrile and propionitrile; organic acids such as acetic acid and propionic acid; and water. One of these inert solvents may be used alone, and also two or more of them may be used as a mixture. The amount of the inert solvent used is not particularly limited as long as it is sufficient to dissolve the compounds, and is usually selected as appropriate from the range of 0.5 L to 100 L relative to 1 mole of the compound represented by the general formula (VIII-3).

The reaction temperature in this reaction is usually in the range of 0°C to the boiling point of the solvent used. The reaction time varies with the reaction scale, the reaction temperature and the like and is not the same in every case, but is selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [g-3]

The compound represented by the general formula (VIII-5) can be produced by reacting the compound represented by the general formula (VIII-4) with an oxidizing agent in the presence of an inert solvent.

Examples of the oxidizing agent that can be used in this reaction include peroxides such as a hydrogen peroxide solution, peroxybenzoic acid and m-chloroperoxybenzoic acid. The amount of the oxidizing agent used is usually in the range of a 1- to 5-fold molar amount relative to the compound represented by the general formula (VIII-4).

The inert solvent that can be used in this reaction may be any solvent that does not markedly inhibit the reaction, and examples include chain or cyclic ethers such as diethyl ether, tetrahydrofuran and dioxane; aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; nitriles such as acetonitrile; esters such as ethyl acetate; organic acids such as formic acid and acetic acid; and polar solvents such as N,N-dimethylformamide, N,N-dimethylacetamide, 1,3-dimethyl-2-imidazolidinone and water. One of these inert solvents may be used alone, and also two or more of them may be used as a mixture. The amount of the inert solvent used is not particularly limited as long as it is sufficient to dissolve the compounds, and is usually selected as appropriate from the range of 0.5 L to 100 L relative to 1 mole of the compound represented by the general formula (VIII-4).

The reaction temperature in this reaction is usually in the range of -10°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [g-4]

The compound represented by the general formula (II-1-2-1) can be produced by reacting the compound represented by the general formula (VIII-5) with the compound represented by the general formula (VIII-6) or a salt thereof in the presence of a base and an inert solvent.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (VIII-5).

The inert solvent used in this reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; esters such as ethyl acetate; amides such as N,N-dimethylformamide and N,N-dimethylacetamide; ketones such as acetone and methyl ethyl ketone; and polar solvents such as dimethyl sulfoxide, 1,3-dimethyl-2-imidazolidinone, and acetic acid. One of these inert solvents may be used alone, and also two or more of them may be used as a mixture. The amount of the inert solvent used is not particularly limited as long as it is sufficient to dissolve the compounds, and is usually selected as appropriate from the range of 0.5 L to 100 L relative to 1 mole of the compound represented by the general formula (VIII-5).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method 8 for starting compound

The compound represented by the general formula (VIII-5) can also be produced from the compound represented by the general formula (VIII-3) through the steps [h-1] and [h-2] described below.

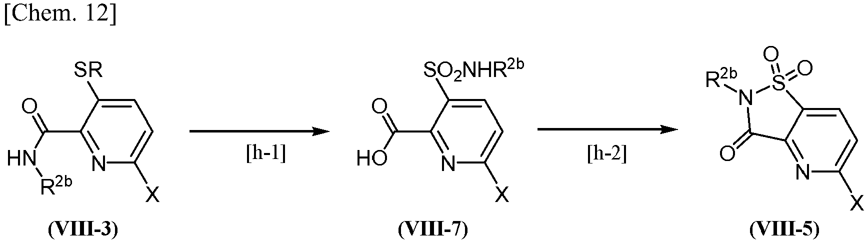

In the formula, R^{2b} is the same as above; R represents a (C₁-C₄) alkyl group such as a methyl group or an ethyl group; and X represents a leaving group of a halogen atom such as fluorine, chlorine, bromine, or iodine.

### Production method at step [h-1]

The compound represented by the general formula (VIII-7) can be produced by reacting the compound represented by the general formula (VIII-3) with a halogenating agent in the presence of water and an inert solvent.

The amount of the water used is usually selected as appropriate from the range of a 0.5-to 10-fold molar amount relative to the compound represented by the general formula (VIII-3).

Examples of the halogenating agent that can be used in the reaction include thionyl chloride, chlorine, bromine, sulfuryl chloride, 1,3-dichloro-5,5-dimethylhydantoin, and N-chlorosuccinimide. The amount of the halogenating agent used is usually selected as appropriate from the range of a 0.5- to 5-fold molar amount relative to the compound represented by the general formula (VIII-3).

The inert solvent used in the reaction may be any solvent that does not markedly inhibit the progress of the reaction, and examples include halogenated hydrocarbons such as methylene chloride, chloroform and carbon tetrachloride; halogenated aromatic hydrocarbons such as chlorobenzene and dichlorobenzene; nitriles such as acetonitrile and propionitrile; and organic acids such as acetic acid and propionic acid. One of these inert solvents may be used alone, and also two or more of them may be used as a mixture. The amount of the inert solvent used is not particularly limited as long as it is sufficient to dissolve the compounds, and is usually selected as appropriate from the range of 0.5 L to 100 L relative to 1 mole of the compound represented by the general formula (VIII-3).

The reaction temperature in this reaction is usually in the range of 0°C to the boiling point of the solvent used. The reaction time varies with the reaction scale, the reaction temperature and the like and is not the same in every case, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [h-2]

The compound represented by the general formula (VIII-5) can be produced by reacting the compound represented by the general formula (VIII-7) with a halogenating agent in the presence of an inert solvent.

Examples of the halogenating agent that can be used in the reaction include halogenated carbonyl compounds such as oxalyl chloride, phosgene, diphosgene and triphosgene; halogenated sulfur compounds such as thionyl chloride and thionyl bromide; and halogenated phosphorus compounds such as phosphorus oxychloride, phosphorus oxybromide, phosphorus trichloride, phosphorus tribromide and phosphorus pentachloride. One of these halogenating agents may be used alone, and also two or more of them may be used as a mixture. The amount of the halogenating agent used is usually selected as appropriate from the range of a 0.5- to 10-fold molar amount relative to the compound represented by the general formula (VIII-7). The halogenating agent itself can be used as a solvent. A catalyst that are commonly known to promote halogenation reaction, such as N,N-dimethylformamide, can also be used.

The inert solvent used in the reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; and halogenated aromatic hydrocarbons such as chlorobenzene and dichlorobenzene. One of these inert solvents may be used alone, and also two or more of them may be used as a mixture. The amount of the inert solvent used is not particularly limited as long as it is sufficient to dissolve the compounds, and is usually selected as appropriate from the range of 0.5 L to 100 L relative to 1 mole of the compound represented by the general formula (VIII-7). When the halogenating agent itself is used as a solvent, the inert solvent is not used.

The reaction temperature in this reaction is usually in the range of 0°C to the boiling point of the solvent used. The reaction time varies with the reaction scale, the reaction temperature and the like and is not the same in every case, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method 9 for starting compound

The compound represented by the general formula (VIII-6) can be produced from the compound represented by the general formula (IX-1) through the step [i-1] described below, or can be produced from the compound represented by the general formula (IX-2) through the steps [i-2] and [i-1] described below. The compound can also be produced by the method described in WO 2006/018725 or their modified methods.

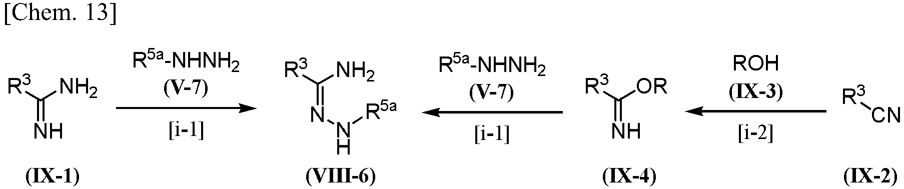

In the formula, R³ and R^{5a} are the same as above; and R represents a (C₁-C₄) alkyl group such as a methyl group or an ethyl group.

### Production method at step [i-1]

The compound represented by the general formula (VIII-6) or a salt thereof can be produced by reacting the compound represented by the general formula (IX-1) or the compound represented by the general formula (IX-4) described later or a salt thereof with the compound represented by the general formula (V-7) or a salt thereof in the presence of an inert solvent.

The inert solvent used in this reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; esters such as ethyl acetate; amides such as N,N-dimethylformamide and N,N-dimethylacetamide; ketones such as acetone and methyl ethyl ketone; and polar solvents such as dimethyl sulfoxide, and 1,3-dimethyl-2-imidazolidinone. One of these inert solvents may be used alone, and also two or more of them may be used as a mixture. The amount of the inert solvent used is not particularly limited as long as it is sufficient to dissolve the compounds, and is usually selected as appropriate from the range of 0.5 L to 100 L relative to 1 mole of the compound represented by the general formula (IX-1) or the compound represented by the general formula (IX-4) described later.

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [i-2]

The compound represented by the general formula (IX-4) or a salt thereof can be produced by reacting the compound represented by the general formula (IX-2) with the compound represented by the general formula (IX-3) in the presence of acetyl chloride and an inert solvent.

The amount of the acetyl chloride used is usually selected as appropriate from the range of a 0.5- to 10-fold molar amount relative to the compound represented by the general formula (IX-2).

The inert solvent used in this reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; esters such as ethyl acetate; amides such as N,N-dimethylformamide and N,N-dimethylacetamide; ketones such as acetone and methyl ethyl ketone; and polar solvents such as dimethyl sulfoxide, and 1,3-dimethyl-2-imidazolidinone. One of these inert solvents may be used alone, and also two or more of them may be used as a mixture. The amount of the inert solvent used is not particularly limited as long as it is sufficient to dissolve the compounds, and is usually selected as appropriate from the range of 0.5 L to 100 L relative to 1 mole of the compound represented by the general formula (IX-2). When the compound represented by the general formula (IX-3) itself is used as a solvent, the inert solvent may not be used.

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

Representative examples of the compound represented by the general formula (I) of the present invention are shown in Tables 1 to 3, but the present invention is not limited thereto.

In the tables below, Me stands for a methyl group, Et stands for an ethyl group, i-Pr stands for an isopropyl group, n-Pr stands for a n-propyl group, c-Pr stands for a cyclopropyl group, i-Bu stands for an isobutyl group, n-Bu stands for a n-butyl group, t-Bu stands for a tert-butyl group, n-Pen stands for a n-pentyl group, c-Pen stands for a cyclopentyl group, c-Hex stands for a cyclohexyl group, n-Hep stands for a n-heptyl group, Ac stands for an acetyl group, Ph stands for a phenyl group, and Bn stands for a benzyl group. Shown in the column of "Physical property value" is a melting point (°C) or "¹H-NMR". ¹H-NMR data are shown in Table 4.

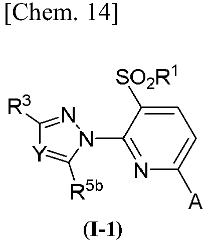

### [Table 1]

**Table 1**

| Compound No. | R¹ | R³ | R^{5b} | Y | A | Physical Property value |
|---|---|---|---|---|---|---|
| 1-1 | Me | 2-Cl-Ph | Me | N | 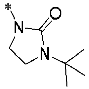 | NMR |
| 1-2 | Me | 2-Cl-Ph | Me | CH | 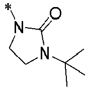 | NMR |
| 1-3 | Me | 2-Cl-Ph | Me | CH | 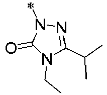 | NMR |

| | | | | | | |
|---|---|---|---|---|---|---|
| (In the Table, * indicates the position of binding.) | | | | | | |

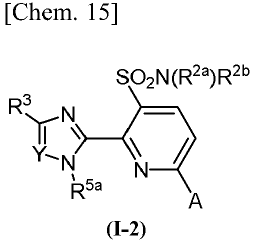

### [Table 2]

**Table 2**

| Compound No. | R^{2a} | R^{2b} | R³ | R^{5a} | Y | A | Physical Property value |
|---|---|---|---|---|---|---|---|
| 2-1 | H | Me | Ph | Me | N | 3,5-Me₂-pyrazol-1-yl | NMR |
| 2-2 | H | Me | pyridin-2-yl | Me | N | 3,5-Me₂-1,2,4-triazol-1-yl | NMR |
| 2-3 | H | Me | 4-Cl-Ph | Me | N | 3,5-Me₂-1,2,4-triazol-1-yl | NMR |
| 2-4 | H | Me | 2-Cl-Ph | Me | N | 3,5-Me₂-1,2,4-triazol-1-yl | NMR |
| 2-5 | H | Me | 2-F-Ph | Me | N | 3,5-Me₂-1,2,4-triazol-1-yl | NMR |
| 2-6 | H | Me | 4-Me-thiazol-5-yl | Me | N | 3,5-Me₂-1,2,4-triazol-1-yl | NMR |
| 2-7 | H | Me | 2-Me-Ph | Me | N | 3,5-Me₂-1,2,4-triazol-1-yl | NMR |
| 2-8 | H | Me | 2-OMe-Ph | Me | N | 3,5-Me₂-1,2,4-triazol-1-yl | NMR |
| 2-9 | H | Me | Ph | Me | N | 3,5-Me₂-1,2,4-triazol-1-yl | NMR |
| 2-10 | H | Me | 3,5-OMe₂-Ph | Me | N | 3,5-Me₂-1,2,4-triazol-1-yl | NMR |
| 2-11 | H | Me | 1-Ph-c-Pr | Me | N | 3,5-Me₂-1,2,4-triazol-1-yl | NMR |
| 2-12 | H | Me | 2-OMe-Bn | Me | N | 3,5-Me₂-1,2,4-triazol-1-yl | NMR |
| 2-13 | H | Me | 2-Cl-Bn | Me | N | 3,5-Me₂-1,2,4-triazol-1-yl | NMR |
| 2-14 | H | Me | naphthalen-1-yl | Me | N | 3,5-Me₂-1,2,4-triazol-1-yl | NMR |
| 2-15 | H | Me | 2,6-F₂-Ph | Me | N | 3,5-Me₂-1,2,4-triazol-1-yl | NMR |
| 2-16 | H | Me | Ph | Me | CH | 3,5-Me₂-1,2,4-triazol-1-yl | 134-135 |
| 2-17 | H | Me | 2-OH-Ph | Me | N | 3,5-Me₂-1,2,4-triazol-1-yl | 160-161 |
| 2-18 | H | Me | 3-CH₂OH-Ph | Me | N | 3,5-Me₂-1,2,4-triazol-1-yl | NMR |
| 2-19 | H | Me | 2-Me-Ph | Me | N | 3,5-Et₂-1,2,4-triazol-1-yl | NMR |
| 2-20 | H | Me | 3-F-Ph | Me | N | 3,5-Me₂-1,2,4-triazol-1-yl | 188-189 |

**[Table 3]**

| Table 2 (continued) | | | | | | | |
|---|---|---|---|---|---|---|---|
| Compound No. | R^{2a} | R^{2b} | R³ | R^{5a} | Y | A | Physical Property value |
| 2-21 | H | Me | 3-Me-Ph | Me | N | 3,5-Me₂-1,2,4-triazol-1-yl | 212-213 |
| 2-22 | H | Me | c-Hex | Me | N | 3,5-Me₂-1,2,4-triazol-1-yl | 240-241 |
| 2-23 | CO₂(t-Bu) | Me | 2-Cl-Ph | Me | N | 3,5-Me₂-1,2,4-triazol-1-yl | NMR |
| 2-24 | CO₂(i-Bu) | Me | 2-Cl-Ph | Me | N | 3,5-Me₂-1,2,4-triazol-1-yl | NMR |
| 2-25 | CO₂(n-Pr) | Me | 2-Cl-Ph | Me | N | 3,5-Me₂-1,2,4-triazol-1-yl | NMR |
| 2-26 | CO(c-Pen) | Me | 2-Cl-Ph | Me | N | 3,5-Me₂-1,2,4-triazol-1-yl | NMR |
| 2-27 | CO(n-Pr) | Me | 2-Cl-Ph | Me | N | 3,5-Me₂-1,2,4-triazol-1-yl | NMR |
| 2-28 | CO(i-Bu) | Me | 2-Cl-Ph | Me | N | 3,5-Me₂-1,2,4-triazol-1-yl | 82-83 |
| 2-29 | CO(n-Pen) | Me | 2-Cl-Ph | Me | N | 3,5-Me₂-1,2,4-triazol-1-yl | 67-68 |
| 2-30 | CO(n-Hep) | Me | 2-Cl-Ph | Me | N | 3,5-Me₂-1,2,4-triazol-1-yl | 118-119 |
| 2-31 | COEt | Me | 2-Cl-Ph | Me | N | 3,5-Me₂-1,2,4-triazol-1-yl | NMR |
| 2-32 | Ac | Me | 2-Cl-Ph | Me | N | 3,5-Me₂-1,2,4-triazol-1-yl | NMR |
| 2-33 | CO₂Me | Me | 2-Cl-Ph | Me | N | 3,5-Me₂-1,2,4-triazol-1-yl | NMR |
| 2-34 | H | Me | 2,3-Cl₂-Ph | Me | N | 3,5-Me₂-pyrazol-1-yl | NMR |
| 2-35 | H | Me | 5-Me-pyridin-2-yl | Me | N | 3,5-Me₂-pyrazol-1-yl | NMR |
| 2-36 | H | Me | pyridin-4-yl | Me | N | 3,5-Me₂-pyrazol-1-yl | NMR |
| 2-37 | H | Me | 2,5-F₂-Ph | Me | N | 3,5-Me₂-pyrazol-1-yl | NMR |
| 2-38 | H | Me | 3-Br-Ph | Me | N | 3,5-Me₂-pyrazol-1-yl | NMR |
| 2-39 | H | Me | 3-CHF₂-Ph | Me | N | 3,5-Me₂-pyrazol-1-yl | NMR |
| 2-40 | H | Me | 3-CF₃-pyridin-2-yl | Me | N | 3,5-Me₂-pyrazol-1-yl | NMR |

**[Table 4]**

| Table 2 (continued) | | | | | | | |
|---|---|---|---|---|---|---|---|
| Compound No. | R^{2a} | R^{2b} | R³ | R^{5a} | Y | A | Physical Property value |
| 2-41 | H | Me | thiophen-2-yl | Me | N | 3,5-Me₂-1,2,4-triazol-1-yl | 120-121 |
| 2-42 | H | Me | furan-2-yl | Me | N | 3,5-Me₂-1,2,4-triazol-1-yl | 193-194 |
| 2-43 | H | Me | 2-Cl-6-F-Ph | Me | N | 3,5-Me₂-1,2,4-triazol-1-yl | NMR |
| 2-44 | H | Me | 2,3-Cl₂-Ph | Me | N | 3,5-Me₂-1,2,4-triazol-1-yl | NMR |
| 2-45 | H | Me | 5-Me-pyridin-2-yl | Me | N | 3,5-Me₂-1,2,4-triazol-1-yl | NMR |
| 2-46 | H | Me | pyridin-4-yl | Me | N | 3,5-Me₂-1,2,4-triazol-1-yl | NMR |
| 2-47 | H | Me | 2,5-F₂-Ph | Me | N | 3,5-Me₂-1,2,4-triazol-1-yl | NMR |
| 2-48 | H | Me | 3-CHF₂-Ph | Me | N | 3,5-Me₂-1,2,4-triazol-1-yl | NMR |
| 2-49 | H | Me | 3-CF₃-pyridin-2-yl | Me | N | 3,5-Me₂-1,2,4-triazol-1-yl | NMR |
| 2-50 | H | Me | CH₂OMe | Me | N | 3,5-Me₂-1,2,4-triazol-1-yl | NMR |
| 2-51 | H | Me | thiophen-2-yl | Me | N | 3,5-Et₂-1,2,4-triazol-1-yl | NMR |
| 2-52 | H | Me | 3-Me-butan-1-yl | Me | N | 3,5-Me₂-1,2,4-triazol-1-yl | NMR |
| 2-53 | H | Me | i-Pr | Me | N | 3,5-Me₂-1,2,4-triazol-1-yl | NMR |
| 2-54 | H | Me | i-Pr | Me | N | 3,5-Et₂-1,2,4-triazol-1-yl | NMR |
| 2-55 | H | Me | thiophen-3-yl | Me | N | 3,5-Me₂-1,2,4-triazol-1-yl | NMR |
| 2-56 | H | Me | Ph | Et | N | 3,5-Me₂-1,2,4-triazol-1-yl | NMR |
| 2-57 | H | Me | n-Bu | Me | N | 3,5-Me₂-1,2,4-triazol-1-yl | NMR |
| 2-58 | H | Me | n-Bu | Me | N | 3,5-Et₂-1,2,4-triazol-1-yl | NMR |
| 2-59 | H | Me | thiazol-5-yl | Me | N | 3,5-Me₂-1,2,4-triazol-1-yl | NMR |
| 2-60 | H | Me | i-Bu | Me | N | 3,5-Me₂-1,2,4-triazol-1-yl | NMR |

**[Table 5]**

| Table 2 (continued) | | | | | | | |
|---|---|---|---|---|---|---|---|
| Compound No. | R^{2a} | R^{2b} | R³ | R^{5a} | Y | A | Physical Property value |
| 2-61 | H | Me | i-Bu | Me | N | 3,5-Et₂-1,2,4-triazol-1-yl | NMR |
| 2-62 | H | Me | thiophen-3-yl | Me | N | 3,5-Et₂-1,2,4-triazol-1-yl | NMR |
| 2-63 | H | Me | thiophen-2-yl | Et | N | 3,5-Me₂-1,2,4-triazol-1-yl | NMR |
| 2-64 | H | Me | Ph | Et | N | 3,5-Et₂-1,2,4-triazol-1-yl | NMR |
| 2-65 | H | Me | i-Pr | Et | N | 3,5-Me₂-1,2,4-triazol-1-yl | NMR |
| 2-66 | H | Me | 4-Me-thiazol-5-yl | Me | N | 3,5-Me₂-1,2,4-triazol-1-yl | NMR |
| 2-67 | H | Me | t-Bu | Me | N | 3,5-Me₂-1,2,4-triazol-1-yl | NMR |
| 2-68 | H | Et | thiophen-2-yl | Me | N | 3,5-Me₂-1,2,4-triazol-1-yl | NMR |
| 2-69 | H | Me | t-Bu | Me | N | 3,5-Me₂-pyrazol-1-yl | NMR |
| 2-70 | H | Me | thiophen-2-yl | Et | N | 3-Et-1,2,4-triazol-1-yl | NMR |
| 2-71 | H | Me | thiazol-5-yl | Me | N | 3-I-5-Me-pyrazol-1-yl | NMR |
| 2-72 | H | Me | Ph | Et | N | 3-Et-1,2,4-triazol-1-yl | NMR |
| 2-73 | H | Me | thiophen-2-yl | Me | N | 3-Et-1,2,4-triazol-1-yl | NMR |
| 2-74 | H | Me | 2,6-F₂-Ph | Me | N | 3-Et-1,2,4-triazol-1-yl | NMR |
| 2-75 | H | Me | thiophen-2-yl | Me | N | 3-(i-Pr)-1,2,4-triazol-1-yl | NMR |
| 2-76 | H | Me | i-Pr | Et | N | 3,5-Me₂-pyrazol-1-yl | NMR |
| 2-77 | H | Me | t-Bu | Me | N | 3-(i-Pr)-1,2,4-triazol-1-yl | NMR |
| 2-78 | H | Me | thiophen-3-yl | Me | N | 3-(i-Pr)-1,2,4-triazol-1-yl | NMR |
| 2-79 | H | Me | 2-F-Ph | Et | N | 3,5-Me₂-1,2,4-triazol-1-yl | NMR |
| 2-80 | H | Me | Ph | Et | N | 3-(n-Pr)-1,2,4-triazol-1-yl | NMR |

**[Table 6]**

| Table 2 (continued) | | | | | | | |
|---|---|---|---|---|---|---|---|
| Compound No. | R^{2a} | R^{2b} | R³ | R^{5a} | Y | A | Physical Property value |
| 2-81 | H | Me | 2,6-F₂-Ph | Me | N | 3-Me-pyrazol-1-yl | NMR |
| 2-82 | H | Me | 2,6-F₂-Ph | Me | N | 3-I-5-Me-pyrazol-1-yl | NMR |
| 2-83 | H | Me | 2,6-F₂-Ph | Me | N | 3-Cl-pyrazol-1-yl | NMR |
| 2-84 | H | Me | 4-Me-thiazol-5-yl | Me | N | 3,5-Me₂-pyrazol-1-yl | NMR |
| 2-85 | H | Me | c-Pr | Me | N | 3,5-Me₂-pyrazol-1-yl | NMR |
| 2-86 | H | Me | c-Pr | Me | N | 3-(i-Pr)-1,2,4-triazol-1-yl | NMR |
| 2-87 | H | Me | thiophen-2-yl | Me | N | 3-(n-Pr)-1,2,4-triazol-1-yl | NMR |
| 2-88 | H | Me | t-Bu | Me | N | 3-(n-Pr)-1,2,4-triazol-1-yl | NMR |
| 2-89 | H | Me | 2,6-F₂-Ph | Me | N | 3-(n-Pr)-1,2,4-triazol-1-yl | NMR |
| 2-90 | H | Me | 2-F-Ph | Et | N | 3-Et-1,2,4-triazol-1-yl | NMR |
| 2-91 | H | Me | 2-F-Ph | Et | N | 3-(i-Pr)-1,2,4-triazol-1-yl | NMR |
| 2-92 | H | Me | c-Pr | Me | N | 3-(n-Pr)-1,2,4-triazol-1-yl | NMR |
| 2-93 | H | Me | 2,5-F₂-Ph | Me | N | pyrazol-1-yl | NMR |
| 2-94 | H | Me | 2,5-F₂-Ph | Me | N | 3-Me-pyrazol-1-yl | NMR |
| 2-95 | H | Me | 2,5-F₂-Ph | Me | N | 3-I-pyrazol-1-yl | NMR |
| 2-96 | H | Me | 2,5-F₂-Ph | Me | N | 3-CO₂Me-pyrazol-1-yl | NMR |
| 2-97 | H | Me | 2,5-F₂-Ph | Me | N | 1,2,4-triazol-1-yl | NMR |
| 2-98 | H | Me | 2,5-F₂-Ph | Me | N | 3-Me-1,2,4-triazol-1-yl | NMR |
| 2-99 | H | Me | 2,5-F₂-Ph | Me | N | 3-(n-Pr)-1,2,4-triazol-1-yl | NMR |
| 2-100 | H | Me | 3-CHF₂-Ph | Me | N | 3-(n-Pr)-1,2,4-triazol-1-yl | NMR |

**[Table 7]**

| Table 2 (continued) | | | | | | | |
|---|---|---|---|---|---|---|---|
| Compound No. | R^{2a} | R^{2b} | R³ | R^{5a} | Y | A | Physical Property value |
| 2-101 | H | Me | 3-CF₃-pyridin-2-yl | Me | N | 3-(n-Pr)-1,2,4-triazol-1-yl | NMR |
| 2-102 | H | Me | 2,3-Cl₂-Ph | Me | N | 3-(n-Pr)-1,2,4-triazol-1-yl | NMR |
| 2-103 | H | Me | pyridin-2-yl | Me | N | 3-(n-Pr)-1,2,4-triazol-1-yl | NMR |
| 2-104 | H | Me | 5-Me-pyridin-2-yl | Me | N | 3-(n-Pr)-1,2,4-triazol-1-yl | NMR |
| 2-105 | H | Me | thiophen-3-yl | Me | N | 3-(n-Pr)-1,2,4-triazol-1-yl | NMR |
| 2-106 | H | Me | 4-Me-thiazol-5-yl | Me | N | 3-(n-Pr)-1,2,4-triazol-1-yl | NMR |
| 2-107 | H | Me | i-Pr | Et | N | 3-(n-Pr)-1,2,4-triazol-1-yl | NMR |
| 2-108 | H | Me | 2-Cl-Bn | Me | N | 3-(n-Pr)-1,2,4-triazol-1-yl | NMR |
| 2-109 | H | Me | 3,5-OMe₂-Ph | Me | N | 3-(n-Pr)-1,2,4-triazol-1-yl | NMR |
| 2-110 | H | Me | Ph | Et | N | 3-Et-5-Me-1,2,4-triazol-1-yl | NMR |
| 2-111 | H | Me | 2-F-Ph | Et | N | 3-SEt-5-Me-1,2,4-triazol-1-yl | NMR |
| 2-112 | H | Me | 2-F-Ph | Et | N | 3-Me-5-SEt-1,2,4-triazol-1-yl | NMR |
| 2-113 | H | Me | Ph | Et | N | 3-OMe-5-Me-pyrazol-1-yl | NMR |
| 2-114 | H | Me | 2-F-Ph | Et | N | 3-OMe-5-Me-pyrazol-1-yl | NMR |
| 2-115 | H | Me | 2,6-F₂-Ph | Et | N | 3,5-Me₂-1,2,4-triazol-1-yl | |
| 2-116 | H | Me | 2,3-F₂-Ph | Me | N | 3,5-Me₂-1,2,4-triazol-1-yl | |
| 2-117 | H | Me | 2,3-F₂-Ph | Et | N | 3,5-Me₂-1,2,4-triazol-1-yl | |
| 2-118 | H | Me | 2,3-F₂-Ph | Me | N | 3,5-Me₂-pyrazol-1-yl | |
| 2-119 | H | Me | 2-SMe-Ph | Me | N | 3,5-Me₂-1,2,4-triazol-1-yl | |
| 2-120 | H | Me | 2-SMe-Ph | Et | N | 3,5-Me₂-1,2,4-triazol-1-yl | |

**[Table 8]**

| Table 2 (continued) | | | | | | | |
|---|---|---|---|---|---|---|---|
| Compound No. | R^{2a} | R^{2b} | R³ | R^{5a} | Y | A | Physical Property value |
| 2-121 | H | Me | 2-SMe-Ph | Me | N | 3,5-Me₂-pyrazol-1-yl | |
| 2-122 | H | Me | 2-SOMe-Ph | Me | N | 3,5-Me₂-1,2,4-triazol-1-yl | |
| 2-123 | H | Me | 2-SOMe-Ph | Et | N | 3,5-Me₂-1,2,4-triazol-1-yl | |
| 2-124 | H | Me | 2-SOMe-Ph | Me | N | 3,5-Me₂-pyrazol-1-yl | |
| 2-125 | H | Me | 2-SO₂Me-Ph | Me | N | 3,5-Me₂-1,2,4-triazol-1-yl | |
| 2-126 | H | Me | 2-SO₂Me-Ph | Et | N | 3,5-Me₂-1,2,4-triazol-1-yl | |
| 2-127 | H | Me | 2-SO₂Me-Ph | Me | N | 3,5-Me₂-pyrazol-1-yl | |
| 2-128 | H | Me | 2-CN-Ph | Me | N | 3,5-Me₂-1,2,4-triazol-1-yl | |
| 2-129 | H | Me | 2-CN-Ph | Et | N | 3,5-Me₂-1,2,4-triazol-1-yl | |
| 2-130 | H | Me | 2-CN-Ph | Me | N | 3,5-Me₂-pyrazol-1-yl | |
| 2-131 | H | Me | 2-OCHF₂-Ph | Me | N | 3,5-Me₂-1,2,4-triazol-1-yl | |
| 2-132 | H | Me | 2-OCHF₂-Ph | Et | N | 3,5-Me₂-1,2,4-triazol-1-yl | |
| 2-133 | H | Me | 2-OCHF₂-Ph | Me | N | 3,5-Me₂-pyrazol-1-yl | |
| 2-134 | H | Me | 2-(c-Pr)-Ph | Me | N | 3,5-Me₂-1,2,4-triazol-1-yl | |
| 2-135 | H | Me | 2-(c-Pr)-Ph | Et | N | 3,5-Me₂-1,2,4-triazol-1-yl | |
| 2-136 | H | Me | 2-(c-Pr)-Ph | Me | N | 3,5-Me₂-pyrazol-1-yl | |
| 2-137 | H | Me | 2-CHF₂-Ph | Me | N | 3,5-Me₂-1,2,4-triazol-1-yl | |
| 2-138 | H | Me | 2-CHF₂-Ph | Et | N | 3,5-Me₂-1,2,4-triazol-1-yl | |
| 2-139 | H | Me | 2-CHF₂-Ph | Me | N | 3,5-Me₂-pyrazol-1-yl | |
| 2-140 | H | Me | 2-NHAc-Ph | Me | N | 3,5-Me₂-1,2,4-triazol-1-yl | |

**[Table 9]**

| Table 2 (continued) | | | | | | | |
|---|---|---|---|---|---|---|---|
| Compound No. | R^{2a} | R^{2b} | R³ | R^{5a} | Y | A | Physical Property value |
| 2-141 | H | Me | 2-NHAc-Ph | Et | N | 3,5-Me₂-1,2,4-triazol-1-yl | |
| 2-142 | H | Me | 2-NHAc-Ph | Me | N | 3,5-Me₂-pyrazol-1-yl | |
| 2-143 | H | Me | 2-F-Ph | Me | N | 3,5-Me₂-pyrazol-1-yl | |
| 2-144 | H | Me | 3-F-Ph | Et | N | 3,5-Me₂-1,2,4-triazol-1-yl | |
| 2-145 | H | Me | 3-F-Ph | Me | N | 3,5-Me₂-pyrazol-1-yl | |
| 2-146 | H | Me | 4-F-Ph | Me | N | 3,5-Me₂-1,2,4-triazol-1-yl | |
| 2-147 | H | Me | 4-F-Ph | Et | N | 3,5-Me₂-1,2,4-triazol-1-yl | |
| 2-148 | H | Me | 4-F-Ph | Me | N | 3,5-Me₂-pyrazol-1-yl | |
| 2-149 | H | Me | 2,6-F₂-Ph | Me | N | 3,5-Me₂-pyrazol-1-yl | |
| 2-150 | H | Me | 2,6-F₂-Ph | Me | N | 3,5-Et₂-1,2,4-triazol-1-yl | |
| 2-151 | H | Me | 2,6-F₂-Ph | Et | N | 3,5-Et₂-1,2,4-triazol-1-yl | |
| 2-152 | H | Me | 2,5-F₂-Ph | Et | N | 3,5-Me₂-1,2,4-triazol-1-yl | |
| 2-153 | H | Me | 2,5-F₂-Ph | Me | N | 3,5-Et₂-1,2,4-triazol-1-yl | |
| 2-154 | H | Me | 2,5-F₂-Ph | Et | N | 3,5-Et₂-1,2,4-triazol-1-yl | |
| 2-155 | H | Me | 2,4-F₂-Ph | Me | N | 3,5-Me₂-1,2,4-triazol-1-yl | |
| 2-156 | H | Me | 2,4-F₂-Ph | Et | N | 3,5-Me₂-1,2,4-triazol-1-yl | |
| 2-157 | H | Me | 2,4-F₂-Ph | Me | N | 3,5-Me₂-pyrazol-1-yl | |
| 2-158 | H | Me | Ph | Et | N | 1-Me-pyrazol-5-yl | NMR |
| 2-159 | H | Me | Ph | Et | N | 1-Me-pyrazol-4-yl | NMR |
| 2-160 | H | Me | thiophen-2-yl | Me | N | 1-Me-pyrazol-5-yl | 205-207 |

**[Table 10]**

| Table 2 (continued) | | | | | | | |
|---|---|---|---|---|---|---|---|
| Compound No. | R^{2a} | R^{2b} | R³ | R^{5a} | Y | A | Physical Property value |
| 2-161 | H | Me | thiophen-2-yl | Me | N | 4-Me-thiazol-2-yl | 98-100 |
| 2-162 | H | Me | thiophen-2-yl | Et | N | 3-Et-4,5-dihydro-5-oxo-pyrazol-1-yl | NMR |
| 2-163 | H | Me | thiophen-2-yl | Et | N | 4,5-dihydro-3-(i-Pr)-5-oxo-pyrazol-1-yl | NMR |
| 2-164 | H | Me | Ph | Me | N | 4-Me-thiazol-2-yl | |
| 2-165 | H | Me | Ph | Me | N | 4-Et-thiazol-2-yl | |
| 2-166 | H | Me | thiophen-2-yl | Me | N | 4-Et-thiazol-2-yl | |
| 2-167 | H | Me | n-Bu | Me | N | 4-Me-thiazol-2-yl | |
| 2-168 | H | Me | n-Bu | Me | N | 4-Et-thiazol-2-yl | |
| 2-169 | H | Me | 2,6-F₂-Ph | Me | N | 4-Me-thiazol-2-yl | |
| 2-170 | H | Me | 2,6-F₂-Ph | Me | N | 4-Et-thiazol-2-yl | |
| 2-171 | H | Me | thiophen-2-yl | Me | N | (4-Me-thiazol-2-yl)CH₂O | NMR |
| 2-172 | H | Me | thiophen-2-yl | Me | N | (3,5-Me₂-pyrazol-1-yl)CH₂O | 99-101 |
| 2-173 | H | Me | Ph | Et | N | (3,5-Me₂-pyrazol-1-yl)CH₂O | NMR |
| 2-174 | H | Me | Ph | Et | N | (3,5-Me₂-1,2,4-triazol-1-yl)CH₂O | NMR |
| 2-175 | H | Me | 2-F-Ph | Et | N | (3,5-Me₂-pyrazol-1-yl)CH₂O | NMR |
| 2-176 | H | Me | 2-F-Ph | Et | N | (3,5-Me₂-1,2,4-triazol-1-yl)CH₂O | NMR |
| 2-177 | H | Me | thiophen-2-yl | Et | N | (3,5-Me₂-pyrazol-1-yl)CH₂O | NMR |
| 2-178 | H | Me | thiophen-2-yl | Et | N | (3,5-Me₂-1,2,4-triazol-1-yl)CH₂O | NMR |
| 2-179 | H | Me | thiophen-2-yl | Me | N | (pyrazol-1-yl)CH₂O | 189-191 |
| 2-180 | H | Me | n-Pr | Me | N | (4-Me-thiazol-2-yl)CH₂O | NMR |

**[Table 11]**

| Table 2 (continued) | | | | | | | |
|---|---|---|---|---|---|---|---|
| Compound No. | R^{2a} | R^{2b} | R³ | R^{5a} | Y | A | Physical Property value |
| 2-181 | H | Me | thiophen-2-yl | Me | N | (3,5-Me₂-1,2,4-triazol-1-yl)CH₂O | 130-132 |
| 2-182 | H | Me | 2,5-F₂-Ph | Me | N | (3,5-Me₂-1,2,4-triazol-1-yl)CH₂O | NMR |
| 2-183 | H | Me | 3-CHF₂-Ph | Me | N | (3,5-Me₂-1,2,4-triazol-1-yl)CH₂O | NMR |
| 2-184 | H | Me | 2,3-Cl₂-Ph | Me | N | (3,5-Me₂-1,2,4-triazol-1-yl)CH₂O | NMR |
| 2-185 | H | Me | thiophen-3-yl | Me | N | (3,5-Me₂-1,2,4-triazol-1-yl)CH₂O | NMR |
| 2-186 | H | Me | 4-Me-thiazol-5-yl | Me | N | (3,5-Me₂-1,2,4-triazol-1-yl)CH₂O | NMR |
| 2-187 | H | Me | i-Pr | Et | N | (3,5-Me₂-1,2,4-triazol-1-yl)CH₂O | NMR |
| 2-188 | H | Me | c-Pr | Me | N | (3,5-Me₂-1,2,4-triazol-1-yl)CH₂O | NMR |
| 2-189 | H | Me | n-Bu | Et | N | (3,5-Me₂-pyrazol-1-yl)CH₂O | |
| 2-190 | H | Me | n-Bu | Et | N | (3,5-Me₂-1,2,4-triazol-1-yl)CH₂O | |
| 2-191 | H | Me | n-Bu | Me | N | (3,5-Me₂-pyrazol-1-yl)CH₂O | |
| 2-192 | H | Me | n-Bu | Me | N | (3,5-Me₂-1,2,4-triazol-1-yl)CH₂O | |
| 2-193 | H | Me | 2,6-F₂-Ph | Et | N | (3,5-Me₂-pyrazol-1-yl)CH₂O | |
| 2-194 | H | Me | 2,6-F₂-Ph | Et | N | (3,5-Me₂-1,2,4-triazol-1-yl)CH₂O | |
| 2-195 | H | Me | 2,6-F₂-Ph | Me | N | (3,5-Me₂-pyrazol-1-yl)CH₂O | |
| 2-196 | H | Me | 2,6-F₂-Ph | Me | N | (3,5-Me₂-1,2,4-triazol-1-yl)CH₂O | |
| 2-197 | H | Me | Ph | Me | N | (3,5-Me₂-1,2,4-triazol-1-yl)CH₂O | |
| 2-198 | H | Me | 2,6-F₂-Ph | Me | N | 1-(1,3-thiazol-2-yl)ethoxy | NMR |
| 2-199 | H | Me | Ph | Et | CH | 3,5-Me₂-1,2,4-triazol-1-yl | NMR |

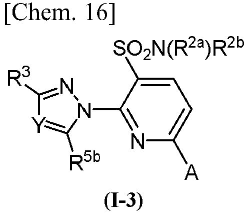

**[Table 12]**

| Table 3 | | | | | | | |
|---|---|---|---|---|---|---|---|
| Compound No. | R^{2a} | R^{2b} | R³ | R^{5b} | Y | A | Physical Property value |
| 3-1 | H | Me | Ph | Me | CH | 3,5-Me₂-1,2,4-triazol-1-yl | NMR |
| 3-2 | H | Me | 2-Cl-Ph | Me | N | 3,5-Me₂-1,2,4-triazol-1-yl | NMR |
| 3-3 | H | Me | 2-Cl-Ph | Me | N | 3-(2-Cl-Ph)-5-Me-1,2,4-triazol-1-yl | NMR |
| 3-4 | H | Me | CO₂Et | Me | CH | 3,5-Me₂-1,2,4-triazol-1-yl | NMR |
| 3-5 | H | Me | c-Pr | Me | CH | 3,5-Me₂-1,2,4-triazol-1-yl | NMR |
| 3-6 | H | Me | thiophen-2-yl | Me | N | 3-(thiophen-2-yl)-5-Me-1,2,4-triazol-1-yl | NMR |
| 3-7 | H | Me | thiophen-2-yl | Me | N | 3,5-Me₂-1,2,4-triazol-1-yl | NMR |
| 3-8 | H | Me | thiophen-2-yl | Et | N | 3,5-Me₂-1,2,4-triazol-1-yl | NMR |
| 3-9 | H | Me | thiophen-2-yl | Et | N | 3-(thiophen-2-yl)-5-Et-1,2,4-triazol-1-yl | NMR |
| 3-10 | H | Me | SMe | Me | N | 3-SMe-5-Me-1,2,4-triazol-1-yl | |
| 3-11 | H | Me | prop-1-en-2-yl | H | N | 3-(prop-1-en-2-yl)-1,2,4-triazol-1-yl | NMR |
| 3-12 | H | Me | c-Hex | H | N | 3-(c-Hex)-1,2,4-triazol-1-yl | NMR |
| 3-13 | H | Me | thiophen-2-yl | Me | N | 3-(c-Hex)-1,2,4-triazol-1-yl | NMR |
| 3-14 | H | Me | thiophen-2-yl | Me | N | 3-(prop-1-en-2-yl)-1,2,4-triazol-1-yl | NMR |
| 3-15 | H | Me | prop-1-en-1-yl | H | N | 3-(prop-1-en-1-yl)-1,2,4-triazol-1-yl | NMR |
| 3-16 | H | Me | Br | H | CH | 3-Br-pyrazol-1-yl | NMR |
| 3-17 | H | Me | CF₃ | Me | CH | 3-CF₃-5-Br-pyrazol-1-yl | NMR |
| 3-18 | H | Me | thiophen-2-yl | Me | N | 3-CF₃-5-Br-pyrazol-1-yl | NMR |
| 3-19 | H | Me | Ph | Et | N | 3,5-Me₂-1,2,4-triazol-1-yl | NMR |
| 3-20 | H | Me | Ph | Me | N | 3,5-Me₂-1,2,4-triazol-1-yl | |

**[Table 13]**

| Table 3 (continued) | | | | | | | |
|---|---|---|---|---|---|---|---|
| Compound No. | R^{2a} | R^{2b} | R³ | R^{5b} | Y | A | Physical Property value |
| 3-21 | H | Me | Ph | Me | N | 3,5-Me₂-pyrazol-1-yl | |
| 3-22 | H | Me | thiophen-2-yl | Et | N | 3,5-Me₂-1,2,4-triazol-1-yl | |
| 3-23 | H | Me | thiophen-2-yl | Me | N | 3,5-Et₂-1,2,4-triazol-1-yl | |
| 3-24 | H | Me | thiophen-2-yl | Me | N | 3,5-Me₂-pyrazol-1-yl | |
| 3-25 | H | Me | 2-Cl-Ph | Me | CH | 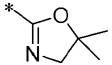 | NMR |
| 3-26 | H | Me | 2-Cl-Ph | Me | N | 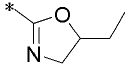 | 188-189 |
| 3-27 | H | Me | 2-Cl-Ph | Me | N | 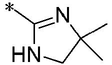 | NMR |
| 3-28 | H | Me | 2-Cl-Ph | Me | N | 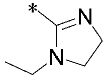 | NMR |
| 3-29 | H | Me | 2-Cl-Ph | Me | N | 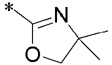 | NMR |
| 3-30 | H | Me | 2-Cl-Ph | Me | N | 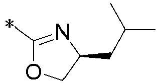 | 155-156 |
| 3-31 | H | Me | 2-Cl-Ph | Me | N | 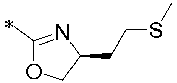 | NMR |
| 3-32 | H | Me | 2-Cl-Ph | Me | CH | 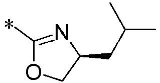 | NMR |
| 3-33 | H | Me | 2-Cl-Ph | Me | N | 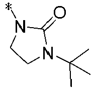 | NMR |
| 3-34 | H | Me | 2-Cl-Ph | Me | CH | 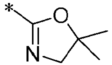 | NMR |

| | | | | | | | |
|---|---|---|---|---|---|---|---|
| (In the Table, * indicates the position of binding.) | | | | | | | |

**[Table 14]**

| Table 3 (continued) | | | | | | | |
|---|---|---|---|---|---|---|---|
| Compound No. | R^{2a} | R^{2b} | R³ | R^{5b} | Y | A | Physical Property value |
| 3-35 | H | Me | Ph | Me | CH | (3,5-Me₂-1,2,4-triazol-1-yl)CH₂O | NMR |
| 3-36 | H | Me | Ph | Me | CH | (3,5-Me₂-pyrazol-1-yl)CH₂O | NMR |
| 3-37 | H | Me | Ph | Me | CH | (5-Me-tetrahydrofuran-2-yl)CH₂O | NMR |
| 3-38 | H | Me | Ph | Me | CH | (2,2-Me₂-1,3-dioxolan-4-yl)CH₂O | NMR |
| 3-39 | H | Me | Ph | Me | CH | (3-(i-Pr)-4,5-dihydro-1,2-oxazol-5-yl)CH₂O | NMR |
| 3-40 | H | Me | thiophen-2-yl | Me | CH | (3,5-Me₂-1,2,4-triazol-1-yl)CH₂O | |
| 3-41 | H | Me | thiophen-2-yl | Me | CH | (3,5-Me₂-pyrazol-1-yl)CH₂O | |
| 3-42 | H | Me | n-Bu | Me | CH | (3,5-Me₂-1,2,4-triazol-1-yl)CH₂O | |
| 3-43 | H | Me | n-Bu | Me | CH | (3,5-Me₂-pyrazol-1-yl)CH₂O | |
| 3-44 | H | Me | 2,6-F₂-Ph | Me | CH | (3,5-Me₂-1,2,4-triazol-1-yl)CH₂O | |
| 3-45 | H | Me | 2,6-F₂-Ph | Me | CH | (3,5-Me₂-pyrazol-1-yl)CH₂O | |

**[Table 15]**

| Table 4 | |
|---|---|
| Compound No. | ¹H-NMR data (CDCl₃/TMS, ppm) |
| 1-1 | δ 8.63(d, 1H), 8.37(d, 1H), 8.05-8.03(m, 1H), 7.49-7.47(m, 1H), 7.38-7.34(m, 2H), 3.95(t, 2H), 3.57(t, 2H), 3.43(s, 3H), 2.55(s, 3H), 1.46(s, 9H). |
| 1-2 | δ 8.54(d, 1H), 8.06(d, 1H), 7.77-7.75(m, 1H), 7.47-7.44(m, 1H), 7.36-7.29(m, 2H), 6.74(s, 1H), 3.91(t, 3H), 3.61(t, 3H), 3.42(s, 3H), 2.39(s, 3H), 1.45(s, 9H). |
| 1-3 | δ 8.61(d, 1H), 8.27(d, 1H), 7.73-7.70(m, 1H), 7.46-7.44(m, 1H), 7.30-7.27(m, 2H), 6.71(s, 1H), 3.79(q, 2H), 3.61(s, 3H), 2.95-2.88(m, 1H), 2.64(s, 3H), 1.40(d, 6H), 1.38(t, 3H). |
| 2-1 | δ 8.52(d, 1H), 8.23(d, 1H), 8.09-7.95(m, 2H), 7.54-7.39(m, 4H), 6.08(s, 1H), 4.09(s, 3H), 2.81(s, 3H), 2.66(d, 3H), 2.33(s, 3H). |
| 2-2 | δ 8.77-8.73(m, 1H), 8.62(d, 1H), 8.20(d, 1H), 8.05-8.01(m, 1H), 7.86-7.81(m, 1H), 7.45-7.40(m, 1H), 7.39-7.35(m, 1H), 4.13(s, 3H), 2.85-2.81(m, 6H), 2.45(s, 3H). |
| 2-3 | δ 8.62(d, 1H), 8.21(d, 1H), 7.97-7.93(m, 2H), 7.45-7.43(m, 2H), 7.41-7.35(m, 1H), 4.08(s, 3H), 2.85-2.81(m, 6H), 2.45(s, 3H). |
| 2-4 | δ 8.63(d, 1H), 8.19(d, 1H), 7.97-7.92(m, 1H), 7.70-7.64(m, 1H), 7.54-7.50(m, 1H), 7.42-7.35(m, 2H), 4.14(s, 3H), 2.87(s, 3H), 2.76(d, 3H), 2.45(s, 3H). |
| 2-5 | δ 8.64(d, 1H), 8.18(d, 1H), 8.11-8.03(m, 1H), 7.75-7.68(m, 1H), 7.46-7.40(m, 1H), 7.26-7.18(m, 2H), 4.15(s, 3H), 2.85(s, 3H), 2.82(d, 3H), 2.45(s, 3H). |
| 2-6 | δ 8.61(d, 1H), 8.21(d, 1H), 8.02-7.99(m, 1H), 7.92-7.87(m, 1H), 7.44-7.39(m, 2H), 7.33-7.28(m, 1H), 4.08(s, 3H), 2.95-2.82(m, 6H), 2.45(s, 3H). |
| 2-7 | δ 8.62(d, 1H), 8.18(d, 1H), 7.81(d, 1H), 7.66-7.60(m, 1H), 7.37-7.29(m, 3H), 4.12(s, 3H), 2.95(s, 3H), 2.87(d, 3H), 2.62(d, 3H), 2.45(s, 3H). |
| 2-8 | δ 8.64(d, 1H), 8.18-8.09(m, 2H), 7.95-7.90(m, 1H), 7.47-7.41(m, 1H), 7.10-7.05(m, 2H), 4.14(s, 3H), 3.97(s, 3H), 2.87(s, 3H), 2.79(d, 3H), 2.46(s, 3H). |
| 2-9 | δ 8.61(d, 1H), 8.21(d, 1H), 8.02-7.99(m, 1H), 7.92-7.87(m, 1H), 7.43-7.40(m, 2H), 7.34-7.29(m, 1H), 4.08(s, 3H), 2.86-2.82(m, 6H), 2.45(s, 3H). |
| 2-10 | δ 8.61(d, 1H), 8.19(d, 1H), 7.50-7.43(m, 1H), 7.17(d, 2H), 6.56(t, 1H), 4.08(s, 3H), 3.86(s, 6H), 2.85-2.79(m, 6H), 2.46(s, 3H). |

**[Table 16]**

| Table 4 (continued) | |
|---|---|
| Compound No. | ¹H-NMR data (CDCl₃/TMS, ppm) |
| 2-11 | δ 8.50(d, 1H), 8.08(d, 1H), 7.50-7.46(m, 2H), 7.40-7.35(m, 2H), 7.31-7.29(m, 1H), 6.97-6.90(m, 1H), 3.97(s, 3H), 2.81(s, 3H), 2.45-2.41(m, 6H), 1.62-1.59(m, 2H), 1.43-1.38(m, 2H). |
| 2-12 | δ 8.51(d, 1H), 8.09(d, 1H), 7.29-7.24(m, 2H), 7.03-6.89(m, 3H), 4.13(s, 2H), 4.00(s, 3H), 3.82(s, 3H), 2.81(s, 3H), 2.49(d, 3H), 2.42(s, 3H). |
| 2-13 | δ 8.53(d, 1H), 8.12(d, 1H), 7.43-7.39(m, 1H), 7.37-7.33(m, 1H), 7.27-7.20(m, 2H), 7.09-7.02(m, 1H), 4.26(s, 2H), 4.01(s, 3H), 2.81(s, 3H), 2.57(d, 3H), 2.43(s, 3H). |
| 2-14 | δ 8.81(d, 1H), 8.65(d, 1H), 8.21(d, 1H), 8.03-7.91(m, 3H), 7.63-7.54(m, 4H), 4.22(s, 3H), 2.91(s, 3H), 2.73(d, 3H), 2.46(s, 3H). |
| 2-15 | δ 8.62(d, 1H), 8.19(d, 1H), 7.46-7.32(m, 2H), 7.05(t, 2H), 4.15(s, 3H), 2.86(s, 3H), 2.80(d, 3H), 2.44(s, 3H). |
| 2-18 | δ 8.62(d, 1H), 8.20(d, 1H), 8.03(s, 1H), 7.99-7.95(m, 1H), 7.52-7.42(m, 3H), 5.19(s, 2H), 4.10(s, 3H), 2.86-2.83(m, 6H), 2.45(s, 3H). |
| 2-19 | δ 8.61(d, 1H), 8.19(d, 1H), 7.85-7.81(m, 1H), 7.59-7.54(m, 1H), 7.37-7.29(m, 3H), 4.10(s, 3H), 3.28(q, 2H), 2.82(q, 2H), 2.76(d, 3H), 2.64(s, 3H), 1.42-1.36(m, 6H). |
| 2-23 | δ 8.79(d, 1H), 8.24(d, 1H), 8.05-8.00(m, 1H), 7.55-7.50(m, 1H), 7.38-7.34(m, 2H), 3.93(s, 3H), 2.87(s, 3H), 2.80(s, 3H), 2.45(s, 3H), 1.42(s, 9H). |
| 2-24 | δ 8.82(d, 1H), 8.24(d, 1H), 8.02-7.96(m, 1H), 7.55-7.50(m, 1H), 7.38-7.32(m, 2H), 3.94(s, 3H), 3.89(d, 2H), 2.98(s, 3), 2.79(s, 3H), 2.41(s, 3H), 1.94-1.84(m, 1H), 0.87(d, 6H). |
| 2-25 | δ 8.81(d, 1H), 8.24(d, 1H), 8.01-7.95(m, 1H), 7.55-7.50(m, 1H), 7.39-7.32(m, 2H), 4.08(t, 2H), 3.94(s, 3H), 2.98(s, 3H), 2.80(s, 3H), 2.44(s, 3H), 1.67-1.60(m, 2H), 0.90(t, 3H). |
| 2-26 | δ 8.88(d, 1H), 8.24(d, 1H), 7.96-7.92(m, 1H), 7.55-7.51(m, 1H), 7.39-7.34(m, 2H), 3.96(s, 3H), 3.02(s, 3H), 2.89-2.82(m, 1H), 2.81(s, 3H), 2.41(s, 3H), 1.86-1.75(m, 4H), 1.67-1.63(m, 2H), 1.56-1.47(m, 2H). |

**[Table 17]**

| Table 4 (continued) | |
|---|---|
| Compound No. | ¹H-NMR data (CDCl₃/TMS, ppm) |
| 2-27 | δ 8.88(d, 1H), 8.24(d, 1H), 7.98-7.92(m, 1H), 7.56-7.51(m, 1H), 7.40-7.33(m, 2H), 3.95(s, 3H), 3.00(s, 3H), 2.80(s, 3H), 2.44(s, 3H), 2.33(t, 2H), 1.58-1.53(m, 2H), 0.89(t, 3H). |
| 2-31 | δ 8.88(d, 1H), 8.24(d, 1H), 7.97-7.91(m, 1H), 7.56-7.48(m, 1H), 7.41-7.33(m, 2H), 3.96(s, 3H), 3.00(s, 3H), 2.79(s, 3H), 2.46-2.36(m, 5H), 1.05(t, 3H). |
| 2-32 | δ 8.85(d, 1H), 8.24(d, 1H), 7.97-7.93(m, 1H), 7.55-7.50(m, 1H), 7.39-7.35(m, 2H), 3.96(s, 3H), 3.21(s, 3H), 3.01(s, 3H), 2.80(s, 3H), 2.44(s, 3H), 2.16(s, 3H). |
| 2-33 | δ 8.82(d, 1H), 8.25(d, 1H), 7.99-7.94(m, 1H), 7.55-7.50(m, 1H), 7.39-7.34(m, 2H), 3.94(s, 3H), 3.74(s, 3H), 2.98(s, 3H), 2.80(s, 3H), 2.44(s, 3H). |
| 2-34 | δ 8.52(d, 1H), 8.21(d, 1H), 7.83-7.79(m, 1H), 7.60-7.55(m, 1H), 7.46(q, 1H), 7.32(t, 1H), 6.08(s, 1H), 4.12(s, 3H), 2.74(d, 3H), 2.67(s, 3H), 2.32(s, 3H). |
| 2-35 | δ 8.57(s, 1H), 8.51(d, 1H), 8.21(d, 1H), 7.91(d, 1H), 7.64-7.59(m, 1H), 7.39(q, 1H), 6.06(s, 1H), 4.10(s, 3H), 2.81(d, 3H), 2.62(s, 3H), 2.41(s, 3H), 2.32(s, 3H). |
| 2-36 | δ 8.76-8.71(m, 2H), 8.51(d, 1H), 8.25(d, 1H), 7.90-7.86(m, 2H), 7.15-7.07(m, 1H), 6.08(s, 1H), 4.09(s, 3H), 2.83(d, 3H), 2.65(s, 3H), 2.32(s, 3H). |
| 2-37 | δ 8.52(d, 1H), 8.21(d, 1H), 7.79-7.73(m, 1H), 7.48(q, 1H), 7.21-7.07(m, 2H), 6.07(s, 1H), 4.12(s, 3H), 2.79(d, 3H), 2.65(s, 3H), 2.32(s, 3H). |
| 2-38 | δ 8.50(d, 1H), 8.23(d, 1H), 8.19-8.16(m, 1H), 7.96-7.92(m, 1H), 7.59-7.53(m, 1H), 7.34(t, 3H), 7.22(q, 1H), 6.07(s, 1H), 4.06(s, 3H), 2.81(d, 3H), 2.64(s, 3H), 2.32(s, 3H). |
| 2-39 | δ 8.51(d, 1H), 8.23(d, 1H), 8.20-8.16(m, 1H), 8.14-8.09(m, 1H), 7.60-7.55(m, 2H), 7.28(q, 1H), 6.72(t, 1H), 6.07(s, 1H), 4.06(s, 3H), 2.81(d, 3H), 2.64(s, 3H), 2.32(s, 3H). |
| 2-40 | δ 8.97-8.94(m, 1H), 8.50(d, 1H), 8.23(d, 1H), 8.19-8.15(m, 1H), 7.56-7.52(m, 1H), 6.97(q, 1H), 6.07(s, 1H), 4.13(s, 3H), 2.73(d, 3H), 2.64(s, 3H), 2.33(s, 3H). |

**[Table 18]**

| Table 4 (continued) | |
|---|---|
| Compound No. | ¹H-NMR data (CDCl₃/TMS, ppm) |
| 2-43 | δ 8.62(d, 1H), 8.20(d, 1H), 7.43-7.37(m, 2H), 7.21-7.13(m, 2H), 4.14(s, 3H), 2.89(s, 3H), 2.74(d, 3H), 2.45(s, 3H). |
| 2-44 | δ 8.63(d, 1H), 8.20(d, 1H), 7.83-7.79(m, 1H), 7.60-7.52(m, 2H), 7.33(t, 1H), 4.15(s, 3H), 2.87(s, 3H), 2.76(d, 3H), 2.45(s, 3H). |
| 2-45 | δ 8.61(d, 1H), 8.57(s, 1H), 8.19(d, 1H), 7.96-7.88(m, 1H), 7.66-7.60(m, 1H), 7.51-7.44(m, 1H), 4.12(s, 3H), 2.85-2.81(m, 6H), 2.46(s, 3H), 2.42(s, 3H). |
| 2-46 | δ 8.78-8.71(m, 2H), 8.62(d, 1H), 8.23(d, 1H), 7.91-7.85(m, 2H), 7.19(q, 1H), 4.11(s, 3H), 2.85-2.83(m, 6H), 2.45(s, 3H). |
| 2-47 | δ 8.63(d, 1H), 8.19(d, 1H), 7.80-7.74(m, 1H), 7.61-7.55(m, 1H), 7.21-7.09(m, 2H), 4.14(s, 3H), 2.85(s, 3H), 2.81(d, 3H), 2.45(s, 3H). |
| 2-48 | δ 8.62(d, 1H), 8.21(d, 1H), 8.17(s, 1H), 8.15-8.10(m, 1H), 7.62-7.56(m, 2H), 7.37(q, 1H), 6.72(t, 1H), 4.11(s, 3H), 2.86-2.83(m, 6H), 2.45(s, 3H). |
| 2-49 | δ 8.96(d, 1H), 8.61(d, 1H), 8.24-8.16(m, 2H), 7.61-7.54(m, 1H), 7.09-7.02(m, 1H), 4.17(s, 3H), 2.85(s, 3H), 2.74(d, 3H), 2.45(s, 3H). |
| 2-50 | δ 8.58(d, 1H), 8.18(d, 1H), 7.33(q, 1H), 4.60(s, 2H), 4.03(s, 3H), 3.48(s, 3H), 2.82(s, 3H), 2.76(d, 3H), 2.45(s, 3H). |
| 2-51 | δ 8.59(d, 1H), 8.19(d, 1H), 7.68-7.61(m, 1H), 7.41-7.38(m, 1H), 7.30-7.26(m, 1H), 7.15-7.12(m, 1H), 4.05(s, 3H), 3.23(t, 2H), 2.85-2.77(m, 5H), 1.41-1.34(m, 6H). |
| 2-52 | δ 8.57(d, 1H), 8.15(d, 1H), 7.67(q, 1H), 3.98(s, 3H), 2.83(s, 3H), 2.81-2.75(m, 5H), 2.44(s, 3H), 1.71-1.63(m, 3H), 0.96(d, 6H). |
| 2-53 | δ 8.58(d, 1H), 8.14(d, 1H), 7.77(q, 1H), 3.99(s, 3H), 3.17-3.08(m, 1H), 2.84(s, 3H), 2.76(d, 3H), 2.44(s, 3H), 1.37(d, 6H). |
| 2-54 | δ 8.56(d, 1H), 8.15(d, 1H), 7.74(q, 1H), 3.98(s, 3H), 3.25(t, 2H), 3.16-3.08(m, 1H), 2.80(t, 2H), 2.76(d, 3H), 2.17(s, 3H), 1.40-1.34(m, 12H). |
| 2-55 | δ 8.60(d, 1H), 8.19(d, 1H), 7.88-7.85(m, 1H), 7.62-7.59(m, 1H), 7.49(q, 1H), 7.44-7.40(m, 1H), 4.07(s, 3H), 2.85-2.79(m, 6H), 2.44(s, 3H). |

**[Table 19]**

| Table 4 (continued) | |
|---|---|
| Compound No. | ¹H-NMR data(CDCl₃/TMS, ppm) |
| 2-56 | δ 8.61(d, 1H), 8.19(d, 1H), 8.05-8.00(m, 2H), 7.51-7.43(m, 3H), 7.32(q, 1H), 4.40(q, 2H), 2.84-2.79(m, 6H), 2.45(s, 3H), 1.50(t, 3H). |
| 2-57 | δ 8.57(d, 1H), 8.15(d, 1H), 7.67(q, 1H), 3.98(s, 3H), 2.83(s, 3H), 2.81-2.73(m, 5H), 2.44(s, 3H), 1.81-1.71(m, 2H), 1.49-1.38(m, 2H), 0.97(t, 3H). |
| 2-58 | δ 8.56(d, 1H), 8.15(d, 1H), 7.61(q, 1H), 3.98(s, 3H), 3.24(t, 2H), 2.83-2.72(m, 7H), 2.17(s, 3H), 1.82-1.73(m, 2H), 1.50-1.41(m, 2H), 1.41-1.33(m, 6H), 0.97(t, 3H). |
| 2-59 | δ 8.86(s, 1H), 8.61(s, 1H), 8.41(s, 1H), 8.2(d, 1H), 7.05(q, 1H), 4.11(s, 1H), 2.85-2.79(m, 6H), 2.45(s, 3H). |
| 2-60 | δ 8.57(d, 1H), 8.15(d, 1H), 7.67(q, 1H), 3.99(s, 3H), 2.84(s, 3H), 2.75(d, 3H), 2.67(d, 2H), 2.44(s, 3H), 2.17-2.07(m, 1H), 1.02(d, 6H). |
| 2-61 | δ 8.56(d, 1H), 8.15(d, 1H), 7.59(q, 1H), 3.98(s, 3H), 3.24(q, 2H), 2.81(q, 2H), 2.75(d, 3H), 2.65(d, 2H), 2.16-2.08(m, 1H), 1.41-1.33(m, 6H), 1.03(d, 6H). |
| 2-62 | δ 8.59(d, 1H), 8.19(d, 1H), 7.89-7.84(m, 1H), 7.64-7.58(m, 1H), 7.45-7.38(m, 2H), 4.04(s, 3H), 3.24(q, 2H), 2.84-2.79(m, 5H), 1.43-1.34(m, 6H). |
| 2-63 | δ 8.60(d, 1H), 8.19(d, 1H), 7.69-7.64(m, 1H), 7.41-7.36(m, 1H), 7.17-7.09(m, 2H), 4.38(q, 2H), 2.85-2.78(m, 6H), 2.45(s, 3H), 1.48(t, 3H). |
| 2-64 | δ 8.59(d, 1H), 8.19(d, 1H), 8.06-8.02(m, 1H), 7.51-7.42(m, 3H), 7.29-7.24(m, 1H), 4.38(q, 2H), 3.23(q, 2H), 2.86-2.78(m, 5H), 1.49(t, 3H), 1.41-1.32(m, 6H). |
| 2-65 | δ 8.56(d, 1H), 8.14(d, 1H), 7.55(q, 1H), 4.32(q, 2H), 3.18-3.09(m, 1H), 2.82(s, 3H), 2.76(d, 3H), 2.44(s, 3H), 1.43-1.35(m, 9H). |
| 2-66 | δ 8.72(s, 1H), 8.61(d, 1H), 8.20(d, 1H), 7.18(q, 1H), 4.10(s, 3H), 2.85-2.79(m, 9H), 2.45(s, 3H). |
| 2-67 | δ 8.58(d, 1H), 8.14(d, 1H), 7.88(q, 1H), 4.00(s, 3H), 2.84(s, 3H), 2.75(d, 3H), 2.44(s, 3H), 1.41(s, 9H). |

**[Table 20]**

| Table 4 (continued) | |
|---|---|
| Compound No. | ¹H-NMR data (CDCl₃/TMS, ppm) |
| 2-68 | δ 8.61 (d, 1H), 8.19 (d, 1H), 7.65 (dd, 1H), 7.42-7.37 (m, 2H), 7.14 (t, 1H), 4.07 (s, 3H), 3.17 (dt, 2H), 2.84 (s, 3H), 2.45 (s, 3H), 1.31 (t, 3H). |
| 2-69 | δ 8.47(d, 1H), 8.16(d, 1H), 7.81(q, 1H), 6.06(s, 1H), 3.98(s, 3H), 2.74(d, 3H), 2.65(s, 3H), 2.31(s, 3H), 1.41(s, 9H). |
| 2-70 | δ 9.00(s, 1H), 8.64(d, 1H), 8.12(d, 1H), 7.67-7.64(m, 1H), 7.50(q, 1H), 7.41-7.36(m, 1H), 7.16-7.10(m, 1H), 4.43(q, 2H), 2.89(q, 2H), 2.83(d, 3H), 1.57(t, 3H), 1.41(t, 3H). |
| 2-71 | δ 8.85(s, 1H), 8.55(d, 1H), 8.40(s, 1H), 8.28(d, 1H), 6.99(q, 1H), 4.05(s, 3H), 2.81(d, 3H), 2.66(s, 3H). |
| 2-72 | δ 8.64(d, 1H), 8.13(d, 1H), 8.06-8.00(m, 2H), 7.72(q, 1H), 7.50-7.44(m, 3H), 4.43(q, 2H), 2.89(q, 2H), 2.83(d, 3H), 1.59(t, 3H), 1.41(t, 3H). |
| 2-73 | δ 8.65(d, 1H), 8.11(d, 1H), 7.66-7.59(m, 2H), 7.41-7.38(m, 1H), 7.15-7.10(m, 1H), 4.13(s, 3H), 2.89(q, 2H), 2.82(d, 3H), 1.40(t, 3H). |
| 2-74 | δ 8.67(d, 1H), 8.12(d, 1H), 7.62(q, 1H), 7.44-7.38(m, 1H), 7.10-7.03(m, 2H), 4.22(s, 3H), 2.89(q, 2H), 2.77(d, 3H), 1.41(t, 3H). |
| 2-75 | δ 8.65(d, 1H), 8.12(d, 1H), 7.66-7.59(m, 2H), 7.41-7.37(m, 1H), 7.16-7.11(m, 1H), 4.14(s, 1H), 3.23-3.14(m, 1H), 2.82(d, 3H), 1.41(d, 6H). |
| 2-76 | δ 8.45(d, 1H), 8.16(d, 1H), 7.48(q, 1H), 6.05(s, 1H), 4.31(q, 2H), 3.17-3.08(m, 1H), 2.74(d, 3H), 2.62(s, 3H), 2.31(s, 3H), 1.41-1.35(m, 9H). |
| 2-77 | δ 8.62(d, 1H), 8.17-8.06(m, 2H), 4.06(s, 1H), 3.23-3.15(m, 1H), 2.75(d, 3H), 1.43-1.38(m, 15H). |
| 2-78 | δ 8.65(d, 1H), 8.12(d, 1H), 7.87-7.77(m, 2H), 7.61-7.59(m, 1H), 7.44-7.39(m, 1H), 4.14(s, 3H), 3.25-3.15(m, 1H), 2.80(d, 3H), 1.41(d, 6H). |
| 2-79 | δ 8.61(d, 1H), 8.17(d, 1H), 8.11-8.06(m, 1H), 7.53-7.39(m, 2H), 7.28-7.18(m, 2H), 4.46(q, 2H), 2.84-2.78(m, 6H), 2.45(s, 3H), 1.51(t, 3H). |
| 2-80 | δ 8.65(d, 1H), 8.12(d, 1H), 8.05-7.99(m, 2H), 7.71(q, 1H), 7.51-7.42(m, 3H), 4.46(q, 2H), 2.86-2.80(m, 5H), 1.92-1.82(m, 1H), 1.59(t, 3H), 1.05(3H). |
| 2-81 | δ 8.57(d, 1H), 8.40(s, 1H), 8.15(d, 1H), 7.65-7.56(m, 1H), 7.44-7.37(m, 1H), 7.06(t, 2H), 6.35(s, 1H), 4.23(s, 3H), 2.76(d, 3H), 2.41(s, 3H). |

**[Table 21]**

| Table 4 (continued) | |
|---|---|
| Compound No. | ¹H-NMR data (CDCl₃/TMS, ppm) |
| 2-82 | δ 8.57(d, 1H), 8.27(d, 1H), 7.47-7.30(m, 2H), 7.12-7.03(m, 2H), 6.42(s, 1H), 4.14(s, 3H), 2.78(d, 3H), 2.69(s, 3H). |
| 2-83 | δ 8.62(d, 1H), 8.17(d, 1H), 7.60-7.54(m, 1H), 7.45-7.38(m, 1H), 7.07(t, 2H), 6.50(d, 1H), 4.21(s, 3H), 2.77(d, 3H). |
| 2-84 | δ 8.71(s, 1H), 8.49(d, 1H), 8.23(d, 1H), 7.12(q, 1H), 4.07(s, 3H), 2.83(s, 3H), 2.78(d, 3H), 2.64(s, 3H), 2.32(s, 3H). |
| 2-85 | δ 8.45(d, 1H), 8.16(d, 1H), 7.50(q, 1H), 6.05(s, 1H), 3.95(s, 3H), 2.72(d, 3H), 2.62(s, 3H), 2.31(s, 3H), 2.13-2.06(m, 1H), 1.06-0.99(m, 2H), 0.99-0.92(m, 2H). |
| 2-86 | δ 8.60(d, 1H), 8.07(d, 1H), 7.84(q, 1H), 4.02(s, 3H), 3.22-3.14(m, 1H), 2.73(d, 3H), 2.13-2.06(m, 1H), 1.41(d, 6H), 1.07-1.00(m, 2H), 0.99-0.93(m, 2H). |
| 2-87 | δ 8.65(d, 1H), 8.11(d, 1H), 7.67-7.57(m, 2H), 7.41-7.37(m, 1H), 7.17-7.11(m, 1H), 4.13(s, 3H), 2.85-2.77(m, 5H), 1.91-1.81(m, 2H), 1.07-1.00(m, 3H). |
| 2-88 | δ 8.62(d, 1H), 8.13(q, 1H), 8.06(d, 1H), 4.07(s, 3H), 2.82(t, 2H), 2.74(d, 3H), 1.90-1.82(m, 1H), 1.04(t, 3H). |
| 2-89 | δ 8.67(d, 1H), 8.12(d, 1H), 7.65-7.59(m, 1H), 7.45-7.37(m, 1H), 7.06(t, 3H), 4.22(s, 3H), 2.84(q, 2H), 2.78(d, 3H), 1.90-1.82(m, 2H), 1.04(t, 3H). |
| 2-90 | δ 8.70(d, 1H), 8.14-8.05(m, 2H), 7.89-7.82(m, 1H), 7.46-7.39(m, 1H), 7.25-7.15(m, 2H), 4.52(q, 2H), 2.88(q, 2H), 2.80(d, 3H), 1.60(t, 3H), 1.41(t, 3H). |
| 2-91 | δ 8.66(d, 1H), 8.15-8.06(m, 2H), 7.90-7.84(m, 1H), 7.46-7.39(m, 1H).7.25-7.17(m, 2H), 4.51(q, 2H), 3.21-3.16(m, 2H), 2.81(d, 3H), 1.61(t, 3H), 1.42(d, 6H). |
| 2-92 | δ 8.60(d, 1H), 8.06(d, 1H), 7.82(q, 1H), 4.03(s, 3H), 2.82(t, 2H), 2.74(d, 3H), 2.13-2.06(m, 1H), 1.89-1.81(m, 2H), 1.06-0.99(m, 5H), 0.99-0.92(m, 2H). |
| 2-93 | δ 8.62(d, 1H), 8.51-8.49(m, 1H), 8.23(d, 1H), 7.86-7.74(m, 2H), 7.24-7.07(m, 2H), 6.56-6.55(m, 1H), 4.23(s, 3H), 2.80(d, 3H). |
| 2-94 | δ 8.56(d, 1H), 8.38(d, 1H), 8.14(d, 1H), 7.80-7.73(m, 2H), 7.21-7.06(m, 2H), 6.34(d, 1H), 4.21(s, 3H), 2.79(s, 3H), 2.41(s, 3H). |

**[Table 22]**

| Table 4 (continued) | |
|---|---|
| Compound No. | ¹H-NMR data (CDCl₃/TMS, ppm) |
| 2-95 | δ 8.61(d, 1H), 8.31(d, 1H), 8.22(d, 1H), 7.80-7.73(m, 2H), 7.21-7.07(m, 2H), 6.71(d, 1H), 4.20(s, 3H), 2.80(d, 3H). |
| 2-96 | δ 8.66(d, 1H), 8.55(d, 1H), 8.38(d, 1H), 7.83-7.74(m, 2H), 7.22-7.09(m, 2H), 7.06(d, 1H), 4.23(s, 3H), 4.01(s, 3H), 2.81(d, 3H). |
| 2-97 | δ 9.14(s, 1H), 8.72(d, 1H), 8.21-8.16(m, 2H), 7.86-7.75(m, 2H), 7.22-7.08(m, 2H), 4.23(s, 3H), 2.82(d, 3H). |
| 2-98 | δ 9.01(s, 1H), 8.68(d, 1H), 8.10(d, 1H), 7.85-7.74(m, 2H), 7.21-7.08(m, 2H), 4.21(s, 3H), 2.80(d, 3H), 2.53(s, 3H). |
| 2-99 | δ 9.02(s, 1H), 8.67(d, 1H), 8.11(d, 1H), 7.85-7.75(m, 2H), 7.21-7.08(m, 2H), 4.21(s, 3H), 2.86-2.79(m, 5H), 1.92-1.82(m, 2H), 1.05(t, 3H). |
| 2-100 | δ 9.03(s, 1H), 8.66(d, 1H), 8.18-8.09(m, 3H), 7.66-7.57(m, 3H), 6.72(t, 1H), 4.16(s, 1H), 2.86-2.81(m, 5H), 1.90-1.82(m, 2H), 1.04(t, 3H). |
| 2-101 | δ 9.04(s, 1H), 8.97(d, 1H), 8.66(d, 1H), 8.19-8.12(m, 2H), 7.58-7.54(m, 1H), 7.31(q, 1H), 4.23(s, 3H), 2.84(t, 2H), 2.74(d, 3H), 1.92-1.81(m, 2H), 1.03(t, 3H). |
| 2-102 | δ 9.04(s, 1H), 8.67(d, 1H), 8.12(d, 1H), 7.81-7.74(m, 2H), 7.61-7.56(m, 1H), 7.33(t, 1H), 4.22(s, 3H), 2.84(t, 2H), 2.76(d, 3H), 1.91-1.82(m, 2H), 1.05(t, 3H). |
| 2-103 | δ 9.03(s, 1H), 8.77-8.73(m, 1H), 8.66(d, 1H), 8.12(d, 1H), 8.03(d, 1H), 7.86-7.81(m, 1H), 7.74(q, 1H), 7.40-7.35(m, 1H), 4.21(s, 3H), 2.87-2.80(m, 5H), 1.92-1.81(m, 2H), 1.04(t, 3H). |
| 2-104 | δ 9.02(s, 1H), 8.66(d, 1H), 8.57(s, 1H), 8.11(d, 1H), 7.91(d, 1H), 7.77(q, 1H), 7.63(dd, 1H), 4.20(s, 3H), 2.86-2.79(m, 5H), 2.42(s, 3H), 1.91-1.81(m, 2H), 1.04(t, 3H). |
| 2-105 | δ 9.02(s, 1H), 8.65(d, 1H), 8.11(d, 1H), 7.88-7.48(m, 1H), 7.80(q, 1H), 7.62-7.57(m, 1H), 7.43-7.38(m, 1H), 4.11(s, 3H), 2.86-2.77(m, 5H), 1.91-1.81(m, 2H), 1.04(t, 3H). |
| 2-106 | δ 9.02(s, 1H), 8.72(s, 1H), 8.65(d, 1H), 8.13(d, 1H), 7.41(q, 1H), 4.13(s, 3H), 2.85-2.77(m, 8H), 1.91-1.81(m, 2H), 1.04(t, 3H). |
| 2-107 | δ 8.99(s, 1H), 8.61(d, 1H), 8.08(d, 1H), 7.91(q, 1H), 4.36(q, 2H), 3.17-3.09(m, 1H), 2.82(t, 2H), 2.75(d, 3H), 1.90-1.81(m, 2H), 1.51(t, 3H), 1.37(d, 6H), 1.03(t, 3H). |

**[Table 23]**

| Table 4 (continued) | |
|---|---|
| Compound No. | ¹H-NMR data (CDCl₃/TMS, ppm) |
| 2-108 | δ 8.99(s, 1H), 8.57(d, 1H), 8.05(d, 1H), 7.43-7.29(m, 3H), 7.27-7.21(m, 2H), 4.27(s, 2H), 4.09(s, 3H), 2.81(t, 2H), 2.56(d, 3H), 1.89-1.79(m, 2H), 1.02(t, 3H). |
| 2-109 | δ 9.02(s, 1H), 8.65(d, 1H), 8.11(d, 1H), 7.77(q, 1H), 7.18-7.15(m, 2H), 6.57-6.53(m, 1H), 4.15(s, 3H), 3.87(s, 6H), 2.85-2.78(m, 5H), 1.92-1.81(m, 2H), 1.04(t, 3H). |
| 2-110 | δ 8.60(d, 1H), 8.21(d, 1H), 8.06-8.01(m, 2H), 7.51-7.44(m, 3H), 7.33(q, 1H), 4.44-4.35(m, 2H), 3.75-3.67(m, 2H), 2.86-2.79(m, 6H), 1.52-1.47(m, 3H), 1.41-1.33(m, 3H). |
| 2-111 | δ 8.51(d, 1H), 8.21(d, 1H), 8.11-8.05(m, 1H), 7.44-7.38(m, 2H), 7.25-7.17(m, 2H), 6.17(s, 1H), 4.45(q, 2H), 3.11(q, 2H), 2.80(d, 3H), 2.65(s, 3H), 1.51-1.41(m, 6H). |
| 2-112 | δ 8.52(d, 1H), 8.16(d, 1H), 8.13-8.07(m, 1H), 7.62(q, 1H), 7.44-7.36(m, 1H), 7.25-7.18(m, 2H), 6.07(s, 1H), 4.62(q, 2H), 2.92(q, 2H), 2.78(d, 3H), 2.92(s, 3H), 1.48(t, 3H), 1.37(t, 3H). |
| 2-113 | δ 8.44(d, 1H), 8.09(d, 1H), 8.06-8.02(m, 2H), 7.49-7.42(m, 3H), 7.17(q, 1H), 5.75(s, 1H), 4.42-4.28(m, 4H), 2.79(d, 3H), 2.62(s, 3H), 1.51-1.42(m, 6H). |
| 2-114 | δ 8.46(d, 1H), 8.11-8.04(m, 2H), 7.43-7.33(m, 2H), 7.24-7.16(m, 2H), 5.75(s, 1H), 4.46(q, 2H), 4.32(q, 2H), 2.78(d, 3H), 2.63(s, 3H), 1.51-1.41(m, 6H). |
| 2-158 | δ 8.51(d, 1H), 8.06-8.00(m, 2H), 7.84(d, 1H), 7.59(s, 1H), 7.56-7.42(m, 4H), 6.79(s, 1H), 4.43(q, 2H), 4.22(s, 3H), 2.83(d, 3H), 1.51(t, 3H). |
| 2-159 | δ 8.41(d, 1H), 8.06-8.00(m, 3H), 7.98(s, 1H), 7.79(q, 1H), 7.63(d, 1H), 7.49-7.42(m, 3H), 4.48(q, 2H), 4.00(s, 3H), 2.79(d, 3H), 1.58(t, 3H). |
| 2-162 | δ 8.52(d, 1H), 7.89(d, 1H), 7.67-7.64(m, 1H), 7.39-7.37(m, 1H), 7.14-7.11(m, 1H), 6.98(q, 1H), 5.85(s, 1H), 4.36(q, 2H), 3.06(q, 2H), 2.81(d, 3H), 1.48(t, 3H), 1.26(t, 3H). |
| 2-163 | δ 8.47(d, 1H), 7.77(q, 1H), 7.60-7.58(m, 1H), 7.36-7.33(m, 1H), 7.21(d, 1H), 7.11-7.08(m, 1H), 5.84(s, 1H), 4.25(q, 2H), 3.03-2.93(m, 1H), 2.77(d, 3H), 1.33-1.23(m, 12H). |
| 2-171 | δ 8.41(d, 1H), 7.62(dd, 1H), 7.57(q, 1H), 7.37(dd, 1H), 7.12(dd, 1H), 7.10(d, 1H), 6.92(d, 1H), 5.70(s, 2H), 4.00(s, 3H), 2.78(d, 3H), 2.46(d, 3H). |

**[Table 24]**

| Table 4 (continued) | |
|---|---|
| Compound No. | ¹H-NMR data (CDCl₃/TMS, ppm) |
| 2-173 | δ 8.37(d, 1H), 8.02(d, 2H), 7.50-7.40(m, 4H), 6.99(d, 1H), 6.24(s, 2H), 5.93(s, 1H), 4.49(q, 2H), 2.77(d, 3H), 2.32(s, 3H), 2.23(s, 3H), 1.56(t, 3H). |
| 2-174 | δ 8.60(d, 1H), 8.19(d, 1H), 8.06-7.97(m, 2H), 7.50-7.40(m, 3H), 7.35-7.28(m, 1H), 4.40(q, 2H), 2.84-2.81(m, 6H), 2.44(d, 3H), 1.49(t, 3H). |
| 2-175 | δ 8.40(d, 1H), 8.10-8.05(m, 1H), 7.63(q, 1H), 7.42-7.37(m, 1H), 7.24-7.16(m, 2H), 7.00(d, 1H), 6.24(s, 2H), 5.93(s, 1H), 2.76(d, 3H), 2.32(s, 3H), 2.23(s, 3H), 1.56(t, 3H). |
| 2-176 | δ 8.61(d, 1H), 8.17(d, 3H), 8.11-8.06(m, 1H), 7.51(q, 1H), 7.45-7.38(m, 1H), 7.26-7.17(m, 2H), 4.46(q, 2H), 2.83-2.80(m, 6H), 2.45(s, 3H), 1.50(t, 3H). |
| 2-177 | δ 8.36(d, 1H), 7.66-7.62(m, 1H), 7.37(d, 1H), 7.14-7.10(m, 1H), 7.00(d, 1H), 6.23(s, 2H), 5.92(s, 1H), 4.47(q, 2H), 2.77(d, 3H), 2.32(s, 3H), 2.23(s, 3H), 1.54(t, 3H). |
| 2-178 | δ 8.63-8.57(m, 1H), 8.23-8.17(m, 1H), 7.69-7.62(m, 1H), 7.43-7.36(m, 1H), 7.30-7.25(m, 1H), 7.18-7.09(m, 2H), 4.43-4.33(m, 2H), 2.88-2.70(m, 6H), 2.49-2.44(m, 3H), 1.49(m, 3H). |
| 2-180 | δ 8.38(d, 1H), 7.88(q, 1H), 7.06(d, 1H), 6.92(s, 1H), 5.70(s, 2H), 3.90(s, 3H), 2.75-2.71(m, 5H), 2.46(d, 3H), 1.79(m, 2H), 1.01(t, 3H). |
| 2-182 | δ 8.63(d, 1H), 8.19(d, 1H), 7.83-7.78(m, 1H), 7.61-7.56(m, 1H), 7.52(q, 1H), 7.32(t, 1H), 4.14(s, 3H), 2.87(s, 3H), 2.76(d, 3H), 2.45(s, 3H). |
| 2-183 | δ 8.62(d, 1H), 8.23-8.16(m, 2H), 8.14-8.10(m, 1H), 7.61-7.56(m, 2H), 7.33(q, 1H), 6.72(t, 1H), 4.09(s, 1H), 2.85-2.82(m, 6H), 2.45(s, 3H). |
| 2-184 | δ 8.63(d, 1H), 8.19(d, 1H), 7.83-7.78(m, 1H), 7.61-7.56(m, 1H), 7.52(q, 1H), 7.32(t, 1H), 4.14(s, 3H), 2.87(s, 3H), 2.76(d, 3H), 2.45(s, 3H). |
| 2-185 | δ 8.60(d, 1H), 8.19(d, 1H), 7.89-7.53(m, 1H), 7.62-7.59(m, 1H), 7.49(q, 1H), 7.43-7.40(m, 1H), 4.06(s, 3H), 2.83-2.80(m, 6H), 2.44(s, 3H). |

**[Table 25]**

| Table 4 (continued) | |
|---|---|
| Compound No. | ¹H-NMR data (CDCl₃/TMS, ppm) |
| 2-186 | δ 8.72(s, 1H), 8.61(d, 1H), 8.21(d, 1H), 7.19(q, 1H), 4.08(s, 3H), 2.87-2.78(m, 9H), 2.45(s, 3H). |
| 2-187 | δ 8.56(d, 1H), 8.14(d, 1H), 7.57(q, 1H), 4.31(q, 2H), 3.17-3.10(m, 1H), 2.83(s, 3H), 2.76(d, 3H), 2.45(s, 3H), 1.43-1.35(m, 9H). |
| 2-188 | δ 8.56(d, 1H), 8.14(d, 1H), 7.57(q, 1H), 3.96(s, 3H), 2.81(s, 3H), 2.74(d, 3H), 2.44(s, 3H), 2.12-2.06(m, 1H), 1.08-1.00(m, 2H), 0.99-0.92(m, 2H). |
| 2-198 | δ 8.40(d, 1H), 7.60(dd, 1H), 7.54(q, 1H), 7.36(dd, 1H), 7.30(t, 2H), 7.11(dd, 1H), 7.09(d, 1H), 6.53(q, 1H), 3.83(s, 3H), 2.77(d, 3H), 1.84(d, 3H). |
| 3-1 | δ 8.54(d, 1H), 8.40(d, 1H), 7.92-7.86(m, 2H), 7.49-7.37(m, 3H), 6.59(s, 1H), 6.36(q, 1H), 2.77(d, 3H), 2.71(s, 3H), 2.55(s, 3H), 2.45(s, 3H). |
| 3-2 | δ 8.63(d, 1H), 8.26-8.20(m, 2H), 7.52-7.48(m, 1H), 7.45-7.36(m, 2H), 6.66(q, 1H), 2.83(s, 3H), 2.77(d, 3H), 2.66(s, 3H), 2.45(s, 3H). |
| 3-3 | δ 8.67(d, 1H), 8.37(d, 1H), 8.27-8.23(m, 1H), 8.02-7.98(m, 1H), 7.56-7.48(m, 2H), 7.44-7.36(m, 4H), 6.70(q, 1H), 2.96(s, 3H), 2.78(d, 3H), 2.69(s, 3H). |
| 3-4 | δ 8.62(d, 1H), 8.14(d, 1H), 6.77(s, 1H), 6.59(q, 1H), 4.39(q, 2H), 2.82-2.80(m, 6H), 2.47(s, 3H), 2.44(s, 3H), 1.40(t, 3H). |
| 3-5 | δ 8.57(d, 1H), 8.09(d, 1H), 6.89(q, 1H), 5.88(s, 1H), 2.85(s, 3H), 2.75(d, 3H), 2.43(s, 3H), 2.28(s, 3H), 1.97-1.91(m, 1H), 0.91-0.82(m, 2H), 0.76-0.70(m, 2H). |
| 3-6 | δ 8.66(d, 1H), 8.34(d, 1H), 7.88(dd, 1H), 7.72(dd, 1H), 7.44(dd, 1H), 7.41(dd, 1H), 7.17-7.14(m, 2H), 6.20(q, 1H), 2.91(s, 3H), 2.84(d, 3H), 2.64(s, 3H). |
| 3-7 | δ 8.62(d, 1H), 8.21(d, 1H), 7.72(dd, 1H), 7.41(dd, 1H), 7.14(dd, 1H), 6.19(q, 1H), 2.83(d, 3H), 2.82(s, 3H), 2.62(s, 3H), 2.41(s, 3H). |
| 3-8 | δ 8.62(d, 1H), 8.21(d, 1H), 7.73(dd, 1H), 7.40(dd, 1H), 7.14(dd, 1H), 6.14(q, 1H), 2.93(q, 2H), 2.82(d, 3H), 2.81(s, 3H), 2.44(s, 3H), 1.33(t, 3H). |

**[Table 26]**

| Table 4 (continued) | |
|---|---|
| Compound No. | ¹H-NMR data (CDCl₃/TMS, ppm) |
| 3-9 | δ 8.65(d, 1H), 8.32(d, 1H), 7.81(dd, 1H), 7.75(dd, 1H), 7.44(dd, 1H), 7.40(dd, 1H), 7.16-7.13(m, 2H), 6.10(q, 1H), 3.28(q, 3H), 2.93(q, 2H), 2.83(d, 3H), 1.40(t, 3H), 1.34(t, 3H). |
| 3-11 | δ 9.02(s, 1H), 8.92(s, 1H), 8.70(d, 1H), 8.06(d, 1H), 7.14(q, 1H), 6.24-6.22(m, 2H), 5.50-5.45(m, 2H), 2.81(d, 3H), 2.25(s, 3H), 2.22(s, 3H). |
| 3-12 | δ 8.99(s, 1H), 8.83(s, 1H), 8.67(d, 1H), 8.00(d, 1H), 7.23(q, 1H), 2.93-2.82(m, 2H), 2.80(d, 3H), 2.19-2.07(m, 4H), 1.89-1.83(m, 4H), 1.78-1.73(m, 2H), 1.69-1.29(m, 10H). |
| 3-13 | δ 8.95(s, 1H), 8.66(d, 1H), 8.11(d, 1H), 7.72(dd, 1H), 7.41(dd, 1H), 7.15(dd, 1H), 6.37(q, 1H), 2.92-2.85(m, 1H), 2.83(d, 3H), 2.67(s, 3H), 2.11-2.07(m, 2H), 1.89-1.84(m, 2H), 1.69-1.59(m, 2H), 1.50-1.30(m, 4H). |
| 3-14 | δ 8.99(s, 1H), 8.67(d, 1H), 8.15(d, 1H), 7.72(dd, 1H), 7.41(dd, 1H), 7.15(dd, 1H), 6.37(q, 1H), 6.22(s, 1H), 5.46-5.45(m, 1H), 2.84(d, 3H), 2.67(s, 3H), 2.26(s, 3H). |
| 3-15 | δ 8.98(s, 1H), 8.84(s, 1H), 8.68(d, 1H), 7.99(d, 1H), 7.07(q, 1H), 7.00-6.91(m, 2H), 6.51-6.44(m, 2H), 2.80(d, 3H), 1.99(d, 3H), 1.97(d, 3H). |
| 3-16 | δ 8.61(d, 1H), 8.38(d, 1H), 8.20(d, 1H), 8.02(d, 1H), 6.79(q, 1H), 6.58(d, 1H), 6.56(d, 1H), 2.81(d, 3H). |
| 3-17 | δ 8.62(d, 1H), 8.32(d, 1H), 6.57(s, 1H), 6.51(s, 1H), 5.84(q, 1H), 2.78(d, 3H), 2.70(s, 3H), 2.42(s, 3H). |
| 3-18 | δ 8.65(d, 1H), 8.32(d, 1H), 7.72(dd, 1H), 7.41(dd, 1H), 7.14(dd, 1H), 6.58(s, 1H), 6.17(q, 1H), 2.83(d, 3H), 2.71(s, 3H), 2.61(s, 3H). |
| 3-19 | δ 8.63(d, 1H), 8.34(d, 1H), 8.21-8.18(m, 2H), 7.50-7.46(m, 3H), 6.36(q, 1H), 3.30(q, 2H), 2.78(d, 3H), 2.55(s, 3H), 2.46(s, 3H), 1.43(t, 3H). |
| 3-25 | δ 8.57(d, 1H), 8.15(d, 1H), 7.63-7.61(m, 1H), 7.48-7.45(m, 1H), 7.33-7.30(m, 2H), 7.17(q, 1H), 6.63(s, 1H), 3.88(s, 2H), 3.48(s, 3H), 2.70(d, 3H), 2.55(s, 3H), 1.54(s, 6H). |
| 3-27 | δ 8.57(d, 1H), 8.45(d, 1H), 8.18-8.16(m, 1H), 7.51-7.49(m, 1H), 7.43-7.36(m, 2H), 6.75(q, 1H), 5.70(brs, 1H), 3.87(brs, 1H), 3.95(brs, 1H), 2.74(d, 3H), 2.66(s, 3H), 1.38(s, 6H). |
| 3-28 | δ 8.55(d, 1H), 8.20-8.18(m, 1H), 8.18(d, 1H), 7.51-7.48(m, 1H), 7.42-7.35(m, 2H), 6.80(q, 1H), 3.96(t, 2H), 3.57(t, 2H), 3.45(q, 2H), 2.76(d, 3H), 2.66(s, 3H), 1.15(t, 3H). |

**[Table 27]**

| Table 4 (continued) | |
|---|---|
| Compound No. | ¹H-NMR data (CDCl₃/TMS, ppm) |
| 3-29 | δ 8.60(d, 1H), 8.31(d, 1H), 8.11-8.08(m, 1H), 7.50-7.47(m, 1H), 7.41-7.34(m, 2H), 6.87(q, 1H), 4.24(s, 2H), 2.75(d, 3H), 2.71(s, 3H), 1.44(s, 6H). |
| 3-31 | δ 8.61(d, 1H), 8.31(d, 1H), 8.12-8.09(m, 1H), 7.50-7.47(m, 1H), 7.41-7.34(m, 2H), 6.92(q, 1H), 4.67(dd, 1H), 4.63-4.52(m, 1H), 4.19(dd, 1H), 2.75(d, 3H), 2.72-2.68(m, 5H), 2.14(s, 3H), 2.11-2.02(m, 1H), 1.97-1.88(m, 1H). |
| 3-32 | δ 8.56(d, 1H), 8.21(d, 1H), 7.63-7.60(m, 1H), 7.48-7.45(m, 2H), 7.34-7.28(m, 2H), 7.13(q, 1H), 6.63(s, 1H), 4.63(dd, 1H), 4.48-4.40(m, 1H), 4.11(dd, 1H), 2.70(d, 3H), 2.53(s, 3H), 1.91-1.81(m, 1H), 1.78-1.72(m, 1H), 1.47-1.40(m, 1H), 0.99(t, 6H). |
| 3-33 | δ 8.40(d, 1H), 7.97-7.95(m, 2H), 7.53-7.51(m, 1H), 7.38-7.36(m, 2H), 7.03(q, 1H), 4.03-4.00(m, 2H), 3.65(t, 2H), 2.98(s, 3H), 2.82(d, 3H), 1.46(s, 9H). |
| 3-34 | δ 8.60(d, 1H), 8.25(d, 1H), 8.12-8.09(m, 1H), 7.50-7.47(m, 1H), 7.41-7.34(m, 2H), 6.94(q, 1H), 3.89(s, 2H), 2.75(d, 3H), 2.72(s, 3H), 1.54(s, 6H). |
| 3-35 | δ 8.42(d, 1H), 7.72-7.70(m, 2H), 7.45-7.35(m, 3H), 7.00(d, 1H), 6.60(s, 1H), 6.49(q, 1H), 6.22(s, 2H), 2.75(d, 3H), 2.45(s, 6H), 2.34(s, 3H). |
| 3-36 | δ 8.38(d, 1H), 7.73-7.70(m, 2H), 7.45-7.35(m, 3H), 6.96(d, 1H), 6.62(q, 1H), 6.60(s, 1H), 6.19(s, 2H), 5.91(s, 1H), 2.76(d, 3H), 2.48(s, 3H), 2.30(s, 3H), 2.24(s, 3H). |
| 3-37 | δ 8.33(d, 1H), 7.71-7.68(m, 2H), 7.45-7.33(m, 3H), 6.96(d, 1H), 6.67(q, 1H), 6.57(s, 1H), 4.46-4.06(m, 4H), 2.75(d, 3H), 2.45(s, 3H), 2.17-2.01(m, 3H), 1.80-1.70(m, 1H), 1.28-1.26(m, 3H). |
| 3-38 | δ 8.35(d, 1H), 7.71-7.69(m, 2H), 7.44-7.33(m, 3H), 6.95(d, 1H), 6.68(q, 1H), 6.57(s, 1H), 4.52-4.46(m, 1H), 4.41-4.39(m, 2H), 4.17-4.14(m, 1H), 3.88-3.84(m, 1H), 2.75(d, 3H), 2.46(s, 3H), 1.45(s, 3H), 1.40(s, 3H). |
| 3-39 | δ 8.36(d, 1H), 7.71-7.68(m, 2H), 7.47-7.34(m, 3H), 6.93(d, 1H), 6.68(q, 1H), 6.57(s, 1H), 4.95-4.88(m, 1H), 4.41(dd, 2H), 3.11(dd, 1H), 2.87(dd, 1H), 2.83(d, 3H), 2.79-2.72(m, 1H), 2.45(s, 3H), 1.19(d, 6H). |
| 2-199 | δ 8.59(d, 1H), 8.10(d, 1H), 7.88-7.84(m, 2H), 7.54-7.48(m, 1H), 7.48-7.43(m, 2H), 7.38-7.32(m, 1H), 4.31(q, 2H), 2.83(s, 3H), 2.79(d, 3H), 2.45(s, 3H), 1.30(t, 3H) |

The agricultural or horticultural herbicide comprising the compound represented by the general formula (I) of the present invention or a salt thereof as an active ingredient has excellent physical properties suitable for various kinds of labor-saving application methods, excellent systemic activity, and excellent environment safety such as suitable soil residual characteristics.

The agricultural or horticultural herbicide comprising the compound represented by the general formula (I) of the present invention or a salt thereof as an active ingredient is highly safe for animals including humans.

Examples of useful plants for which the compound represented by the general formula (I) of the present invention or a salt thereof can be used include, but are not particularly limited to, cereals (e.g., rice, barley, wheat, rye, oats, corn, etc.), legumes (e.g., soybeans, azuki beans, broad beans, green peas, kidney beans, peanuts, etc.), fruit trees and fruits (e.g., apples, citrus fruits, pears, grapes, peaches, plums, cherries, walnuts, chestnuts, almonds, bananas, etc.), leaf and fruit vegetables (e.g., cabbages, tomatoes, spinach, broccoli, lettuce, onions, green onions (chives and Welsh onions), green peppers, eggplants, strawberries, pepper crops, okra, Chinese chives, etc.), root vegetables (e.g., carrots, potatoes, sweet potatoes, taros, Japanese radishes, turnips, lotus roots, burdock roots, garlic, Chinese scallions, etc.), crops for processing (e.g., cotton, hemp, beet, hops, sugarcane, sugar beet, olives, rubber, coffee, tobacco, tea, etc.), gourds (e.g., Japanese pumpkins, cucumbers, watermelons, oriental sweet melons, melons, etc.), pasture grass (e.g., orchardgrass, sorghum, timothy, clover, alfalfa, etc.), lawn grass (e.g., Korean lawn grass, bent grass, etc.), spice and aromatic crops and ornamental crops (e.g., lavender, rosemary, thyme, parsley, pepper, ginger, etc.), ornamental flowering plants (e.g., chrysanthemum, rose, carnation, orchid, tulip, lily, etc.), garden trees (e.g., ginkgo trees, cherry trees, Japanese aucuba, etc.) and forest trees (e.g., *Abies sachalinensis*, *Picea jezoensis*, pine, yellow cedar, Japanese cedar, hinoki cypress, eucalyptus, etc.).

The above-mentioned "plants" also include plants provided with herbicide tolerance by a classical breeding technique or a gene recombination technique. Examples of such herbicide tolerance include tolerance to HPPD inhibitors, such as isoxaflutole; ALS inhibitors, such as imazethapyr and thifensulfuron-methyl; EPSP synthase inhibitors, such as glyphosate; glutamine synthetase inhibitors, such as glufosinate; acetyl-CoA carboxylase inhibitors, such as sethoxydim; or other herbicides, such as bromoxynil, dicamba and 2,4-D.

Examples of the plants provided with herbicide tolerance by a classical breeding technique include varieties of rapeseed, wheat, sunflower and rice tolerant to the imidazolinone family of ALS-inhibiting herbicides such as imazethapyr. Such a rice variety is sold under the trade name of Clearfield (registered trademark). Also included is a variety of soybean provided with tolerance to the sulfonyl urea family of ALS-inhibiting herbicides such as thifensulfuron-methyl by a classical breeding technique, and this is sold under the trade name of STS soybean. Also included are plants provided with tolerance to acetyl-CoA carboxylase inhibitors such as trione oxime herbicides and aryloxy phenoxy propionic acid herbicides by a classical breeding technique, for example, SR corn and the like.

Plants provided with tolerance to acetyl-CoA carboxylase inhibitors are described in Proc. Natl. Acad. Sci. USA, 87, 7175-7179 (1990), and the like. Further, acetyl-CoA carboxylase mutants resistant to acetyl-CoA carboxylase inhibitors are reported in Weed Science, 53, 728-746 (2005), and the like, and by introducing the gene of such an acetyl-CoA carboxylase mutant into plants by a gene recombination technique, or introducing a resistance-conferring mutation into acetyl-CoA carboxylase of plants, plants tolerant to acetyl-CoA carboxylase inhibitors can be engineered. Alternatively, by introducing a nucleic acid causing base substitution mutation into plant cells (a typical example of this technique is chimeraplasty technique (Gura T. 1999. Repairing the Genome's Spelling Mistakes. Science 285: 316-318.)) to allow site-specific substitution mutation in the amino acids encoded by an acetyl-CoA carboxylase gene, an ALS gene or the like of plants, plants tolerant to acetyl-CoA carboxylase inhibitors, ALS inhibitors or the like can be engineered. The compound represented by the general formula (I) of the present invention or a salt thereof can be used for these plants as well. The compound represented by the general formula (I) of the present invention does not do damage to these useful plants.

Further, exemplary toxins expressed in genetically modified plants include insecticidal proteins of *Bacillus cereus* or *Bacillus popilliae; Bacillus thuringiensis* δ-endotoxins, such as Cry1Ab, CrylAc, CrylF, CrylFa2, Cry2Ab, Cry3A, Cry3Bb1 and Cry9C, and other insecticidal proteins, such as VIP1, VIP2, VIP3 and VIP3A; nematode insecticidal proteins; toxins produced by animals, such as scorpion toxins, spider toxins, bee toxins and insect-specific neurotoxins; toxins of filamentous fungi; plant lectins; agglutinin; protease inhibitors, such as trypsin inhibitors, serine protease inhibitors, patatin, cystatin and papain inhibitors; ribosome inactivating proteins (RIP), such as ricin, maize RIP, abrin, luffin, saporin and bryodin; steroid metabolizing enzymes, such as 3-hydroxy steroid oxidase, ecdysteroid-UDP-glucosyltransferase and cholesterol oxidase; ecdysone inhibitors; HMG-CoA reductase; ion channel inhibitors, such as sodium channel inhibitors and calcium channel inhibitors; juvenile hormone esterase; diuretic hormone receptors; stilbene synthase; bibenzyl synthase; chitinase; and glucanase.

Also included are hybrid toxins, partially deficient toxins and modified toxins derived from the following: δ-endotoxin proteins such as Cry1Ab, CrylAc, CrylF, CrylFa2, Cry2Ab, Cry3A, Cry3Bb1, Cry9C, Cry34Ab and Cry35Ab, and other insecticidal proteins such as VIP1, VIP2, VIP3 and VIP3A. The hybrid toxin can be produced by combining some domains of these proteins differently from the original combination in nature with the use of a recombination technique. As the partially deficient toxin, a Cry1Ab toxin in which a part of the amino acid sequence is deleted is known. In the modified toxin, one or more amino acids of a naturally occurring toxin are substituted.

Examples of the foregoing toxins and genetically modified plants capable of synthesizing these toxins are described in EP0374753, WO93/07278, WO95/34656, EP0427529, EP0451878, WO03/052073, etc.

Due to the toxins contained in such genetically modified plants, the plants exhibit resistance to pests, in particular, Coleopteran insect pests, Hemipteran insect pests, Dipteran insect pests, Lepidopteran insect pests and nematodes. The above-described technologies and the agricultural or horticultural herbicide of the present invention can be used in combination or used systematically.

Examples of the weed that can be controlled by the compound represented by the general formula (I) of the present invention or a salt thereof include dicotyledonous genera such as *Ipomoea*, *Lindernia*, *Sesbania*, *Abutilon*, *Matricaria*, *Rorippa*, *Urtica*, *Lamium*, *Xanthium*, *Sinapis*, *Rotala*, *Veronica*, *Papaver*, *Chenopodium*, *Trifolium*, *Portulaca*, *Viola*, *Pharbitis*, *Galeopsis*, *Datura*, *Solanum*, *Capsella*, *Cirsium*, *Sonchus*, *Galinsoga*, *Stellaria*, *Senecio*, *Amaranthus*, *Ambrosia*, *Kochia*, *Lamium*, *Leipidium*, *Polygonum*, *Galium*, *Centaurea*, and *Artemisia*.

Examples of the weed that can be controlled by the compound represented by the general formula (I) of the present invention or a salt thereof include monocotyledonous genera such as *Leptochloa*, *Phleum*, *Poa*, *Bolboschoenus*, *Festuca*, *Setaria*, *Eleusine*, *Sagittaria*, *Agropyron*, *Ischaemum*, *Cyperus*, *Avena*, *Bromus*, *Panicum*, *Cynodon*, *Monochoria*, *Alopecurus*, *Paspalum*, *Commelina*, *Fimbristylis*, *Lolium*, *Brachiaria*, *Agrostis*, *Eleocharis*, *Echinochloa esculenta*, *Scirpus*, *Schoenoplectus*, *Digitaria*, and *Sorghum*.

Other examples of the weed that can be controlled by the compound represented by the general formula (I) of the present invention or a salt thereof include *Spirogyra* sp., *Amaranthus retroflexus*, *Amaranthus viridis*, *Setaria faberi*, *Leersia japonica*, *Leptochloa chinensis*, *Lindernia angustifolia*, *Lindernia procumbens*, *Dopatrium junceum*, *Ipomoea hederacea*, *Lindernia dubia*, *Sida spinosa*, *Polygonum pensylvanicum*, *Sesbania exaltata*, *Geranium carolinense*, *Chenopodium ambrosioides*, *Conyza bonariensis*, *Setaria italica*, *Amaranthus powellii*, *Polygonum cuspidatum*, *Abutilon theophrasti*, *Matricaria perforata*, *Polygonum longisetum*, *Veronica polita*, *Echinochloa crus-galli*, *Amaranthus lividus*, *Solanum nigrum*, *Schoenoplectus juncoides* (Roxb.) Palla, *Bromus catharticus*, *Murdannia keisak*, *Bolboschoenus fluviatilis*, *Scirpus maritimus*, *Bromus tectorum*, *Sagittaria pygmaea* Miq, *Rumex obtusifolius*, *Leersia oryzoides* (L.) Sw., *Setaria viridis*, *Cassia obtusifolia*, *Conyza sumatrensis*, *Veronica persica*, *Spirodela polyrhiza*, *Xanthium canadens*, *Coreopsis lanceolata*, *Panicum dichotomiflorum*, *Asclepias syriaca*, *Euphorbia maculata*, *Plantago asiatica*, *Rudbeckia laciniata*, *Amaranthus palmeri*, *Avena sativa*, *Xanthium strumarium*, *Avena sterilis*, *Eleusine indica*, *Sagittaria trifolia*, *Erodium cicutarium*, *Cerastium glomeratum*, *Matricaria matricarioides*, *Matricaria chamomilla*, *Vicia angustifolia*, *Bromus secalinus*, *Avena fatua*, *Rotala indica* Koehne, *Rumex japonicus*, *Paspalum distichum*, *Bromus remotiflorus*, *Cyperus esculentus*, *Galium kinuta*, *Setaria glauca*, *Pueraria lobata*, *Eleocharis kuroguwai* Ohwi, *Sagittaria trifolia* Caerulea, *Ambrosia trifida*, *Hydrilla verticillata*, *Bolboschoenus maritimus* (L.) Palla, *Chrysanthemum segetum*, *Cyperus iria*, *Monochoria vaginalis*, *Echinochloa colona*, *Alisma plantago-aquatica*, *Oryza sativa*, *Polygonum lapathifolium*, *Eleusine coracana*, *Schoenoplectus nipponicus*, *Cyperus malaccensis*, *Agropyron repens*, *Sorghum vulgare*, *Apera spica-venti*, *Chenopodium album*, *Trifolium repens*, *Datura stramonium*, *Equisetum arvense*, *Poa annua*, *Bromus japonicus*, *Alopecurus aequalis*, *Portulaca oleracea*, *Solidago altissima*, *Sorghum halepense*, *Brassica juncea*, *Taraxacum officinale*, *Convolvulus arvensis*, *Oenanthe javanica*, *Polygonum convolvulus*, *Echinochloa oryzicola* Vasing, *Ischaemum rugosum*, *Veronica arvensis*, *Cyperus difformis* L., *Amaranthus rudis*, *Phleum pratense*, *Ludwigia prostrata* Roxburgh, *Commelina communis*, *Panicum texanum*, *Euphorbia helioscopia*, *Festuca parvigluma*, *Rumex crispus*, *Capsella bursa-pastoris*, *Euphorbia pseudochamaesyce*, *Brachiaria plantaginea*, *Lolium multiflorum*, *Cirsium japonicum*, *Alopecurus myosuroides*, *Sinapis arvensis*, *Senecio vulgaris*, *Galinsoga ciliata*, *Amaranthus tricolor*, *Stellaria media*, *Cyperus papyrus*, *Cyperus rotundus*, *Amaranthus spinosus*, *Polygonum persicaria*, *Senecio cannabifolius*, *Cyperus flaccidus*, *Papaver rhoeas*, *Helianthus annuus*, *Lamium purpureum*, *Kyllinga gracillima*, *Ammannia multiflora*, *Erigeron canadensis*, *Potamogeton distinctus* A. Benn, *Amaranthus tuberculatus*, *Viola arvensis*, *Cirsium purpuratum*, *Ambrosia artemisiifolia*, *Schoenoplectus tabernaemontani*, *Veronica hederaefolia*, *Alopecurus myosuroides*, *Desmodium tortuosum*, *Plantago lanceolata*, *Kochia scoparia*, *Lolium rigidum*, *Ammannia coccinea*, *Lolium perenne*, *Scirpus juncoides* Roxburgh, *Lamium amplexicaule*, *Najas graminea*, *Amaranthus hybridus*, *Eleocharis acicularis* L., *Portulaca grandiflora*, *Ipomoea lacunosa*, *Ipomoea purpurea*, *Ipomoea hederacea* var. *integriuscula*, *Commelina bengharensis*, *Monochoria korsakowii*, *Cyperus serotinus* Rottboel, *Elatine triandra* Schk, *Digitaria ciliaris*, *Digitaria sanguinalis*, *Sorghum bicolor*, *Galium aparine*, *Artemisia princeps*, *Viola tricolor*, *Raphanus raphanistrum*, *Myosotis arvensis*, and *Alisma canaliculatum*. The compound represented by the general formula (I) of the present invention or a salt thereof inhibits growth of these weeds.

When the compound represented by the general formula (I) of the present invention or a salt thereof is used, it is commonly formulated into a convenient form for application, which is prepared by the usual method for preparing agrochemical formulations.

That is, the compound represented by the general formula (I) of the present invention or a salt thereof and an appropriate inactive carrier, and if needed an adjuvant, are blended at an appropriate ratio, and through the step of dissolution, separation, suspension, mixing, impregnation, adsorption and/or adhesion, are formulated into an appropriate form for application, such as a suspension concentrate, an emulsifiable concentrate, a soluble concentrate, a wettable powder, a water-dispersible granule, a granule, a dust, a tablet and a pack.

The composition (agricultural or horticultural herbicide) of the present invention can optionally contain an additive usually used for agrochemical formulations or agricultural or horticultural herbicides in addition to the active ingredient. Examples of the additive include carriers such as solid or liquid carriers, surfactants, dispersants, wetting agents, binders, tackifiers, thickeners, colorants, spreaders, sticking/spreading agents, antifreezing agents, anti-caking agents, disintegrants and stabilizing agents. If needed, preservatives, plant fragments, etc. may also be used as the additive. One of these additives may be used alone, and also two or more of them may be used in combination.

Examples of the solid carrier include natural minerals, such as quartz, clay, kaolinite, pyrophyllite, sericite, talc, bentonite, acid clay, attapulgite, zeolite and diatomite; inorganic salts, such as calcium carbonate, ammonium sulfate, sodium sulfate and potassium chloride; organic solid carriers, such as synthetic silicic acid, synthetic silicates, starch, cellulose and plant powders (for example, sawdust, coconut shell, corn cob, tobacco stalk, etc.); plastics carriers, such as polyethylene, polypropylene and polyvinylidene chloride; urea; hollow inorganic materials; hollow plastic materials; and fumed silica (white carbon). One of these may be used alone, and also two or more of them may be used in combination.

Examples of the liquid carrier include alcohols including monohydric alcohols, such as methanol, ethanol, propanol, isopropanol and butanol, and polyhydric alcohols, such as ethylene glycol, diethylene glycol, propylene glycol, hexylene glycol, polyethylene glycol, polypropylene glycol and glycerin; polyol compounds, such as propylene glycol ether; ketones, such as acetone, methyl ethyl ketone, methyl isobutyl ketone, diisobutyl ketone and cyclohexanone; ethers, such as ethyl ether, dioxane, ethylene glycol monoethyl ether, dipropyl ether and tetrahydrofuran; aliphatic hydrocarbons, such as normal paraffin, naphthene, isoparaffin, kerosene and mineral oil; aromatic hydrocarbons, such as benzene, toluene, xylene, solvent naphtha and alkyl naphthalene; halogenated hydrocarbons, such as dichloromethane, chloroform and carbon tetrachloride; esters, such as ethyl acetate, diisopropyl phthalate, dibutyl phthalate, dioctyl phthalate and dimethyl adipate; lactones, such as gamma-butyrolactone; amides, such as N,N-dimethylformamide, N,N-diethylformamide, N,N-dimethylacetamide and N-alkyl pyrrolidinone; nitriles, such as acetonitrile; sulfur compounds, such as dimethyl sulfoxide; vegetable oils, such as soybean oil, rapeseed oil, cotton seed oil and castor oil; and water. One of these may be used alone, and also two or more of them may be used in combination.

Exemplary surfactants used as a dispersant, a wetting agent, a spreader, a sticking/spreading agent, etc. include nonionic surfactants, such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkyl aryl ether, polyoxyethylene alkyl phenyl ether, polyoxyethylene dialkyl phenyl ether, polyoxyethylene alkyl phenyl ether-formaldehyde condensates, polyoxyethylene-polyoxypropylene block copolymers, polystyrene-polyoxyethylene block polymers, alkyl polyoxyethylene-polypropylene block copolymer ether, polyoxyethylene alkylamine, polyoxyethylene fatty acid amide, polyoxyethylene fatty acid bis(phenyl ether), polyalkylene benzyl phenyl ether, polyoxyalkylene styryl phenyl ether, acetylene diol, polyoxyalkylene-added acetylene diol, polyoxyethylene ether-type silicone, ester-type silicone, fluorosurfactants, polyoxyethylene castor oil and polyoxyethylene hydrogenated castor oil; anionic surfactants, such as alkyl sulfates, polyoxyethylene alkyl ether sulfates, polyoxyethylene alkyl phenyl ether sulfates, polyoxyethylene styryl phenyl ether sulfates, alkylbenzene sulfonates, alkylaryl sulfonates, lignosulfonates, alkyl sulfosuccinates, naphthalene sulfonates, alkylnaphthalene sulfonates, salts of naphthalenesulfonic acid-formaldehyde condensates, salts of alkylnaphthalenesulfonic acid-formaldehyde condensates, fatty acid salts, polycarboxylic acid salts, polyacrylates, N-methyl-fatty acid sarcosinates, resinates, polyoxyethylene alkyl ether phosphates and polyoxyethylene alkyl phenyl ether phosphates; cationic surfactants including alkyl amine salts, such as lauryl amine hydrochloride, stearyl amine hydrochloride, oleyl amine hydrochloride, stearyl amine acetate, stearyl aminopropyl amine acetate, alkyl trimethyl ammonium chloride and alkyl dimethyl benzalkonium chloride; and amphoteric surfactants, such as amino acid-type or betaine-type amphoteric surfactants. One of these surfactants may be used alone, and also two or more of them may be used in combination.

Examples of the binder or the tackifier include carboxymethyl cellulose or salts thereof, dextrin, soluble starch, xanthan gum, guar gum, sucrose, polyvinyl pyrrolidone, gum arabic, polyvinyl alcohol, polyvinyl acetate, sodium polyacrylate, polyethylene glycols with an average molecular weight of 6,000 to 20,000, polyethylene oxides with an average molecular weight of 100,000 to 5,000,000, phospholipids (for example, cephalin, lecithin, etc.), cellulose powder, dextrin, modified starch, polyaminocarboxylic acid chelating compounds, cross-linked polyvinyl pyrrolidone, maleic acid-styrene copolymers, (meth)acrylic acid copolymers, half esters of polyhydric alcohol polymer and dicarboxylic anhydride, water soluble polystyrene sulfonates, paraffin, terpene, polyamide resins, polyacrylates, polyoxyethylene, waxes, polyvinyl alkyl ether, alkylphenol-formaldehyde condensates and synthetic resin emulsions.

Examples of the thickener include water soluble polymers, such as xanthan gum, guar gum, diutan gum, carboxymethyl cellulose, polyvinyl pyrrolidone, carboxyvinyl polymers, acrylic polymers, starch compounds and polysaccharides; and inorganic fine powders, such as high grade bentonite and fumed silica (white carbon).

Examples of the colorant include inorganic pigments, such as iron oxide, titanium oxide and Prussian blue; and organic dyes, such as alizarin dyes, azo dyes and metal phthalocyanine dyes.

Examples of the antifreezing agent include polyhydric alcohols, such as ethylene glycol, diethylene glycol, propylene glycol and glycerin.

Examples of the adjuvant serving to prevent caking or facilitate disintegration include polysaccharides (starch, alginic acid, mannose, galactose, etc.), polyvinyl pyrrolidone, fumed silica (white carbon), ester gum, petroleum resin, sodium tripolyphosphate, sodium hexametaphosphate, metal stearates, cellulose powder, dextrin, methacrylate copolymers, polyvinyl pyrrolidone, polyaminocarboxylic acid chelating compounds, sulfonated styrene-isobutylene-maleic anhydride copolymers and starch-polyacrylonitrile graft copolymers.

Examples of the stabilizing agent include desiccants, such as zeolite, quicklime and magnesium oxide; antioxidants, such as phenolic compounds, amine compounds, sulfur compounds and phosphoric acid compounds; and ultraviolet absorbers, such as salicylic acid compounds and benzophenone compounds.

Examples of the preservative include potassium sorbate and 1,2-benzothiazolin-3-one.

Further, other adjuvants including functional spreading agents, activity enhancers such as metabolic inhibitors (piperonyl butoxide etc.), antifreezing agents (propylene glycol etc.), antioxidants (BHT etc.) and ultraviolet absorbers can also be used if needed.

The amount of the active ingredient compound in the agricultural or horticultural herbicide of the present invention can be adjusted as needed, and basically, the amount of the active ingredient compound is appropriately selected from the range of 0.01 to 90 parts by weight in 100 parts by weight of the agricultural or horticultural herbicide. For example, in the case where the agricultural or horticultural herbicide is a dust, a granule, an emulsifiable concentrate or a wettable powder, it is suitable that the amount of the active ingredient compound is 0.01 to 50% by weight relative to the total weight of the agricultural or horticultural herbicide.

The application rate of the compound represented by the general formula (I) of the present invention or a salt thereof may vary with various factors, for example, the purpose, the target weed, the growing conditions of crops, the tendency of weed infestation, the weather, the environmental conditions, the formulation, the application method, the application site, the application timing, etc., but basically, the application rate of the active ingredient compound is appropriately selected from the range of 0.001 g to 10 kg, and preferably 0.01 g to 1 kg per 10 ares depending on the purpose.

In order to control weeds, the agricultural or horticultural herbicide comprising the compound represented by the general formula (I) of the present invention or a salt thereof as an active ingredient, with or without appropriate dilution or suspension in water etc., is applied directly to the foliage of weeds in an amount effective for the control of the weeds. In addition to foliar application, seed treatment for useful plants, such as dipping, dust coating and calcium peroxide coating, can be performed. Further, treatment of soil or growing media may also be performed, and examples of such treatment include whole soil incorporation, planting row treatment, bed soil incorporation, plug seedling treatment, planting hole treatment, plant foot treatment, top-dressing, treatment of nursery boxes for paddy rice, and submerged application.

Exemplary methods of seed treatment for useful plants include dipping of seeds in a diluted or undiluted fluid of a liquid or solid formulation for the permeation of agrochemicals into the seeds; mixing or dust coating of seeds with a solid or liquid formulation for the adherence of the formulation onto the surfaces of the seeds; coating of seeds with a mixture of a solid or liquid formulation and an adhesive carrier such as resins and polymers; and application of a solid or liquid formulation to the vicinity of seeds at the same time as seeding.

The term "seed" in the seed treatment refers to a plant body which is in the early stages of cultivation and used for useful plant propagation. The examples include, in addition to a so-called seed, a plant body for vegetative propagation, such as a bulb, a tuber, a seed potato, a bulbil, a propagule, a discoid stem and a stem used for cuttage.

The term "soil" or "growing medium" in the method of the present invention for using an agricultural or horticultural herbicide refers to a support medium for crop cultivation, in particular a support medium which allows crop plants to spread their roots therein, and the materials are not particularly limited as long as they allow useful plants to grow. Examples of the support medium include what is called soils, seedling mats and water, and specific examples of the materials include sand, pumice, vermiculite, diatomite, agar, gelatinous substances, high-molecular-weight substances, rock wool, glass wool, wood chip and bark.

In the case of the application to nursery boxes for paddy rice, the type of the formulation may vary depending on whether the application is performed at the time of seeding, in the greening period, at the time of transplanting, or the like. For example, a dust, a water-dispersible granule, a granule, or the like may be used. Such a formulation can be applied by incorporation into nursery soil. For example, a dust, a water-dispersible granule, a granule, or the like may be incorporated into bed soil, covering soil, or the whole nursery soil. Simply, nursery soil and such a formulation may be alternately layered.

In the application to paddy fields, a solid formulation, such as a jumbo, a pack, a granule and a water-dispersible granule, or a liquid formulation, such as a flowable and an emulsifiable concentrate, is applied usually to flooded paddy fields. In a rice planting period, a suitable formulation, as it is or after mixed with a fertilizer, may be applied onto soil or injected into soil. In addition, an emulsifiable concentrate, a flowable or the like may be applied to the source of water supply for paddy fields, such as a water inlet and an irrigation device. In this case, treatment can be accomplished with the supply of water and thus achieved in a labor-saving manner.

In the case of using spraying equipment, it can be any equipment that is usually used, and examples include tractor mounted boom sprayers, manned helicopters, radio-controlled helicopters, radio-controlled boats, drones, one-shot sprayers, power (manual or automatic) sprayers, carry power sprayers, backpack power sprayers, and hand operated sprayers.

For the expansion of the range of target weeds and the appropriate time for weed control, or for dose reduction, the compound represented by the general formula (I) of the present invention or a salt thereof can be used after mixed with other herbicides, plant growth regulators, phytotoxicity reducers (safeners), soil conditioners, fertilizers, and/or the like. Further, the compound of the present invention or a salt thereof can be used after mixed with agricultural or horticultural insecticides, acaricides, nematicides, microbicides, biopesticides and/or the like depending on the situation. Non-limiting examples of typical compounds are described below.

Exemplary additional agricultural or horticultural insecticides, acaricides and nematicides used for the above-mentioned purposes include 3,5-xylyl methylcarbamate (XMC), fenobucarb (BPMC), Bt toxin-derived insecticidal compounds, CPCBS (chlorfenson), DCIP (dichlorodiisopropyl ether), D-D (1,3-dichloropropene), DDT, NAC, O-4-dimethylsulfamoylphenyl O,O-diethyl phosphorothioate (DSP), O-ethyl O-4-nitrophenyl phenylphosphonothioate (EPN), tripropylisocyanurate (TPIC), acrinathrin, azadirachtin, acynonapyr, azinphos-methyl, acequinocyl, acetamiprid, acetoprole, acephate, abamectin, afidopyropen, avermectin-B, amidoflumet, amitraz, alanycarb, aldicarb, aldoxycarb, aldrin, alpha-endosulfan, alpha-cypermethrin, albendazole, allethrin, isazofos, isamidofos, isoamidofos isoxathion, isocycloseram, isofenphos, isoflualanam, isoprocarb (MIPC), epsilon-metofluthrin, epsilon-momfluorothrin, ivermectin, imicyafos, imidacloprid, imiprothrin, indazapyroxamet, indoxacarb, vadescana, esfenvalerate, ethiofencarb, ethion, ethiprole, etoxazole, ethofenprox, ethoprophos, etrimfos, emamectin, emamectin-benzoate, endosulfan, empenthrin, oxazosulfyl, oxamyl, oxydemeton-methyl, oxydeprofos (ESP), oxibendazole, oxfendazole, potassium oleate, sodium oleate, cadusafos, kappa-bifenthrin, cartap, carbaryl, carbosulfan, carbofuran, gamma-cyhalothrin, xylylcarb, quinalphos, kinoprene, chinomethionat, cloethocarb, clothianidin, clofentezine, chromafenozide, chlorantraniliprole, chlorethoxyfos, chlordimeform, chlordane, chlorpyrifos, chlorpyrifos-methyl, chlorphenapyr, chlorfenson, chlorfenvinphos, chlorfluazuron, chlorobenzilate, chlorobenzoate, chloroprallethrin, kelthane (dicofol), salithion, cyhalodiamide, cyanophos (CYAP), diafenthiuron, diamidafos, cyantraniliprole, theta-cypermethrin, dienochlor, cyetpyrafen, cyenopyrafen, dioxabenzofos, diofenolan, sigma-cypermethrin, cyclaniliprole, dichlofenthion (ECP), cycloprothrin, dichlorvos (DDVP), dicloromezotiaz, disulfoton, dinotefuran, cyhalodiamide, cyhalothrin, cyphenothrin, cyfluthrin, diflubenzuron, cyflumetofen, diflovidazin, cyproflanilide, cyhexatin, cypermethrin, cybenzoxasulfyl, dimethylvinphos, dimethoate, dimpropyridaz, dimefluthrin, silafluofen, cyromazine, spidoxamat, spinetoram, spinosad, spirodiclofen, spirotetramat, spiropidion, spirobudifen, spiromesifen, sulfiflumin, sulfluramid, sulprofos, sulfoxaflor, zeta-cypermethrin, diazinon, tau-fluvalinate, dazomet, thiacloprid, tiapyrachlor, thiamethoxam, tioxazafen, thiodicarb, thiocyclam, thiosultap, thiosultap-sodium, thionazin, thiometon, tiorantraniliprole, deet, dieldrin, tetrachlorantraniliprole, tyclopyrazoflor, tetrachlorvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, doramectin, tralopyril, tralomethrin, transfluthrin, triazamate, triazuron, trichlamide, trichlorphon (DEP), trifluenfuronate, triflumezopyrium, triflumuron, tolfenpyrad, naled (BRP), nicofluprole, nithiazine, nitenpyram, novaluron, noviflumuron, hydroprene, vaniliprole, vamidothion, parathion, parathion-methyl, halfenprox, halofenozide, pioxaniliprole, bistrifluron, bisultap, bisulflufen, hydramethylnon, hydroxy propyl starch, binapacryl, piperflanilide, pyflubumide, bifenazate, bifemetstrobin, bifenthrin, pymetrozine, pyraclofos, pyrafluprole, pyridafenthion, pyridaben, pyridalyl, pyrifluquinazon, pyriprole, pyriproxyfen, pirimicarb, pyrimidifen, pyriminostrobin, pirimiphos-methyl, pyrethrins, fipronil, fenazaquin, fenamiphos, bromopropylate, fenitrothion (MEP), fenoxycarb, fenothiocarb, phenothrin, fenobucarb, fensulfothion, fenthion (MPP), phenthoate (PAP), fenvalerate, fenpyroximate, fenpropathrin, fenbendazole, fenmezoditiaz, fosthiazate, formetanate, butathiofos, buprofezin, furathiocarb, prallethrin, fluacrypyrim, fluazaindolizine, fluazinam, fluazuron, fluensulfone, fluxametamide, fluchlordiniliprole, flucycloxuron, flucythrinate, fluvalinate, flupyradifurone, flufiprole, flupyradifurone, flupyrazofos, flupyrimin, flufenerim, flufenoxystrobin, flufenoxuron, flufenzine, flufenprox, flupyroxystrobin, fluproxyfen, flubrocythrinate, fluhexafon, flubendiamide, flupentiofenox, flumetnicam, flumethrin, flurimfen, prothiofos, protrifenbute, flonicamid, propaphos, propargite (BPPS), profenofos, broflanilide, profluthrin, propoxur (PHC), flometoquin, alpha-bromadiolone, bromopropylate, beta-cyfluthrin, hexaflumuron, hexythiazox, heptafluthrin, heptenophos, permethrin, benclothiaz, bendiocarb, benzpyrimoxan, bensultap, benzoximate, bentioflumin, benfuracarb, phoxim, phosalone, fosthiazate, fosthietan, phosphamidon, phosphocarb, phosmet (PMP), polynactins, formetanate, formothion, phorate, machine oil, malathion, milbemycin, milbemycin-A, milbemectin, mecarbam, mesulfenfos, methomyl, metaldehyde, metaflumizone, methamidophos, metam-ammonium, carbam (metam-sodium), methiocarb, methidathion (DMTP), methylisothiocyanate, methylneodecanamide, methylparathion, metoxadiazone, methoxychlor, methoxyfenozide, metofluthrin, methoprene, metolcarb, meperfluthrin, mevinphos, monocrotophos, monosultap, momfluorothrin, lambda-cyhalothrin, ryanodine, lufenuron, rescalure, resmethrin, lepimectin, rotenone, levamisole hydrochloride, fenbutatin oxide, morantel tartarate, methyl bromide, tricyclohexyltin hydroxide (cyhexatin), calcium cyanamide, calcium polysulfide, sulfur and nicotine-sulfate.

Exemplary agricultural or horticultural microbicides used for the same purposes as above include aureofungin, azaconazole, azithiram, acypetacs, acibenzolar, acibenzolar-S-methyl, azoxystrobin, anilazine, amisulbrom, ampropylfos, ametoctradin, allyl alcohol, aldimorph, amobam, isotianil, isovaledione, isopyrazam, isofetamid, isoflucypram, isoprothiolane, ipconazole, ipfentrifluconazole, ipflufenoquin, iprodione, iprovalicarb, iprobenfos, imazalil, iminoctadine, metam, iminoctadine-albesilate, iminoctadine-triacetate, imibenconazole, inpyrfluxam, uniconazole, uniconazole-P, echlomezole, edifenphos, etaconazole, ethaboxam, ethirimol, etem, ethoxyquin, etridiazole, enestroburin, enoxastrobin, epoxiconazole, oxadixyl, oxathiapiprolin, oxycarboxin, copper-8-quinolinolate, oxytetracycline, copper-oxinate, oxpoconazole, oxpoconazole-fumarate, oxolinic acid, octhilinone, ofurace, orysastrobin, carbam (metam-sodium), kasugamycin, galquin, carbamorph, carpropamid, carbendazim, carboxin, carvone, quinazamid, quinacetol, quinoxyfen, quinofumelin, chinomethionat, quinomethionate, captafol, captan, kiralaxyl, quinconazole, quintozene, guazatine, cufraneb, cuprobam, coumoxystrobin, glyodin, griseofulvin, climbazole, cresol, kresoxim-methyl, chlozolinate, clotrimazole, chlobenthiazone, chloraniformethan, chloranil, chlorquinox, chloropicrin, chlorfenazole, chloroinconazide, chlorodinitronaphthalene, chlorothalonil, chloroneb, salicylanilide, zarilamid, cyazofamid, diethyl pyrocarbonate, diethofencarb, cyclafuramid, diclocymet, dichlozoline, diclobutrazol, cyclobutrifluram, dichlofluanid, cycloheximide, dichlobentiazox, diclomezine, dicloran, dichlorophen, dichlone, disulfiram, ditalimfos, dithianon, diniconazole, diniconazole-M, zineb, dinocap, dinocton, dinosulfon, dinoterbon, dinobuton, dinopenton, dipymetitrone, dipyrithione, diphenylamine, difenoconazole, cyflufenamid, diflumetorim, cyproconazole, cyprodinil, cyprofuram, cypendazole, simeconazole, dimethirimol, dimethomorph, cymoxanil, dimoxystrobin, ziram, silthiofam, streptomycin, spiroxamine, sultropen, sedaxane, zoxamide, dazomet, thiadiazin, tiadinil, thiadifluor, thiabendazole, tioxymid, thiochlorfenphim, thiophanate, thiophanate-methyl, thifluzamide, thicyofen, thioquinox, thiram, decafentin, tecnazene, tecloftalam, tecoram, tetraconazole, debacarb, dehydroacetic acid, tebuconazole, tebufloquin, dodicin, dodine, dodecyl benzensulfonate bis-ethylene diamine copper(II) (DBEDC), dodemorph, drazoxolon, triadimenol, triadimefon, triazbutil, triazoxide, triamiphos, triarimol, trichlamide, triclopyricarb, tricyclazole, triticonazole, tridemorph, tributyltin oxide, triflumizole, trifloxystrobin, triforine, tolylfluanid, tolclofos-methyl, tolprocarb, natamycin, nabam, nitrostyrene, nitrothal-isopropyl, nuarimol, copper nonylphenol sulfonate, halacrinate, validamycin, valifenalate, harpin protein, picarbutrazox, bixafen, picoxystrobin, picobenzamide, pydiflumetofen, bithionol, bitertanol, hydroxyisoxazole, hydroxyisoxazole-potassium, binapacryl, biphenyl, piperalin, hymexazol, pyraoxystrobin, pyracarbolid, pyraclostrobin, pyraziflumid, pyrazophos, pyrapropoyne, pyrametostrobin, pyriofenone, pyridinitril, pydiflumetofen, pyrisoxazole, pyridachlometyl, pyrifenox, pyribencarb, pyriminostrobin, pyrimethanil, pyroxychlor, pyroxyfur, pyroquilon, vinclozolin, ferbam, famoxadone, fenapanil, fenamidone, fenaminosulf, fenaminstrobin, fenarimol, fenitropan, feneptamidoquin, fenoxanil, fenopyramid, ferimzone, ferbam, fentin, fenpiclonil, fenpicoxamid, fenpyrazamine, fenbuconazole, fenfuram, fenpropidin, fenpropimorph, fenhexamid, phthalide, buthiobate, butylamine, bupirimate, fuberidazole, blasticidin-S, furametpyr, furalaxyl, fluacrypyrim, fluazinam, fluindapyr, fluoxastrobin, fluoxapiprolin, fluoxytioconazole, fluotrimazole, fluopicolide, fluopimomide, fluopyram, fluoroimide, furcarbanil, fluxapyroxad, fluquinometoate, fluquinconazole, furconazole, furconazole-cis, fludioxonil, flusilazole, flusulfamide, flutianil, flutolanil, flutriafol, flufenoxadiazam, flufenoxystrobin, furfural, flubeneteram, furmecyclox, flumetylsulforim, flumetover, flumorph, proquinazid, prochloraz, procymidone, prothiocarb, prothioconazole, pronitridine, propamocarb, propiconazole, propineb, furophanate, probenazole, bromuconazole, florylpicoxamid, hexachlorobutadiene, hexaconazole, hexylthiofos, bethoxazin, benalaxyl, benalaxyl-M, benodanil, benomyl, pefurazoate, benquinox, penconazole, benzamorf, pencycuron, benzohydroxamic acid, benzovindiflupyr, bentaluron, benthiazole, benthiavalicarb, benthiavalicarb-isopropyl, penthiopyrad, penflufen, boscalid, phosdiphen, fosetyl, fosetyl-Al, polyoxins, polyoxorim, polycarbamate, folpet, formaldehyde, machine oil, maneb, mancozeb, mandipropamid, mandestrobin, myclozolin, myclobutanil, mildiomycin, milneb, mecarbinzid, methasulfocarb, metazoxolon, metam, metam-sodium, metalaxyl, metalaxyl-M, metarylpicoxamid, metiram, methyl isothiocyanate, meptyldinocap, metyltetraprole, metconazole, metsulfovax, methfuroxam, metominostrobin, metrafenone, mepanipyrim, mefenoxam, mefentrifluconazole, meptyldinocap, mepronil, mebenil, iodomethane, rabenzazole, methyl bromide, benzalkonium chloride, basic copper chloride, basic copper sulfate, inorganic microbicides such as silver, sodium hypochlorite, cupric hydroxide, wettable sulfur, calcium polysulfide, potassium hydrogen carbonate, sodium hydrogen carbonate, sulfur, copper sulfate anhydride, nickel dimethyldithiocarbamate, copper compounds such as copper-8-quinolinolate (oxine copper), zinc sulfate and copper sulfate pentahydrate.

Exemplary herbicides used for the same purposes as above include 1-naphthylacetamide, 2,4-PA, 2,3,6-TBA, 2,4,5-T, 2,4,5-TB, 2,4-D, 2,4-DB, 2,4-DEB, 2,4-DEP, 3,4-DA, 3,4-DB, 3,4-DP, 4-CPA, 4-CPB, 4-CPP, MCP, MCPA, MCPA-thioethyl, MCPB, ioxynil, icafolin, aclonifen, azafenidin, acifluorfen, aziprotryne, azimsulfuron, asulam, acetochlor, atrazine, atraton, anisuron, anilofos, aviglycine, abscisic acid, amicarbazone, amidosulfuron, amitrole, aminocyclopyrachlor, aminopyralid, amibuzin, amiprophos-methyl, ametridione, ametryn, alachlor, allidochlor, alloxydim, alorac, iofensulfuron, isouron, isocarbamid, isoxachlortole, isoxapyrifop, isoxaflutole, isoxaben, isocil, isonoruron, isoproturon, isopropalin, isopolinate, isomethiozin, inabenfide, ipazine, iptriazopyrid, ipfencarbazone, iprymidam, imazaquin, imazapic, imazapyr, imazamethapyr, imazamethabenz, imazamethabenz-methyl, imazamox, imazethapyr, imazosulfuron, indaziflam, indanofan, indolauxipyr, indolauxipyr-cyanomethyl, indolebutyric acid, uniconazole-P, eglinazine, esprocarb, ethametsulfuron, ethametsulfuron-methyl, ethalfluralin, ethiolate, ethychlozate-ethyl, ethidimuron, etinofen, ethephon, ethoxysulfuron, ethoxyfen, etnipromid, ethofumesate, etobenzanid, epyrifenacil, epronaz, erbon, endothal, oxadiazon, oxadiargyl, oxaziclomefone, oxasulfuron, oxapyrazon, oxyfluorfen, oryzalin, orthosulfamuron, orbencarb, cafenstrole, cambendichlor, carbasulam, carfentrazone, carfentrazone-ethyl, karbutilate, carbetamide, carboxazole, quizalofop, quizalofop-P, quizalofop-ethyl, xylachlor, quinoclamine, quinonamid, quinclorac, quinmerac, cumyluron, clacyfos, cliodinate, glyphosate, glufosinate, glufosinate-P, credazine, clethodim, cloxyfonac, clodinafop, clodinafop-propargyl, chlorotoluron, clopyralid, cloproxydim, cloprop, chlorbromuron, clofop, clomazone, chlomethoxynil, chlomethoxyfen, clomeprop, chlorazifop, chlorazine, cloransulam, chloranocryl, chloramben, cloransulam-methyl, chloridazon, chlorimuron, chlorimuron-ethyl, chlorsulfuron, chlorthal, chlorthiamid, chlornitrofen, chlorfenac, chlorfenprop, chlorbufam, chlorflurazole, chlorflurenol, chlorprocarb, chlorpropham, chlormequat, chloreturon, chloroxynil, chloroxuron, chloropon, saflufenacil, cyanazine, cyanatryn, di-allate, diuron, diethamquat, dioxopyritrione, dicamba, cycluron, cycloate, cycloxydim, diclosulam, cyclosulfamuron, cyclopyranil, cyclopyrimorate, dichlorprop, dichlorprop-P, dichlobenil, diclofop, diclofop-methyl, dichlormate, dichloralurea, diquat, cisanilide, disul, siduron, dithiopyr, dinitramine, cinidon-ethyl, dinosam, cinosulfuron, dinoseb, dinoterb, dinofenate, dinoprop, cyhalofop-butyl, cypyrafluone, diphenamid, difenoxuron, difenopenten, difenzoquat, cybutryne, cyprazine, cyprazole, diflufenican, diflufenzopyr, dipropetryn, cypromid, cyperquat, gibberellin, simazine, dimexano, dimesulfazet, dimethachlor, dimidazon, dimethametryn, dimethenamid, simetryn, simeton, dimepiperate, dimefuron, cinflubrolin, cinmethylin, swep, sulglycapin, sulcotrione, sulfallate, sulfentrazone, sulfosulfuron, sulfometuron, sulfometuron-methyl, secbumeton, sethoxydim, sebuthylazine, terbacil, daimuron, dazomet, dalapon, thiazafluron, thiazopyr, tiafenacil, thiencarbazone, thiencarbazone-methyl, tiocarbazil, tioclorim, thiobencarb, thidiazimin, thidiazuron, thifensulfuron, thifensulfuron-methyl, desmedipham, desmetryn, tetflupyrolimet, tetrafluron, thenylchlor, tebutam, tebuthiuron, terbumeton, tepraloxydim, tefuryltrione, tembotrione, delachlor, terbacil, terbucarb, terbuchlor, terbuthylazine, terbutryn, topramezone, tralkoxydim, triaziflam, triasulfuron, triafamone, triallate, trietazine, tricamba, triclopyr, tridiphane, tritac, tritosulfuron, tripyrasulfone, trifludimoxazin, triflusulfuron, triflusulfuron-methyl, trifluralin, trifloxysulfuron, tripropindan, tribenuron-methyl, tribenuron, trifop, trifopsime, trimeturon, tolpyralate, naptalam, naproanilide, napropamide, nicosulfuron, nitralin, nitrofen, nitrofluorfen, nipyraclofen, neburon, norflurazon, noruron, barban, paclobutrazol, paraquat, parafluron, haloxydine, halauxifen, haloxyfop, haloxyfop-P, haloxyfop-methyl, halosafen, halosulfuron, halosulfuron-methyl, bixlozone, picloram, picolinafen, bicyclopyrone, bispyribac, bispyribac-sodium, pydanon, pinoxaden, bipyrazone, bifenox, piperophos, hymexazol, pyraquinate, pyraclonil, pyrasulfotole, pyrazoxyfen, pyrazosulfuron, pyrazosulfuron-ethyl, pyrazolate, bilanafos, pyraflufen-ethyl, pyriclor, pyridafol, pyrithiobac, pyrithiobac-sodium, pyridate, pyriftalid, pyributicarb, pyriflubenzoxim, pyribenzoxim, pyrimisulfan, primisulfuron, pyriminobac-methyl, feproxydim, flusulfinam, broclozone, pyroxasulfone, pyroxsulam, fenasulam, phenisopham, fenuron, fenoxasulfone, fenoxaprop, fenoxaprop-P, fenoxaprop-ethyl, phenothiol, fenoprop, phenobenzuron, fenquinotrione, fenthiaprop, fenteracol, fentrazamide, fenpyrazone, phenmedipham, phenmedipham-ethyl, butachlor, butafenacil, butamifos, buthiuron, buthidazole, butylate, buturon, butenachlor, butroxydim, butralin, flazasulfuron, flamprop, furyloxyfen, prynachlor, primisulfuron-methyl, fluazifop, fluazifop-P, fluazifop-butyl, fluazolate, fluchloraminopyr, fluchloraminopyr-tefuryl, fluroxypyr, fluothiuron, fluometuron, fluoroglycofen, flurochloridone, fluorodifen, fluoronitrofen, fluoromidine, flucarbazone, flucarbazone-sodium, fluchloralin, flucetosulfuron, fluthiacet, fluthiacet-methyl, flupyrsulfuron, flufenacet, flufenoximacil, flufenican, flufenpyr, flupropacil, flupropanate, flupoxam, flumioxazin, flumiclorac, flumiclorac-pentyl, flumipropyn, flumezin, fluometuron, flumetsulam, fluridone, flurtamone, fluroxypyr, pretilachlor, proxan, proglinazine, procyazine, prodiamine, prosulfalin, prosulfuron, prosulfocarb, propaquizafop, propachlor, propazine, propanil, propyzamide, propisochlor, prohydrojasmon, propyrisulfuron, propham, profluazol, profluralin, prohexadione-calcium, propoxycarbazone, propoxycarbazone-sodium, profoxydim, bromacil, brompyrazon, prometryn, prometon, bromoxynil, bromofenoxim, bromobutide, bromobonil, florasulam, florpyrauxifen, hexachloroacetone, hexazinone, pethoxamid, benazolin, penoxsulam, pebulate, beflubutamid, beflubutamid-M, vernolate, perfluidone, bencarbazone, benquitrione, benzadox, benzipram, benzylaminopurine, benzthiazuron, benzfendizone, bensulide, bensulfuron-methyl, benzoylprop, benzobicyclon, benzofenap, benzofluor, bentazone, pentanochlor, benthiocarb, pendimethalin, pentoxazone, benfluralin, benfuresate, fosamine, fomesafen, foramsulfuron, forchlorfenuron, maleic hydrazide, mecoprop, mecoprop-P, medinoterb, mesosulfuron, mesosulfuron-methyl, mesotrione, mesoprazine, methoprotryne, metazachlor, methazole, metazosulfuron, methabenzthiazuron, metamitron, metamifop, metam, methalpropalin, methiuron, methiozolin, methiobencarb, methyldymron, metoxuron, metosulam, metsulfuron, metsulfuron-methyl, metflurazon, metobromuron, metobenzuron, methometon, metolachlor, metribuzin, mepiquat-chloride, mefenacet, mefluidide, metproxybicyclone, monalide, monisouron, monuron, monochloroacetic acid, monolinuron, molinate, morfamquat, iodosulfuron, iodosulfuron-methyl-sodium, iodobonil, iodomethane, lactofen, lancotrione, linuron, rimisoxafen, rimsulfuron, lenacil, rhodethanil, calcium peroxide and methyl bromide. Biopesticides available as herbicides, such as *Xanthomonas campestris*, can also be used after mixed with the compound of the present invention or a salt thereof.

Examples of the phytotoxicity reducer (safener) include 1,8-naphthalic anhydride, isoxadifen-ethyl, furilazole, cyprosulfamide, cyometrinil, dichlormid, dimepiperate, thiencarbazone-methyl, fenchlorazole-ethyl, fenclorim, fluxofenim, flurazole, benoxacor, metcamifen, and mefenpyr-diethyl.

Examples of the biopesticide include *Agrobacterium radiobacter* (e.g., Galltrol-A (registered trademark) manufactured by AgBioChem, CA using strain K84 and Nogall (registered trademark) manufactured by Becker Underwood, US using strain K1026), *Agrobacterium radiobacter* (e.g., Bacterose (registered trademark) manufactured by NIHON NOHYAKU Co., Ltd. using strain 84), *Ampelomyces quisqualis* (e.g., AQ 10 (registered trademark) manufactured by IntrachemBio Italia & Co. KG using strain AQ10), *Aspergillus flavus* (e.g., Afla-Guard (registered trademark) manufactured by Syngenta, AF36 (registered trademark) manufactured by Arizona Cotton Research and Protection Council, US using strain AF36, and Afla-Guard manufactured by Syngenta using NRRL 21882), *Aureobasidium pullulans* (e.g., Botector (registered trademark), a mixture of blastospores of strain DSM14940 and blastospores of strain DSM14941, manufactured by bio-ferm, GmbH),
*Bacillus amyloliquefaciens* (e.g., Impression Clear (registered trademark) manufactured by Idemitsu Agri using strain AT-332, Avogreen (registered trademark) manufactured by University of Pretoria using strain B246, Bacstar (registered trademark) manufactured by Etec Crop Solutions, NZ using strain D747, Shelter (registered trademark) manufactured by Dagutat Biolab, ZA using strain DB101, Artemis (registered trademark) manufactured by Dagutat Biolab, ZA using strain DB102, RhizoVital (registered trademark) manufactured by ABiTEP, DE using strain FZB42, Kodiak (registered trademark) manufactured by Bayer CropScience AG, DE using strain GB03, Subtilex (registered trademark) manufactured by Becker Underwood, US using strain MBI600, and Amplitude manufactured by Marrone Bio Innovations, Inc. using strain F727), *Bacillus cepacia* (e.g., Deny Stine (registered trademark) manufactured by Microbial Products), *Bacillus cereus* (e.g., Mepichlor (registered trademark) manufactured by Arysta, US using strain BP01), *Bacillus firmus* (e.g., BioNeem (registered trademark) manufactured by AgoGreen using strain I-1582),
*Bacillus lacticola* (e.g., a product manufactured by Micro Flo Company), *Bacillus lactimorbus* (e.g., a product manufactured by Micro Flo Company), *Bacillus lactis* (e.g., a product manufactured by Micro Flo Company), *Bacillus laterosporus* (e.g., Bio-Tode (registered trademark) manufactured by Agro-Organics, SA), *Bacillus licheniformis* (e.g., EcoGuard Biofungicide (registered trademark) manufactured by Novozymes using strain SB3086), *Bacillus maroccanus* (e.g., a product manufactured by Micro Flo Company), *Bacillus megaterium* (e.g., Bioarc (registered trademark) manufactured by Bio Arc using strain YFM3.25), *Bacillus metiens* (e.g., a product manufactured by Micro Flo Company), *Bacillus mojavensis* (e.g., a product manufactured by Probelte, Sa using strain SR11), *Bacillus mycoides* (e.g., BmJ (registered trademark) manufactured by Certis USA using isolate J.),
*Bacillus nigrificans* (e.g., a product manufactured by Micro Flo Company), *Bacillus popilliae* (e.g., Cronox (registered trademark) manufactured by Bio Crop, CO), *Bacillus pumilus* (e.g., Integral F-33 (registered trademark) manufactured by Becker Underwood, US using strain BUF-33, Yield Shield (registered trademark) manufactured by Bayer CropScience AG, DE using strain GB34, and Sonata (registered trademark) manufactured by Bayer CropScience LP, US using strain QST2808), *Bacillus simplex* (e.g., Momi-Hope WP (registered trademark) manufactured by Arysta LifeScience Corporation using strain CGF2856), *Bacillus sphaericus* (e.g., VectoLex (registered trademark) manufactured by Valent BioSciences, US using serotype H5a5b strain 2362),
*Bacillus subtilis* (e.g., Botokiller WP (registered trademark) manufactured by Idemitsu Agri Co., Taegro (registered trademark) manufactured by Novozyme Biologicals, Inc. US using strain FZB24, SERENADE MAX (registered trademark) manufactured by Bayer CropScience LP, US using strain QST713/AQ713, Serenade-DPZ (registered trademark) using strain AQ30002, EcoShot (registered trademark) manufactured by KUMIAI CHEMICAL INDUSTRY Co., Ltd. using strain D747, Agrocare WP (registered trademark) manufactured by Nisso Green Co., Ltd. using strain HAI-0404, Botopika WP (registered trademark) manufactured by Idemitsu Kosan Co., Ltd. using strain MBI600, BaciStar WP (registered trademark) manufactured by Arysta LifeScience Corporation using strain Y1336, and Companion Biological Fungicide Wettable Powder manufactured by Growth Products Ltd. using strain GB03), *Bacillus thuringiensis* (e.g., VectoBac (registered trademark) manufactured by Valent BioSciences, US using strain AM65-52), *Bacillus thuringienses aizawai* (e.g., XenTari (registered trademark) manufactured by Bayer CropScience AG, DE using strain ABTS-1857, Florbac WG (registered trademark) manufactured by Valent BioSciences, US using serotype H-7, and Agree WG Biological Insecticide manufactured by Certis USA using strain GC-91), *Bacillus thuringiensis* subspecies. *Aegypti* (e.g., Agerin (registered trademark)), *Bacillus thuringienses israelensis* (e.g., Aquabac (registered trademark) manufactured by Becker Microbial Products IL using strain BMP144),
*Bacillus thuringienses kurstaki* (e.g., a product manufactured by Becker Microbial Products, IL using strain BMP 123, Dipel ES (registered trademark) manufactured by Valent BioSciences, US using strain HD-1, BMP 123 manufactured by Becker Microbial Products using strain BMP123, BMP144/Aquabac manufactured by Becker Microbial Products using strain BMP144, Dipel 10G manufactured by Valent U.S.A. LLC using strain ABTS-351, Condor Wettable Powder manufactured by Certis USA using strain EG2348, Crymax Bioinsecticide manufactured by Certis USA using strain EG7841, Deliver Biological Insecticide manufactured by Certis USA using strain SA-12, and Bioprotec PLUS manufactured by AEF Global using strain EVB-113-19), *Bacillus thuringiensis galleriae* (e.g., beetleGONE! manufactured by Phyllom BioProducts and boreGONE! manufactured by Phyllom BioProducts using strain SDS-502),
*Bacillus thuringiensis* var. *colmeri* (e.g., TianBaoBTc (registered trademark) manufactured by Changzhou Jianghai Chemical Factory), *Bacillus thuringienses tenebrionis* (e.g., Novodor FC (registered trademark) manufactured by BioFa DE using strain NB 176), *Bacillus thuringiensis* var. *san diego* (e.g., M-One (registered trademark) from Bacillus thuringiensis var. san diego), *Beauveria bassiana* (e.g., Naturalis (registered trademark) manufactured by Intrachem Bio Italia, Bove Max (registered trademark) manufactured by Novozymes using strain CG716, BioLisa Madara (registered trademark) manufactured by Idemitsu Kosan Co., Ltd. using strain F-263, BotaniGard WP (registered trademark) manufactured by ARYSTA using strain GHA, balEnce manufactured by Terragena, Inc. using strain HF23, BotaniGard ES manufactured by BioWorks Inc. using strain GHA, and BioCeres WP manufactured by BioSafe Systems using strain ANT-03),
*Beauveria brongniartii* (e.g., Beaupro (registered trademark) manufactured by Andermatt Biocontrol AG), *Bradyrhizobium japonicum* (e.g., Optimize (registered trademark) manufactured by Novozymes), *Burkholderia* spp. (e.g., MBI-206 TGAI (registered trademark) manufactured by Marrone Bio Innovations using strain A396), *Candida oleophila* (e.g., Aspire manufactured by Ecogen Inc., US using strain I-82 and Nexy manufactured by BioNext using strain O), *Candida saitoana* (e.g., BIOCURE (registered trademark) manufactured by Micro Flo Company, US (BASF SE)), *Chaetomium cupreum* (e.g., BIOKUPRUM (registered trademark) manufactured by AgriLife), *Chaetomium globosum* (e.g., Rivadiom (registered trademark) manufactured by Rivale), *Chromobacterium subtsugae* (e.g., Grandevo (registered trademark) manufactured by Marrone Bio Innovations using strain PRAA4-1T),
*Cladosporium cladosporioides* (e.g., from Cladosporium cladosporioides), *Clonostachys rosea* f. *catenulate* (e.g., PRESTOP (registered trademark) manufactured by Verdera, Finland using strain J1446), *Colletotrichum gloeosporioides* (e.g., Collego (registered trademark) manufactured by Agriultural Research Initiatives), *Coniothyrium minitans* (e.g., Contans (registered trademark) manufactured by Encore Technologies, LLC using strain CON/M/91-08), *Cryptococcus albidus* (e.g., YieldPlus (registered trademark) manufactured by Anchor Bio Technologies, ZA), *Delftia acidovorans* (e.g., BioBoost (registered trademark) manufactured by Brett Young Seeds using strain RAY209), *Dilophosphora alopecuri* (e.g., Twist Fungus (registered trademark)), *Drechsrela monoceras* (e.g., Tasumato herbicide (registered trademark) manufactured by Mitsui Chemicals Agro, Inc. using strain MTB-951), *Entomophthora virulenta* (e.g., Vektor (registered trademark) manufactured by Ecomic), *Fusarium oxysporum* (e.g., Maruka light manufactured by Eisai Seikaken, Inc. using strain 101-2), *Fusarium oxysporum* (e.g., Fusaclean (registered trademark) manufactured by Natural Plant Protection using strain Fo47),
*Gliocladium* spp. (e.g., Prestop (registered trademark) manufactured by AgBio Inc. using strain J1446 and a product manufactured by W.F. Stoneman Company LLC using strain 321U), *Hirsutella thompsonii* (e.g., Mycohit (registered trademark) manufactured by Agro Bio tech Research Centre, IN), *Lactobacillus acidophilus* (e.g., Fruitsan (registered trademark) manufactured by Inagrosa Industrias Agrobiologicas, S.A.), *Lactobacillus plantarum* (e.g., Lactoguard WP manufactured by Meiji Seika Pharma Co., Ltd. using strain BY), *Lecanicillium lecanii* (e.g., Mycotal (registered trademark) manufactured by Koppert/Arysta using conidiospores of strain KV01), *Metarhizium anisopliae* (e.g., BIO 1020 (registered trademark) manufactured by Bayer CropScience using strain F52, Pirates G (registered trademark) manufactured by Arysta LifeScience Corporation using strain SMZ-2000, and Bio-Blast manufactured by LidoChem Inc using strain ESC1),
*Metarhizium anisopliae* var. *acridum* (e.g., Green Muscle (registered trademark) manufactured by Biological Control Products and GreenGuard (registered trademark) manufactured by Becker Underwood, US), *Metschnikowia fructicola* (e.g., Shemer (registered trademark) manufactured by Bayer CropScience), *Microdochium dimerum* (e.g., ANTIBOT (registered trademark) manufactured by Agrauxine, France), *Microsphaeropsis ochracea* (e.g., Microx (registered trademark) manufactured by Prophyta), *Monacrosporium phymatopagum* (e.g., Nemahiton (registered trademark) manufactured by Tomoe Kagaku Kogyo K.K.), *Mucor haemelis* (e.g., BioAvard (registered trademark) manufactured by Indore Biotech Inputs & Research), *Muscodor albus* (e.g., Arabesque manufactured by Bayer Crop Science using strain QST 20799), *Myrothecium verrucaria* (e.g., DiTera (registered trademark) manufactured by Valent Biosciences using strain AARC-0255), *Paecilomyces fumosoroseus* (e.g., PreFeRal (registered trademark) WG manufactured by Biobest using strain apopka 97, Preferred WP manufactured by Tokai Bussan Co. Ltd., and No Fly (registered trademark) manufactured by Natural Industries Inc. (Novozymes company) using strain FE9901), *Paecilomyces lilacinus* (e.g., BioAct WG (registered trademark) manufactured by Prophyta using strain 251),
*Pacilimyces tenuipes* (e.g., Gottu A manufactured by Idemitsu Kosan Co., Ltd. using strain T1), *Paecilomyces variotii* (e.g., Nemaquim (registered trademark) manufactured by Quimia, MX using strain Q-09), *Paenibacillus polymyxa* (e.g., Topseed (registered trademark) manufactured by Green Biotech Company Ltd. using strain AC-1), *Paenibacillus poppiliae* (e.g., Milky spore disease (registered trademark) manufactured by St. Gabriel Laboratories), *Pasteuria nishizawae* (e.g., oyacyst LF/ST (registered trademark) manufactured by Pasteuria Bioscience and Clariva pn manufactured by Syngenta using strain Pn1), *Pasteuria penetrans* (e.g., Pasteuria (registered trademark) manufactured by Pasteuria Bioscience), *Pasteuria usagae* (e.g., Econem (registered trademark) manufactured by Pasteuria Bioscience),
*Pantoea agglomerans* (e.g., Bloomtime Biological FD Biopesticide manufactured by Nufarm US using strain E325), *Pectobacterium carotovorum* (e.g., Biokeeper (registered trademark) manufactured by Nissan Chemical Corporation and EcoMate (registered trademark) manufactured by KUMIAI CHEMICAL INDUSTRY Co., Ltd. using CGE234M403), *Phoma macrostroma* (e.g., Phoma H (registered trademark) manufactured by Scotts, US using strain 94-44B), *Penicillium bilaii* (e.g., Jump Start (registered trademark) manufactured by Novozymes), *Phlebiopsis gigantea* (e.g., ROTSOP (registered trademark) manufactured by Verdera, Finland using strain FOC PG B22/SP1190/3.2), *Pochonia chlamydosporia* var. *catenulata* (e.g., KlamiC (registered trademark) manufactured by The National Center of Animal and Plant Health (CENSA); CU), *Pseudomonas aureofaciens* (e.g., Spot-Less Biofungicide (registered trademark) manufactured by Eco Soils Systems, CA using strain TX-1), *Pseudomonas chlororaphis* (e.g., ATEze (registered trademark) manufactured by EcoSoil Systems using strain 63-28 and Cedomon (registered trademark) manufactured by Bioagri, S. using strain MA342), *Pseudomonas fluorescens* (e.g., Frostban D (registered trademark) manufactured by Frost Technology Corp. using strain 1629RS, Blightban (registered trademark) manufactured by Blightban using strain A506, Konae Fukudo (registered trademark) manufactured by Taki Chemical Co., Ltd. using strain FPT-9601, Cell Nae Genki (registered trademark), a mixture of strains FPT-9601 and FPH-9601, manufactured by Taki Chemical Co., Ltd., and Vegikeeper (registered trademark) manufactured by Arysta LifeScience Corporation using strain G7090), *Pseudomonas proradix* (e.g., Proradix (registered trademark) manufactured by Sourcon Padena),
*Pseudomonas resinovorans* (e.g., Solanacure (registered trademark) manufactured by Agricultural Research Council, SA), *Pseudomonas syringae* (e.g., Biosave (registered trademark) manufactured by EcoScience, US using strain MA-4, Frostban C (registered trademark) manufactured by Frost Technology Corp using strain 742RS, Bio-save 10LP Biological Fungicide manufactured by Jet Harvest Systems using strain ESC10, and Bio-Save 11LP Biological Fungicide manufactured by Jet Harvest Systems using strain ESC11), *Pseudomonas* spp. (e.g., Masterpiece WP (registered trademark) manufactured by Nippon Soda Co., Ltd. using strain HAI-0804 and Momi-Genki WP manufactured by Nissan Chemical Corporation using strain CAB-02), *Pseudozyma aphidis* (e.g., a product manufactured by Yissum Research Development Company of the Hebrew University of Jerusalem), *Pseudozyma flocculosa* (e.g., Sporodex L manufactured by Plant Products Co. Ltd., CA using strain PF-A22 UL), *Pythium oligandrum* (e.g., Polyversum (registered trademark) manufactured by Bioprepraty, CZ using strain DV74 or strain M1), *Reynoutria sachlinensis* (e.g., REGALIA (registered trademark) manufactured by Marrone BioInnovations, US),
*Rhizopogon amylopogon* (e.g., Myco-Sol (registered trademark) manufactured by Helena Chemical Company), *Rhizopogon fulvigleba* (e.g., Myco-Sol (registered trademark) manufactured by Helena Chemical Company), *Saccharomyces cerevisiae* (e.g., a product manufactured by Lesaffre et Compagnie, FR), *Sclerotinia minor* (e.g., Sarritor (registered trademark) manufactured by Agrium Advanced Technologies), *Serratia entomophila* (e.g., Invade (registered trademark) manufactured by Wrightson Seeds), *Sporothrix insectorum* (e.g., Sporothrix Es (registered trademark) manufactured by Biocerto, BR), *Steinernema carpocapsae* (e.g., Biosafe (registered trademark) manufactured by SDS Biotech K.K.), *Steinernema kushidai* (e.g., Shibaichi-Nema manufactured by Kubota Corporation), *Steinernema glaseri* (e.g., Biotopia (registered trademark) manufactured by Arysta LifeScience Corporation), *Streptomyces acidiscabies* (e.g., MBI-005EP (registered trademark) manufactured by Marrone Bioinnovations, CA using strain RL-110T), *Streptomyces candidus* (e.g., BioBac (registered trademark) manufactured by Biontech, TW using strain Y21007-2),
*Streptomyces galbus* (e.g., Mycostop (registered trademark) manufactured by Verdera using strain K61), *Streptomyces lydicus* (e.g., ACTINOVATE (registered trademark) manufactured by Natural Industries, US using strain WYEC108), *Streptomyces saraceticus* (e.g., Clanda (registered trademark) manufactured by A & A Group (Agro Chemical Corp.)), *Talaromyces flavus* (e.g., Momi-keeper (registered trademark) manufactured by Central Glass Co., Ltd. using strain B-422, Tough Block (registered trademark) manufactured by Idemitsu Kosan Co., Ltd. using strain SAY-Y-94-01, and PROTUS (registered trademark) WG manufactured by Prophyta, DE using strain V117b), *Trichoderma asperellum* (e.g., a product manufactured by Isagro using strain ICC 012, T34 Biocontrol (registered trademark) manufactured by Bioncontrol Technologies, ES using strain T34, and EcoHope (registered trademark) manufactured by KUMIAI CHEMICAL INDUSTRY Co., Ltd. using strain SKT-1), *Trichoderma atroviride* (e.g., Esquive (registered trademark) WP manufactured by Agrauxine, FR, Tenet (registered trademark) manufactured by Agrimm Technologies Ltd, NZ or SENTINEL (registered trademark) each using strain LC52, and EcoHope DJ (registered trademark) manufactured by KUMIAI CHEMICAL INDUSTRY Co., Ltd. using strain SKT-1), *Trichoderma gamsii* (e.g., BIO-TAM (registered trademark) manufactured by Bayer CropScience LP, US),
*Trichoderma harzianum* (e.g., T-Gro 7456 (registered trademark) manufactured by Dagutat Biolab using strain DB103, Trianum-P (registered trademark) manufactured by Koppert using strain ITEM908, Trichoplus (registered trademark) manufactured by Biological Control Products, SA using strain KD, and ROOT PRO (registered trademark) manufactured by Mycontrol Ltd. using strain TH-35), *Trichoderma harzianum* Rifai (e.g., PLANTSHIELD T-22G (registered trademark) manufactured by Firma BioWorks Inc, US using strain T-22 and TRICHODEX (registered trademark) manufactured by Makhteshim Ltd, US using strain T-39), *Trichoderma lignorum* (e.g., Mycotric (registered trademark) manufactured by Futureco Bioscience, ES using strain TL-0601), *Trichoderma polysporum* (e.g., Binab TF WP (registered trademark) manufactured by BINAB Bio-Innovation AB, Sweden), *Trichoderma stromaticum* (e.g., TRICOVAB (registered trademark) manufactured by Ceplac, Brazil), *Trichoderma virens* (e.g., SOILGARD (registered trademark) manufactured by Certis LLC, US using strain GL-21),
*Trichoderma viride* (e.g., REMEDIER (registered trademark) WP manufactured by Isagro Ricerca, ITALIA using strain ICC080 and Trianum-P (registered trademark) manufactured by Koppert using strain TV1), *Tsukamurella paurometabola* (e.g., HeberNem (registered trademark) using strain C-924), *Ulocladium oudemansii* (e.g., Botry-Zen (registered trademark) manufactured by Botry-Zen Ltd, NZ using strain HRU3 and BotryStop manufactured by BioWorks Inc. using strain U3), *Variovorax paradoxus* (e.g., Fieldkeeper WP (registered trademark) manufactured by Central Glass using strain CGF4526), Vesicular-Arbuscular (VA) mycorrhizal fungi (e.g., Dr. Kinkon (registered trademark) manufactured by Idemitsu Agri), *Verticillium alboatrum* (e.g., Dutch Trig (registered trademark) manufactured by Tree Care Innovations using strain WCS850), *Verticillium lecanii* (e.g., Vertalec (registered trademark) manufactured by Arysta LifeScience Corporation using strain IMI 179172 and Mycotal (registered trademark) manufactured by Arysta LifeScience Corporation using strain IMI 263817), *Xanthomonas campestris* (e.g., Camperico L (registered trademark) manufactured by Taki Chemical), *Xanthomonas campestris* pv. *poae*, *Heterorhabditis bacteriophora*, *Steinernema feltiae*, *Steinernema kraussei*, *Steinernema riobrave*, and *Steinernema scapterisci*; and insecticidal and microbicidal strains selected from mutants of the above-listed strains that each have its original distinguishing characteristics, or metabolites that are produced by the above-listed strains and active against plant pathogenic microbes.

### EXAMPLES

Hereinafter, representative Examples in relation to the present invention are shown, but the present invention is not limited thereto.

### Production Example 1

### Production of 1-tert-butyl-3-{6-[3-(2-chlorophenyl)-5-methyl-1H-1,2,4-triazol-1-yl]-5-(methanesulfonyl)pyridin-2-yl}imidazolidin-2-one (Compound No. 1-1)

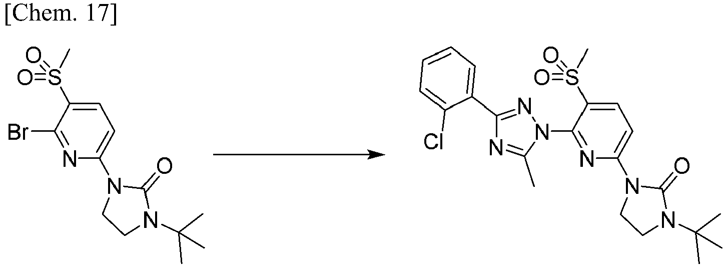

To a solution of 1-[6-bromo-5-(methanesulfonyl)pyridin-2-yl]-3-tert-butylimidazolidin-2-one (60 mg, 0.16 mmol), produced by the method described in Reference Production Example 1 described later, in N,N-dimethylacetamide (1.5 mL), potassium carbonate (44 mg, 0.32 mmol) and 3-(2-chlorophenyl)-5-methyl-1H-1,2,4-triazole (31 mg, 0.16 mmol) were added, and the mixture was stirred at 130°C for 5 hours. Water was added to the reaction mixture, and ethyl acetate extraction was performed. The organic layer was concentrated in vacuo. The residue was purified by silica gel column chromatography to give 1-tert-butyl-3-{6-[3-(2-chlorophenyl)-5-methyl-1H-1,2,4-triazol-1-yl]-5-(methanesulfonyl)pyridin-2-yl}imidazolidin-2-one (63 mg, 0.13 mmol).
Yield: 81%
Physical property: ¹H-NMR (CDCl₃): δ 8.63 (d, 1H), 8.37 (d, 1H), 8.05-8.03 (m, 1H), 7.49-7.47 (m, 1H), 7.38-7.34 (m, 2H), 3.95 (t, 2H), 3.57 (t, 2H), 3.43 (s, 3H), 2.55 (s, 3H), 1.46 (s, 9H)

### Production Example 2

### Production of 6-(3,5-dimethyl-1H-1,2,4-triazol-1-yl)-N-methyl-2-(1-methyl-3-phenyl-1H-1,2,4-triazol-5-yl)pyridine-3-sulfonamide (Compound No. 2-9)

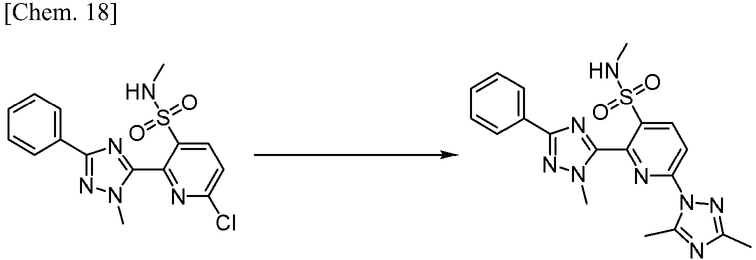

To a solution of 6-chloro-N-methyl-2-(1-methyl-3-phenyl-1H-1,2,4-triazol-5-yl)pyridine-3-sulfonamide (0.20 g, 0.55 mmol), produced by the method described in Reference Production Example 2 described later, in N,N-dimethylacetamide (3.0 mL), 3,5-dimethyl-1H-1,2,4-triazole (81 mg, 0.83 mmol) and cesium carbonate (0.55 g, 1.7 mmol) were added, and the mixture was stirred at 100°C for 2 hours. Water was added to the reaction mixture, and ethyl acetate extraction was performed. The organic layer was concentrated in vacuo. The residue was purified by silica gel column chromatography to give 6-(3,5-dimethyl-1H-1,2,4-triazol-1-yl)-N-methyl-2-(1-methyl-3-phenyl-1H-1,2,4-triazol-5-yl)pyridine-3-sulfonamide (65 mg, 0.15 mmol).
Yield: 28%
Physical property: ¹H-NMR (CDCl₃): δ 8.61 (d, 1H), 8.21 (d, 1H), 8.02-7.99 (m, 1H), 7.92-7.87 (m, 1H), 7.43-7.40 (m, 2H), 7.34-7.29 (m, 1H), 4.08 (s, 3H), 2.86-2.82 (m, 6H), 2.45 (s, 3H)

### Production Example 3

### Production of N-methyl-6-(1-methyl-1H-pyrazol-5-yl)-2-[1-methyl-3-(thiophen-2-yl)-1H-1,2,4-triazol-5-yl]pyridine-3-sulfonamide (Compound No. 2-160)

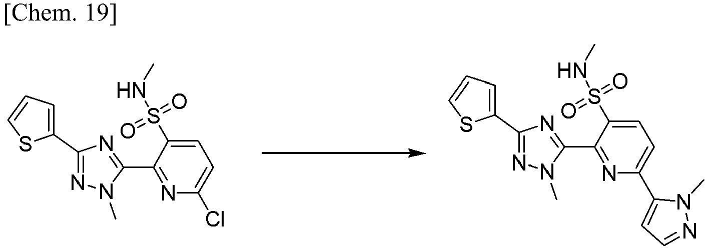

In a mixed solution of 1,4-dioxane (1.0 mL) and water (0.20 mL) was dissolved 6-chloro-N-methyl-2-[1-methyl-3-(thiophen-2-yl)-1H-1,2,4-triazol-5-yl]pyridine-3-sulfonamide (74 mg, 0.20 mmol) produced by the method described in Reference Production Example 6 described later. To this, 1-methyl-1H-pyrazole-5-boronic acid pinacol ester (62 mg, 0.30 mmol), tetrakis(triphenylphosphine)palladium (23 mg, 20 µmol) and potassium carbonate (98 mg, 0.46 mmol) were added, and the mixture was stirred at 110°C for 3 hours. Water was added to the reaction mixture, and ethyl acetate extraction was performed. The organic layer was washed with saturated brine, dried over anhydrous sodium sulfate, and concentrated in vacuo. The residue was purified by silica gel column chromatography to give N-methyl-6-(1-methyl-1H-pyrazol-5-yl)-2-[1-methyl-3-(thiophen-2-yl)-1H-1,2,4-triazol-5-yl]pyridine-3-sulfonamide (76 mg, 0.18 mmol).
Yield: 91%
Physical property: melting point: 205-207°C

### Production Example 4

### Production of 6-[(3,5-dimethyl-1H-1,2,4-triazol-1-yl)methoxy]-2-(1-ethyl-3-phenyl-1H-1,2,4-triazol-5-yl)-N-methylpyridine-3-sulfonamide (Compound No. 2-174)

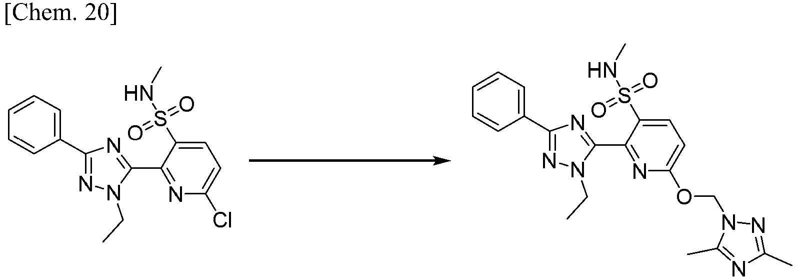

To a solution of 6-chloro-2-(1-ethyl-3-phenyl-1H-1,2,4-triazol-5-yl)-N-methylpyridine-3-sulfonamide (0.38 g, 1.0 mmol), produced by the method described in Reference Production Example 7 described later, in acetonitrile (10 mL), (3,5-dimethyl-1H-1,2,4-triazol-1-yl)methanol (0.19 g, 1.5 mmol) and cesium carbonate (0.98 g, 3.0 mmol) were added, and the mixture was stirred at 70°C for 3 hours. Water was added to the reaction mixture, and ethyl acetate extraction was performed. The organic layer was concentrated in vacuo. The residue was purified by silica gel column chromatography to give 6-[(3,5-dimethyl-1H-1,2,4-triazol-1-yl)methoxy]-2-(1-ethyl-3-phenyl-1H-1,2,4-triazol-5-yl)-N-methylpyridine-3-sulfonamide (0.12 g, 0.26 mmol).
Yield: 26%
Physical property: ¹H-NMR (CDCl₃): δ 8.60 (d, 1H), 8.19 (d, 1H), 8.06-7.97 (m, 2H), 7.50-7.40 (m, 3H), 7.35-7.28 (m, 1H), 4.40 (q, 2H), 2.84-2.81 (m, 6H), 2.44 (d, 3H), 1.49 (t, 3H)

### Production Example 5

### Production of 2-[3-(2-chlorophenyl)-5-methyl-1H-1,2,4-triazol-1-yl]-6-(3,5-dimethyl-1H-1,2,4-triazol-1-yl)-N-methylpyridine-3-sulfonamide (Compound No. 3-2)

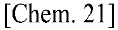

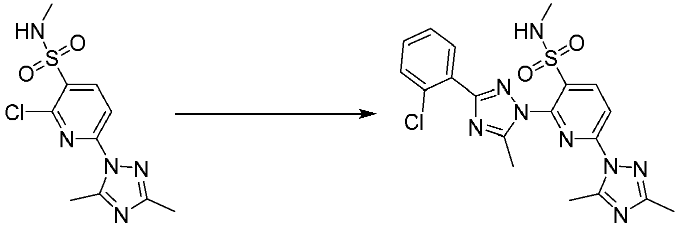

To a solution of 2-chloro-6-(3,5-dimethyl-1H-1,2,4-triazol-1-yl)-N-methylpyridine-3-sulfonamide (1.0 g, 3.3 mmol), produced by the method described in Reference Production Example 3 described later, in N,N-dimethylacetamide (10 mL), 3-(2-chlorophenyl)-5-methyl-1H-1,2,4-triazole (0.96 g, 5.0 mmol) and potassium carbonate (2.0 g, 15 mmol) were added, and the mixture was stirred at 100°C for 2 hours. Water was added to the reaction mixture, and ethyl acetate extraction was performed. The organic layer was concentrated in vacuo. The residue was purified by silica gel column chromatography to give 2-[3-(2-chlorophenyl)-5-methyl-1H-1,2,4-triazol-1-yl]-6-(3,5-dimethyl-1H-1,2,4-triazol-1-yl)-N-methylpyridine-3-sulfonamide (0.23 g, 0.50 mmol).
Yield: 15%
Physical property: ¹H-NMR (CDCl₃): δ 8.63 (d, 1H), 8.26-8.20 (m, 2H), 7.52-7.48 (m, 1H), 7.45-7.36 (m, 2H), 6.66 (q, 1H), 2.83 (s, 3H), 2.77 (d, 3H), 2.66 (s, 3H), 2.45 (s, 3H)

### Production Example 6

### Production of 6-[(3,5-dimethyl-1H-pyrazol-1-yl)methoxy]-N-methyl-2-(5-methyl-3-phenyl-1H-pyrazol-1-yl)pyridine-3-sulfonamide (Compound No. 3-36)

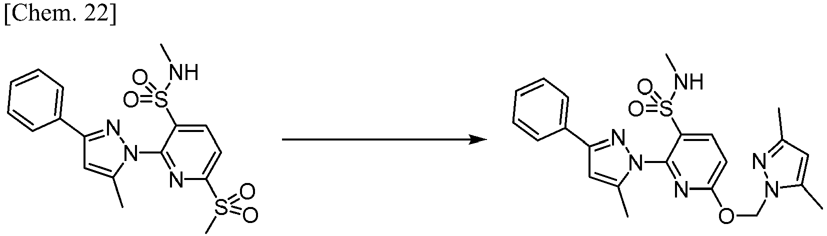

To a solution of 6-(methanesulfonyl)-N-methyl-2-(5-methyl-3-phenyl-1H-pyrazol-1-yl)pyridine-3-sulfonamide (35 mg, 0.086 mmol), produced by the method described in Reference Production Example 4 described later, in acetonitrile (2.0 mL), 1-(hydroxymethyl)-3,5-dimethylpyrazole (11 mg, 0.087 mmol) and potassium carbonate (24 mg, 0.17 mmol) were added, and the mixture was stirred at 60°C for 6 hours. Water was added to the reaction mixture, and ethyl acetate extraction was performed. The organic layer was concentrated in vacuo. The residue was purified by silica gel column chromatography to give 6-[(3,5-dimethyl-1H-pyrazol-1-yl)methoxy]-N-methyl-2-(5-methyl-3-phenyl-1H-pyrazol-1-yl)pyridine-3-sulfonamide (32 mg, 0.071 mmol).
Yield: 80%
Physical property: ¹H-NMR (CDCl₃): δ 8.38 (d, 1H), 7.73-7.70 (m, 2H), 7.45-7.35 (m, 3H), 6.96 (d, 1H), 6.62 (q, 1H), 6.60 (s, 1H), 6.19 (s, 2H), 5.91 (s, 1H), 2.76 (d, 3H), 2.48 (s, 3H), 2.30 (s, 3H), 2.24 (s, 3H)

### Production Example 7

### Production of 6-(3,5-dimethyl-1H-1,2,4-triazol-1-yl)-N-methyl-2-(1-methyl-4-phenylimidazol-2-yl)pyridine-3-sulfonamide (Compound No. 2-16)

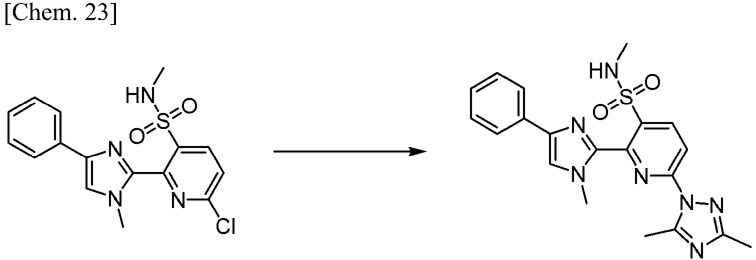

To a solution of 6-chloro-N-methyl-2-(1-methyl-4-phenyl-1H-imidazol-2-yl)pyridine-3-sulfonamide (82 mg, 0.22 mmol), produced by the method described in Reference Production Example 5 described later, in N,N-dimethylacetamide (2.0 mL), 3,5-dimethyl-1H-1,2,4-triazole (32 mg, 0.33 mmol) and cesium carbonate (0.22 g, 0.66 mmol) were added, and the mixture was stirred at 100°C for 2 hours. Water was added to the reaction mixture, and the resulting solid was collected by filtration to give 6-(3,5-dimethyl-1H-1,2,4-triazol-1-yl)-N-methyl-2-(1-methyl-4-phenylimidazol-2-yl)pyridine-3-sulfonamide (32 mg, 0.076 mmol).
Yield: 34%
Physical property: melting point: 134-135°C

### Reference Production Example 1

### Production of 1-[6-bromo-5-(methanesulfonyl)pyridin-2-yl]-3-tert-butylimidazolidin-2-one

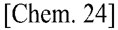

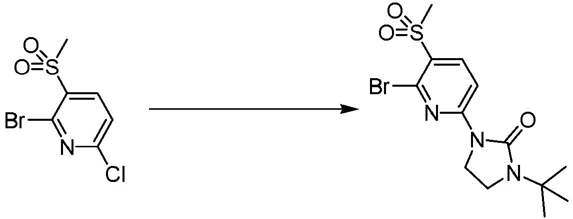

To a solution (11 mL) of 1-(1,1-dimethylethyl)-2-imidazolidinone (0.32 g, 2.3 mmol) in toluene and dimethyl sulfoxide, sodium hydride (60% wt, 0.11 g, 2.8 mmol) was added with cooling in an ice bath, and the mixture was stirred for 30 minutes. To this, 2-bromo-6-chloro-3-(methanesulfonyl)pyridine (0.60 g, 2.2 mmol) was added, and the mixture was stirred at 100°C for 7 hours. Water was added to the reaction mixture, and ethyl acetate extraction was performed. The organic layer was concentrated in vacuo. The residue was purified by silica gel column chromatography to give 1-[6-bromo-5-(methanesulfonyl)pyridin-2-yl]-3-tert-butylimidazolidin-2-one (0.15 g, 0.40 mmol).
Yield: 18%

### Reference Production Example 2

### Production of 6-chloro-N-methyl-2-(1-methyl-3-phenyl-1H-1,2,4-triazol-5-yl)pyridine-3-sulfonamide

### Reference Production Example 2-1

### Production of methyl 3-((4-(tert-butyl)benzyl)thio)-6-chloropicolinate

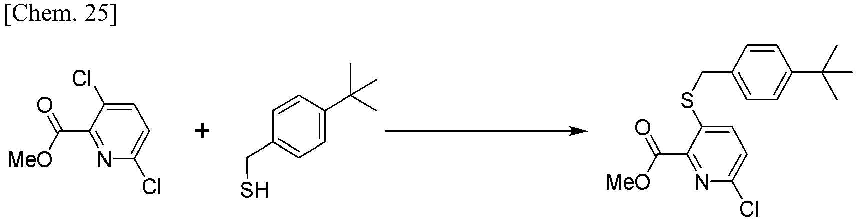

To a solution of methyl 3,6-dichloropicolinate (20 g, 97 mmol) in tetrahydrofuran (0.50 L), sodium hydride (60 wt%, 4.70 g, 0.12 mol) was added with cooling in an ice bath, and the mixture was stirred for 15 minutes. To this, (4-(tert-butyl)phenyl)methanethiol (18 mL, 0.10 mol) was added dropwise, and the mixture was stirred at room temperature for 3 hours. A saturated aqueous ammonium chloride solution was added to the reaction mixture with cooling in an ice bath, and ethyl acetate extraction was performed. The organic layer was dried over anhydrous sodium sulfate and then concentrated. The residue was washed with n-hexane to give methyl 3-((4-(tert-butyl)benzyl)thio)-6-chloropicolinate (30 g, 86 mmol).
Yield: 88%
Physical property: ¹H-NMR (CDCl₃): δ 7.66-7.63 (m, 1H), 7.37-7.28 (m, 5H), 4.12 (s, 2H), 3.98 (s, 3H), 1.31 (s, 9H)

### Reference Production Example 2-2

### Production of 3-((4-(tert-butyl)benzyl)thio)-6-chloropicolinic acid

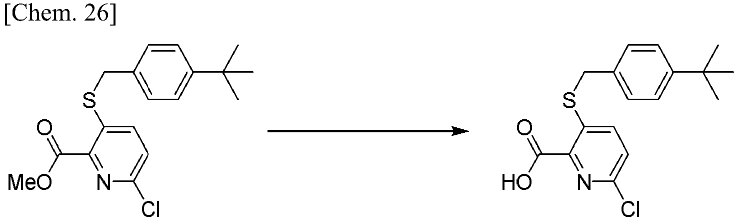

To a solution of methyl 3-((4-(tert-butyl)benzyl)thio)-6-chloropicolinate (30 g, 86 mmol) in methanol (200 mL) and tetrahydrofuran (50 mL), lithium hydroxide (4.3 g, 0.10 mol) was added at room temperature, and the mixture was stirred at room temperature for 6 hours. The reaction mixture was concentrated in vacuo, and 2 M hydrochloric acid was added to the residue. The resulting solid was collected by filtration and washed with n-hexane to give 3-((4-(tert-butyl)benzyl)thio)-6-chloropicolinic acid (30 g, 85 mmol).
Yield: 100%

### Reference Production Example 2-3

### Production of 3-((4-(tert-butyl)benzyl)thio)-6-chloro-2-(1-methyl-3-phenyl-1H-1,2,4-triazol-5-yl)pyridine

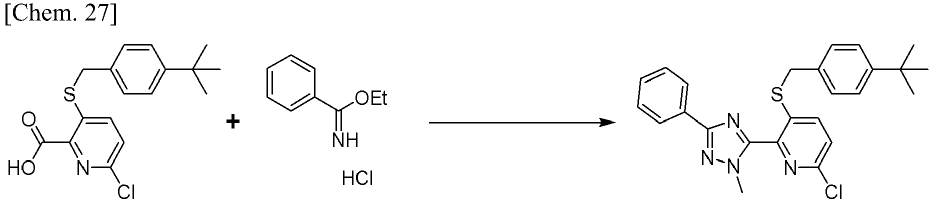

To a solution of 3-((4-(tert-butyl)benzyl)thio)-6-chloropicolinic acid (10 g, 30 mmol) and ethyl benzimidate hydrochloride (5.6 g, 30 mmol) in N,N-dimethylformamide (60 mL), N,N-diisopropylethylamine (15 mL, 90 mmol) and 1-[bis(dimethylamino)methylene]-1H-benzotriazolium 3-oxide hexafluorophosphate (17 g, 45 mmol) were added, and the mixture was stirred at room temperature overnight. Water was added to the reaction mixture, and chloroform extraction was performed. The organic layer was washed with saturated brine, dried over anhydrous sodium sulfate, and concentrated in vacuo. The residue was dissolved in ethanol (60 mL), methylhydrazine (2.4 mL, 45 mmol) was added, and the mixture was heated under reflux for 2 hours. The reaction mixture was concentrated in vacuo, and the residue was purified by silica gel column chromatography to give 3-((4-(tert-butyl)benzyl)thio)-6-chloro-2-(1-methyl-3-phenyl-1H-1,2,4-triazol-5-yl)pyridine (2.1 g, 4.6 mmol).
Yield: 5%

### Reference Production Example 2-4

### Production of 6-chloro-N-methyl-2-(1-methyl-3-phenyl-1H-1,2,4-triazol-5-yl)pyridine-3-sulfonamide

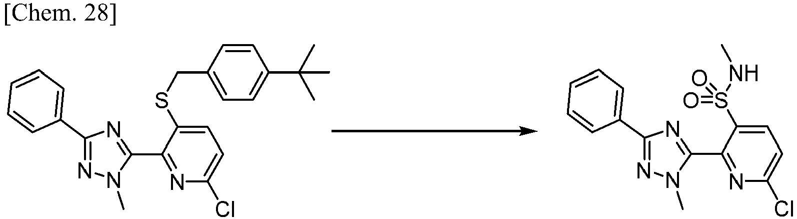

To a solution of 3-((4-(tert-butyl)benzyl)thio)-6-chloro-2-(1-methyl-3-phenyl-1H-1,2,4-triazol-5-yl)pyridine (2.1 g, 4.6 mmol) in chloroform (50 mL), water (0.50 mL, 27 mmol) and acetic acid (0.80 mL, 14 mmol) were added, and 1,3-dichloro-5,5-dimethylhydantoin (2.7 g, 14 mmol) was added with cooling in an ice bath. After the disappearance of the starting compound was confirmed, the reaction mixture was added dropwise to a methylamine-methanol solution (9.8 mol/L, 20 mL) with cooling in an ice bath, and the mixture was stirred for 30 minutes. A saturated aqueous ammonium chloride solution was further added, and ethyl acetate extraction was performed. The organic layer was concentrated, and the residue was purified by silica gel column chromatography to give 6-chloro-N-methyl-2-(1-methyl-3-phenyl-1H-1,2,4-triazol-5-yl)pyridine-3-sulfonamide (0.70 g, 1.9 mmol).
Yield: 42%
Physical property: ¹H-NMR (CDCl₃): δ 8.46 (d, 1H), 8.14 (q, 1H), 8.00-7.95 (m, 2H), 7.59 (d, 1H), 7.50-7.41 (m, 3H), 4.18 (s, 3H), 2.80 (s, 3H)

### Reference Production Example 3

### Production of 2-chloro-6-(3,5-dimethyl-1H-1,2,4-triazol-1-yl)-N-methylpyridine-3-sulfonamide

### Reference Production Example 3-1

### Production of 2,6-dichloro-N-methylpyridine-3-sulfonamide

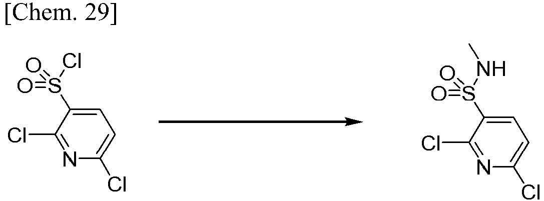

To a solution of 2,6-dichloropyridine-3-sulfonylchloride in tetrahydrofuran (0.20 L), a 7.0% solution of methylamine in tetrahydrofuran (31 mL, 61 mmol) was added under ice cooling, and the mixture was stirred at room temperature for 1 hour. After the completion of the reaction, a saturated aqueous ammonium chloride solution and ethyl acetate were added to the reaction mixture for extraction. The organic layer was dried over anhydrous sodium sulfate and then concentrated in vacuo. The residue was purified by silica gel column chromatography to give 2,6-dichloro-N-methylpyridine-3-sulfonamide (6.4 g, 27 mmol).
Yield: 44%
Physical property: ¹H-NMR (CDCl₃): δ 8.35 (d, 1H), 7.46 (d, 1H), 5.10(br.s, 1H), 2.71 (d, 3H)

### Reference Production Example 3-2

### Production of 2-chloro-6-(3,5-dimethyl-1H-1,2,4-triazol-1-yl)-N-methylpyridine-3-sulfonamide

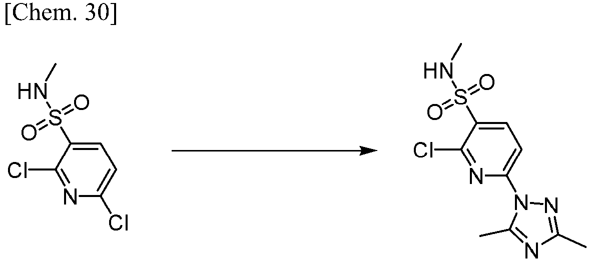

To a solution of 2,6-dichloro-N-methylpyridine-3-sulfonamide (5.0 g, 21 mmol) in N,N-dimethylacetamide (42 mL), 3,5-dimethyl-1H-1,2,4-triazole (3.0 g, 31 mmol) and cesium carbonate (14 g, 42 mmol) were added, and the mixture was stirred at 100°C for 2 hours. Water was added to the reaction mixture, and ethyl acetate extraction was performed. The organic layer was concentrated in vacuo. The residue was purified by silica gel column chromatography to give 2-chloro-6-(3,5-dimethyl-1H-1,2,4-triazol-1-yl)-N-methylpyridine-3-sulfonamide (1.2 g, 4.0 mmol).
Yield: 19%

### Reference Production Example 4

### Production of 6-(methanesulfonyl)-N-methyl-2-(5-methyl-3-phenyl-1H-pyrazol-1-yl)pyridine-3-sulfonamide

### Reference Production Example 4-1

### Production of 2-chloro-N-methyl-6-(methylsulfanyl)pyridine-3-sulfonamide

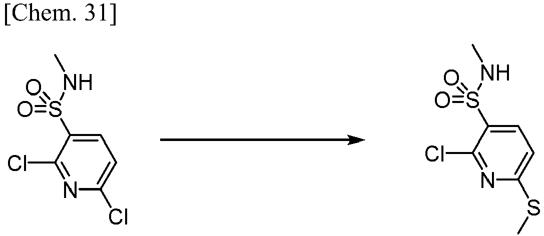

To a solution of 2,6-dichloro-N-methylpyridine-3-sulfonamide (3.4 g, 14 mmol) in N,N-dimethylacetamide (28 mL), methyl mercaptan sodium (1.5 g, 21 mmol) was added, and the mixture was stirred at room temperature for 7 hours. Water was added to the reaction mixture, and ethyl acetate extraction was performed. The organic layer was concentrated in vacuo. The residue was purified by silica gel column chromatography to give 2-chloro-N-methyl-6-(methylsulfanyl)pyridine-3-sulfonamide (3.6 g, 14 mmol) as a crude product.
Yield: 100%

### Reference Production Example 4-2

### Production of 6-N-methyl-2-(5-methyl-3-phenyl-1H-pyrazol-1-yl)-6-(methylsulfanyl)pyridine-3-sulfonamide

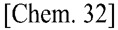

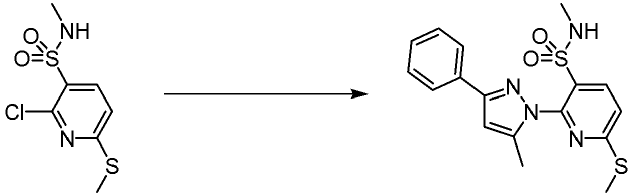

To a solution of the crude product of 2-chloro-N-methyl-6-(methylsulfanyl)pyridine-3-sulfonamide (3.4 g, 13 mmol) in N,N-dimethylacetamide (25 mL), 5-methyl-3-phenyl-1H-pyrazole (1.1 g, 7.0 mmol) and potassium carbonate (2.8 g, 20 mmol) were added, and the mixture was stirred at 130°C for 16 hours. Water was added to the reaction mixture, and ethyl acetate extraction was performed. The organic layer was concentrated in vacuo. The residue was purified by silica gel column chromatography to give 6-N-methyl-2-(5-methyl-3-phenyl-1H-pyrazol-1-yl)-6-(methylsulfanyl)pyridine-3-sulfonamide (0.32 g, 0.85 mmol).
Yield: 13%

### Reference Production Example 4-3

### Production of 6-(methanesulfonyl)-N-methyl-2-(5-methyl-3-phenyl-1H-pyrazol-1-yl)pyridine-3-sulfonamide

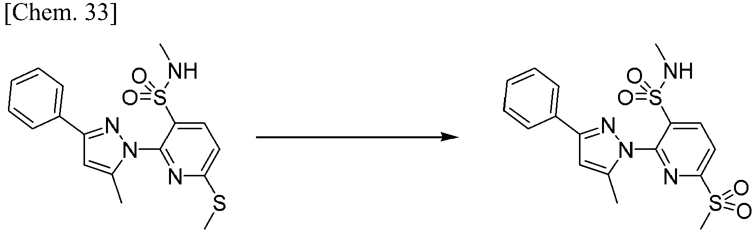

To a solution of N-methyl-2-(5-methyl-3-phenyl-1H-pyrazol-1-yl)-6-(methylsulfanyl)pyridine-3-sulfonamide (0.32 g, 0.85 mmol) in ethyl acetate (5.0 mL), m-chloroperbenzoic acid (water content: 30%, 0.63 g, 2.5 mmol) was added, and the mixture was stirred at room temperature for 6 hours. A saturated aqueous sodium hydrogen carbonate solution and a saturated aqueous sodium thiosulfate solution were added to the reaction mixture, and ethyl acetate extraction was performed. The organic layer was concentrated in vacuo. The residue was purified by column chromatography to give 6-(methanesulfonyl)-N-methyl-2-(5-methyl-3-phenyl-1H-pyrazol-1-yl)pyridine-3-sulfonamide (0.21 g, 0.52 mmol).
Yield: 60%

### Reference Production Example 5

### Production of 6-chloro-N-methyl-2-(1-methyl-4-phenyl-1H-imidazol-2-yl)pyridine-3-sulfonamide

### Reference Production Example 5-1

### Production of 2-oxo-2-phenylethyl 3-((4-(tert-butyl)benzyl)thio)-6-chloropicolinate

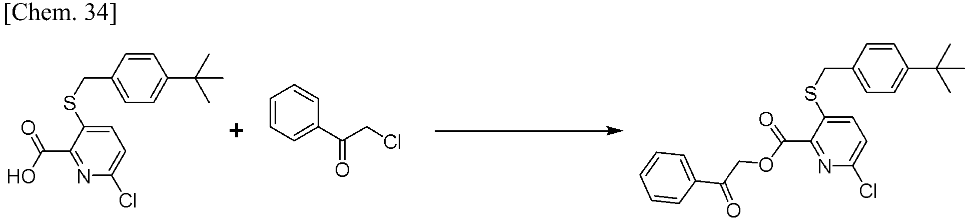

To a solution of 3-((4-(tert-butyl)benzyl)thio)-6-chloropicolinic acid (6.6 g, 20 mmol) in N,N-dimethylformamide (28 mL), phenacyl chloride (3.0 g, 20 mmol) and N,N-diisopropylethylamine (8.3 mL, 49 mmol) were added under ice cooling, and the mixture was stirred at room temperature overnight. After the completion of the reaction, water and ethyl acetate were added to the reaction mixture for extraction. The organic layer was dried over anhydrous sodium sulfate and then concentrated in vacuo to give 2-oxo-2-phenylethyl 3-((4-(tert-butyl)benzyl)thio)-6-chloropicolinate as a crude product (7.8 g, 17 mmol).
Yield: 88%

### Reference Production Example 5-2

### Production of 3-((4-(tert-butyl)benzyl)thio)-6-chloro-2-(4-phenyl-1H-imidazol-2-yl)pyridine

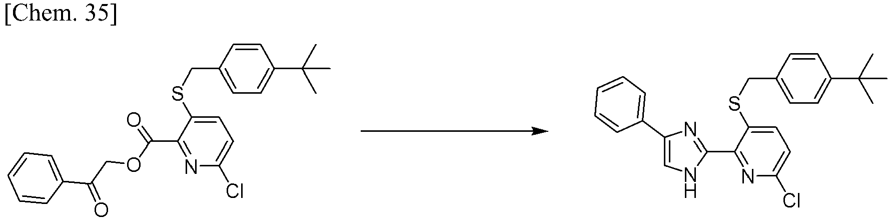

To a solution of 2-oxo-2-phenylethyl 3-((4-(tert-butyl)benzyl)thio)-6-chloropicolinate (7.8 g, 17 mmol) in acetic acid (86 mL), ammonium acetate (27 g, 0.34 mol) was added, and the mixture was stirred and heated under reflux. The reaction mixture was concentrated in vacuo, and extraction was performed with a saturated aqueous sodium hydrogen carbonate solution and ethyl acetate. The organic layer was dried over anhydrous sodium sulfate and then concentrated in vacuo. The residue was purified by silica gel column chromatography to give 3-((4-(tert-butyl)benzyl)thio)-6-chloro-2-(4-phenyl-1H-imidazol-2-yl)pyridine (7.4 g, 17 mmol).
Yield: 99%

### Reference Production Example 5-3

### Production of 3-((4-(tert-butyl)benzyl)thio)-6-chloro-2-(1-methyl-4-phenyl-1H-imidazol-2-yl)pyridine

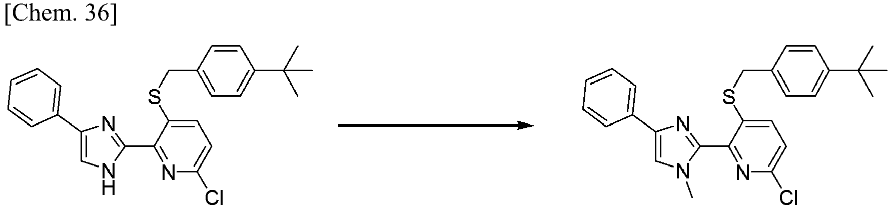

To a solution of 3-((4-(tert-butyl)benzyl)thio)-6-chloro-2-(4-phenyl-1H-imidazol-2-yl)pyridine (7.4 g, 17 mmol) in tetrahydrofuran (90 mL), potassium tert-butoxide (2.1 g, 18.8 mmol) and iodomethane (1.2 mL, 19 mmol) were added under ice cooling, and the mixture was stirred at room temperature for 2 hours. A saturated aqueous ammonium chloride solution and ethyl acetate were added to the reaction mixture for extraction. The organic layer was dried over sodium sulfate and then concentrated. The residue was purified by silica gel column chromatography to give 3-((4-(tert-butyl)benzyl)thio)-6-chloro-2-(1-methyl-4-phenyl-1H-imidazol-2-yl)pyridine (4.6 g, 10 mmol).
Yield: 59%

### Reference Production Example 5-4

### Production of 6-chloro-N-methyl-2-(1-methyl-4-phenyl-1H-imidazol-2-yl)pyridine-3-sulfonamide

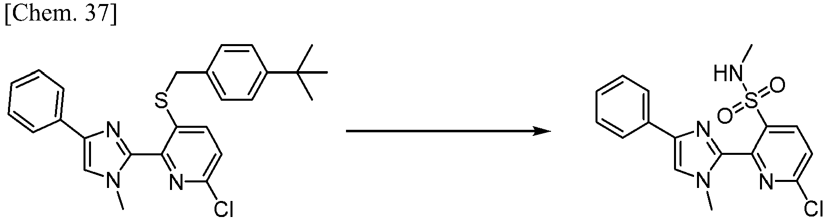

To a solution of 3-((4-(tert-butyl)benzyl)thio)-6-chloro-2-(1-methyl-3-phenyl-1H-imidazol-2-yl)pyridine (2.0 g, 4.5 mmol) in chloroform (50 mL), water (0.50 mL, 27 mmol) and acetic acid (0.77 mL, 13 mmol) were added, and 1,3-dichloro-5,5-dimethylhydantoin (2.6 g, 13 mmol) was added with cooling in an ice bath. After the disappearance of the starting compound was confirmed, a methylamine-methanol solution (30 mL) was added dropwise to the reaction mixture with cooling in an ice bath, and the mixture was stirred for 30 minutes. A saturated aqueous ammonium chloride solution was added, ethyl acetate extraction was performed, and the organic layer was concentrated. The residue was purified by silica gel column chromatography to give 6-chloro-N-methyl-2-(1-methyl-4-phenyl-1H-imidazol-2-yl)pyridine-3-sulfonamide (0.51 mg, 1.4 mmol).
Yield: 31%
Physical property: ¹H-NMR (CDCl₃): δ 8.95 (q, 1H), 8.42 (d, 1H), 7.67-7.63 (m, 2H), 7.48-7.38 (m, 3H), 7.33-7.29 (m, 2H), 3.97 (s, 3H), 2.78 (d, 3H)

### Reference Production Example 6

### Production of 6-chloro-N-methyl-2-[1-methyl-3-(thiophen-2-yl)-1H-1,2,4-triazol-5-yl]pyridine-3-sulfonamide

### Reference Production Example 6-1

### Production of ethyl thiophene-2-carboximidate hydrochloride

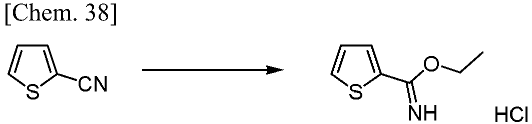

To a solution of thiophene-2-carbonitrile (5.0 g, 46 mmol) in ethanol (28 mL) in an ice bath, acetyl chloride (33 mL, 0.46 mol) was added dropwise. The reaction mixture was stirred at room temperature for 72 hours and then concentrated in vacuo. The residue was washed with hexane to give ethyl thiophene-2-carboximidate hydrochloride (8.3 g, 43 mmol) as a crude product.
Yield: 95%

### Reference Production Example 6-2

### Production of N'-methylthiophene-2-carbohydrazonamide hydrochloride

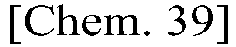

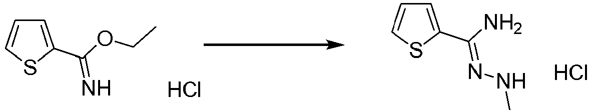

To a solution of ethyl thiophene-2-carboximidate hydrochloride (4.0 g, 21 mmol) in methanol (20 mL), 1-methylhydrazine (1.3 mL, 25 mmol) was added, and the mixture was stirred at room temperature for 18 hours. The reaction mixture was concentrated in vacuo, and the residue was washed with methyl tert-butyl ether to give N'-methylthiophene-2-carbohydrazonamide hydrochloride as a crude product.

### Reference Production Example 6-3

### Production of 6-chloro-N-methyl-2-[1-methyl-3-(thiophen-2-yl)-1H-1,2,4-triazol-5-yl]pyridine-3-sulfonamide

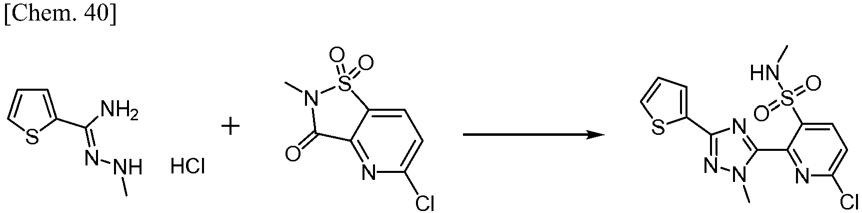

To a solution of N'-methylthiophene-2-carbohydrazonamide hydrochloride (1.4 g, 7.3 mmol) and 5-chloro-2-methyl-1H-1λ⁶-[1,2]thiazolo[4,5-b]pyridine-1,1,3(2H)-trione (1.3 g, 5.8 mmol), produced by the method described in Reference Production Example 8 or 9 described later, in toluene (10 mL), sodium acetate (0.60 g, 7.5 mmol) was added and the mixture was heated to reflux for 6 hours. The reaction mixture was concentrated in vacuo, and the residue was purified by silica gel column chromatography to give 6-chloro-N-methyl-2-[1-methyl-3-(thiophen-2-yl)-1H-1,2,4-triazol-5-yl]pyridine-3-sulfonamide (0.96 g, 2.6 mmol).
Yield: 44%
Physical properties: ¹H-NMR (CDCl₃): δ 8.45 (d, 1H), 7.91-7.86 (m, 1H), 7.63-7.61 (m, 1H), 7.59 (d, 1H), 7.40-7.37 (m, 1H), 7.13-7.10 (m, 1H), 4.16 (s, 3H), 2.80 (d, 3H).

### Reference Production Example 7

### Production of 6-chloro-2-(1-ethyl-3-phenyl-1H-1,2,4-triazol-5-yl)-N-methylpyridine-3-sulfonamide

### Reference Production Example 7-1

### Production of N'-ethyl-benzene-carbohydrazonamide hydrochloride

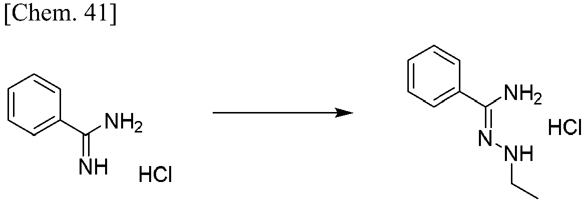

To a solution of benzene carboximidamide hydrochloride (5.0 g, 32 mmol) in methanol (15 mL), 1-ethylhydrazine (1.8 mL, 38 mmol) was added, and the mixture was stirred at room temperature for 18 hours. The reaction mixture was concentrated in vacuo, and the residue was washed with hexane to give N'-ethyl-benzene-carbohydrazonamide hydrochloride as a crude product.

### Reference Production Example 7-2

### Production of 6-chloro-2-(1-ethyl-3-phenyl-1H-1,2,4-triazol-5-yl)-N-methylpyridine-3-sulfonamide

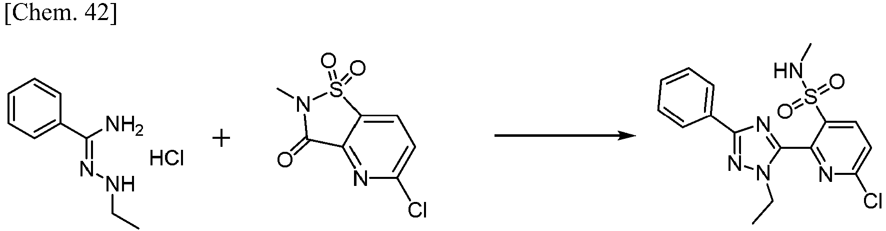

To a solution of N'-ethyl-benzene-carbohydrazonamide hydrochloride (5.0 g, 25 mmol) and 5-chloro-2-methyl-1H-1λ⁶-[1,2]thiazolo[4,5-b]pyridine-1,1,3(2H)-trione (4.5 g, 19 mmol), produced by the method described in Reference Production Example 8 or 9 described later, in acetic acid (10 mL) and toluene (10 mL), sodium acetate (5.3 g, 64 mmol) was added and the mixture was heated to reflux for 3 hours. The reaction mixture was concentrated in vacuo, and the residue was purified by silica gel column chromatography to give 6-chloro-2-(1-ethyl-3-phenyl-1H-1,2,4-triazol-5-yl)-N-methylpyridine-3-sulfonamide (2.6 g, 13 mmol).
Yield: 36%
Physical properties: ¹H-NMR (CDCl₃): δ 8.45 (d, 1H), 8.07-8.02 (m, 1H), 8.02-7.95 (m, 2H), 7.60 (d, 1H), 7.49-7.42 (m, 3H), 4.48 (q, 2H), 2.80 (d, 3H), 1.58 (t, 3H)

### Reference Production Example 8

### Production of 5-chloro-2-methyl-1H-1λ⁶-[1,2]thiazolo[4,5-b]pyridine-1,1,3(2H)-trione Reference Production Example 8-1

### Production of 6-chloro-N-methyl-3-(methylsulfanyl)pyridine-2-carboxamide

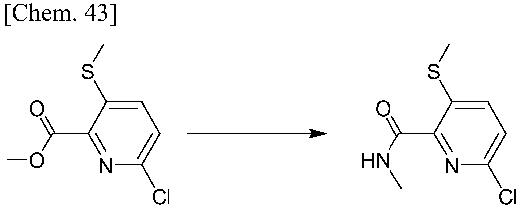

To a solution of methyl 6-chloro-3-(methylsulfanyl)pyridine-2-carboxylate (50 g, 0.23 mol) in methanol (0.40 L), a methylamine solution in methanol (70 mL, 9.8 mol/L, 0.69 mol) was added at room temperature, and the mixture was stirred overnight. The reaction mixture was concentrated in vacuo, and hexane was added. The precipitated solid was collected by filtration to give 6-chloro-N-methyl-3-(methylsulfanyl)pyridine-2-carboxamide (50 g, 0.23 mol).
Yield: 100%
Physical properties: ¹H-NMR (DMSO-d₆): δ 8.54 (s, 1H), 7.86 (d, 1H), 7.63 (d, 1H), 2.77 (d, 3H), 2.39 (s, 3H)

### Reference Production Example 8-2

### Production of 5-chloro-2-methyl[1,2]thiazolo[4,5-b]pyridine-3(2H)-one

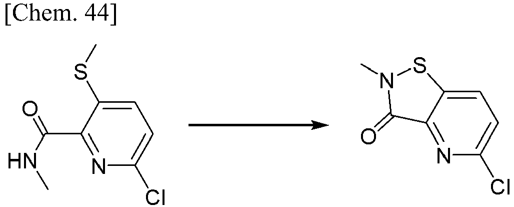

To a solution of 6-chloro-N-methyl-3-(methylsulfanyl)pyridine-2-carboxamide (10 g, 46 mmol) in chloroform (90 mL), sulfuryl chloride (6.3 mL, 50 mmol) was added dropwise at room temperature, and the mixture was stirred at room temperature for 30 minutes, and further stirred at 50°C for 30 minutes. Methyl tert-butyl ether was added to the reaction mixture, and the precipitated solid was collected by filtration to give 5-chloro-2-methyl[1,2]thiazolo[4,5-b]pyridine-3(2H)-one (7.1 g, 35 mmol).
Yield: 76%
Physical properties: ¹H-NMR (CDCl₃): δ 7.95 (d, 1H), 7.55 (d, 1H), 3.51 (s, 3H)

### Reference Production Example 8-3

### Production of 5-chloro-2-methyl-1H-1λ⁶-[1,2]thiazolo[4,5-b]pyridine-1,1,3(2H)-trione

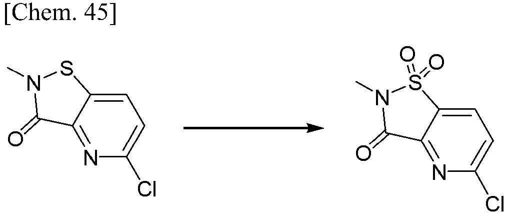

To a solution of 5-chloro-2-methyl[1,2]thiazolo[4,5-b]pyridine-3(2H)-one (6.6 g, 33 mmol) in ethyl acetate (100 mL), m-chloroperoxybenzoic acid (20.4 g, 83 mmol) was added at 0°C, and the mixture was stirred at room temperature overnight. A saturated aqueous sodium hydrogen carbonate solution was added to the reaction mixture, and then a saturated aqueous sodium thiosulfate solution was added. Ethyl acetate extraction was performed. The extract was dried over anhydrous sodium sulfate, and concentrated in vacuo to give 5-chloro-2-methyl-1H-1λ⁶-[1,2]thiazolo[4,5-b]pyridine-1,1,3(2H)-trione (6.5 g, 28 mmol).
Yield: 85%
Physical properties: ¹H-NMR (CDCl₃): δ 8.24 (d, 1H), 7.80 (d, 1H), 3.34 (s, 3H)

### Reference Production Example 9 (alternative method of Reference Production Example 8)

### Production of 5-chloro-2-methyl-1H-1λ⁶-[1,2]thiazolo[4,5-b]pyridine-1,1,3(2H)-trione Reference Production Example 9-1

### Production of 6-chloro-3-(methylsulfamoyl)pyridine-2-carboxylic acid

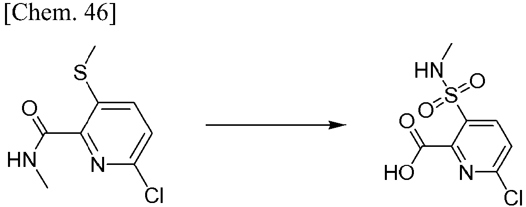

To a solution of 6-chloro-N-methyl-3-(methylsulfanyl)pyridine-2-carboxamide (5.0 g, 24 mmol) in chlorobenzene (50 mL), water (1.3 mL, 73 mmol) was added, and sulfuryl chloride (6.0 mL, 73 mmol) was added dropwise at room temperature. The mixture was stirred at 70°C for 90 minutes. Methyl tert-butyl ether was added to the reaction mixture, and the precipitated solid was collected by filtration to give 6-chloro-3-(methylsulfamoyl)pyridine-2-carboxylic acid (5.1 g, 20 mmol).
Yield: 83%
Physical properties: ¹H-NMR (DMSO-d₆): δ 8.22-8.16 (brs, 1H), 8.13 (d, 1H), 7.54 (d, 1H), 2.71 (d, 3H)

### Reference Production Example 9-2

### Production of 5-chloro-2-methyl-1H-1λ⁶-[1,2]thiazolo[4,5-b]pyridine-1,1,3(2H)-trione

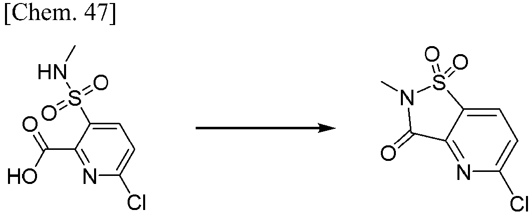

To a suspension of 6-chloro-3-(methylsulfamoyl)pyridine-2-carboxylic acid (1.0 g, 4.0 mmol) in toluene (0.87 mL), N,N-dimethylformamide (70 mg, 1.0 mmol) and thionyl chloride (0.87 mL, 12 mmol) were added at room temperature, and the mixture was stirred at 80°C. Water was added to the reaction mixture at room temperature, and ethyl acetate extraction was performed. The extract was dried over sodium sulfate, and concentrated in vacuo. The residue was washed with ethanol and collected by filtration to give 5-chloro-2-methyl-1H-1λ⁶-[1,2]thiazolo[4,5-b]pyridine-1,1,3(2H)-trione (0.75 g, 3.2 mmol).
Yield: 81%
Physical properties: ¹H-NMR (CDCl₃):δ 8.24 (d, 1H), 7.80 (d, 1H), 3.34 (s, 3H)

Hereinafter, examples of formulations containing the compound of the present invention are shown, but the present invention is not limited thereto. In the formulation examples, "part" means part by weight.

### Formulation Example 1

| | |
|---|---|
| Compound of the present invention | 10 parts |
| Xylene | 70 parts |
| N-methylpyrrolidone | 10 parts |
| Mixture of polyoxyethylene nonylphenyl ether and calcium alkylbenzene sulfonate | 10 parts |

The above ingredients are uniformly mixed for dissolution to give an emulsifiable concentrate formulation.

### Formulation Example 2

| | |
|---|---|
| Compound of the present invention | 3 parts |
| Clay powder | 82 parts |
| Diatomite powder | 15 parts |

The above ingredients are uniformly mixed and then pulverized to give a dust formulation.

### Formulation Example 3

| | |
|---|---|
| Compound of the present invention | 5 parts |
| Mixture of bentonite powder and clay powder | 90 parts |
| Calcium lignosulfonate | 5 parts |

The above ingredients are uniformly mixed. After addition of an appropriate volume of water, the mixture is kneaded, granulated and dried to give a granular formulation.

### Formulation Example 4

| | |
|---|---|
| Compound of the present invention | 20 parts |
| Kaolin and synthetic high-dispersion silicic acid | 75 parts |
| Mixture of polyoxyethylene nonylphenyl ether and calcium alkylbenzene sulfonate | 5 parts |

The above ingredients are uniformly mixed and then pulverized to give a wettable powder formulation.

### Test Example 1: Test for post-emergence herbicidal effect against paddy weeds

Barnyard grass (*Echinochloa crus-galli*) was seeded and grown in test tubes containing hydroponic medium in an artificial climate chamber or a phytotron. Agrochemical formulations containing the compounds of the present invention as active ingredients prepared according to Formulation Example 1 were separately diluted with water so that the concentration of the active ingredient would be a predetermined concentration and used for drop treatment of the barnyard grass. The barnyard grass was grown in the artificial climate chamber at 30°C under full light conditions. Six days after agrochemical treatment, the herbicidal effect was evaluated as compared to the untreated control according to the following criteria.

### Criteria for herbicidal effect (degree of growth inhibition) and phytotoxicity

| | |
|---|---|
| Score 4 | 90% to 100% herbicidal effect |
| Score 3 | 70% to 89% herbicidal effect |
| Score 2 | 40% to 69% herbicidal effect |
| Score 1 | 1% to 39% herbicidal effect |
| Score 0 | 0% herbicidal effect |

As a result of Test Example 1, among the compounds represented by the general formula (I) of the present invention, compounds numbered 1-1, 2-1, 2-2, 2-3, 2-4, 2-5, 2-6, 2-7, 2-8, 2-9, 2-11, 2-12, 2-13, 2-14, 2-15, 2-16, 2-17, 2-18, 2-19, 2-20, 2-21, 2-22, 2-23, 2-25, 2-26, 2-27, 2-28, 2-29, 2-30, 2-31, 2-32, 2-33, 2-42, 2-43, 2-44, 2-45, 2-47, 2-48, 2-49, 2-51, 2-52, 2-53, 2-54, 2-56, 2-57, 2-58, 2-59, 2-60, 2-61, 2-62, 2-63, 2-64, 2-65, 2-66, 2-67, 2-68, 2-69, 2-70, 2-71, 2-72, 2-73, 2-75, 2-76, 2-77, 2-78, 2-79, 2-80, 2-81, 2-84, 2-85, 2-86, 2-87, 2-88, 2-89, 2-90, 2-91, 2-92, 2-93, 2-94, 2-95, 2-99, 2-101, 2-103, 2-104, 2-105, 2-106, 2-107, 2-110, 2-111, 2-113, 2-171, 2-174, 2-176, 2-178, 2-182, 2-185, 2-186, 2-187, 2-188, 2-199, 3-1, 3-2, 3-3, 3-7, 3-8, 3-13, 3-14 and 3-25 showed herbicidal effect against barnyard grass in score 3 or higher according to the above criteria when applied at an active ingredient concentration of 10 ppm.

### INDUSTRIAL APPLICABILITY

The compound represented by the general formula (I) of the present invention or a salt thereof is a highly effective agricultural or horticultural herbicide.

## Claims

1. A compound represented by the general formula (I): 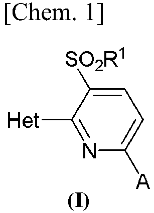 {wherein
R¹ represents
(a1) a (C₁-C₆) alkyl group;
(a2) a halo (C₁-C₆) alkyl group; or
(a3) an N(R^{2a})R^{2b} group
(wherein R^{2a} and R^{2b} represent
(b1) a hydrogen atom,
(b2) a (C₁-C₆) alkyl group,
(b3) a (C₂-C₆) alkenyl group,
(b4) a (C₂-C₆) alkynyl group,
(b5) a (C₃-C₆) cycloalkyl group,
(b6) a halo (C₁-C₆) alkyl group,
(b7) a (C₁-C₇) alkylcarbonyl group,
(b8) a (C₁-C₆) alkoxycarbonyl group,
(b9) a halo (C₁-C₇) alkylcarbonyl group, or
(b10) a halo (C₁-C₆) alkoxycarbonyl group, and
R^{2a} and R^{2b} may be the same or different), and
Het represents the formula (Het-1) or the formula (Het-2): 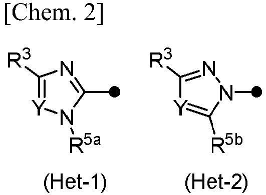 (wherein
R³ represents
(c1) a halogen atom;
(c2) a cyano atom;
(c3) a (C₁-C₆) alkyl group;
(c4) a (C₂-C₆) alkenyl group;
(c5) a (C₂-C₆) alkynyl group;
(c6) a (C₃-C₆) cycloalkyl group;
(c7) a (C₁-C₆) alkoxy group;
(c8) a halo (C₁-C₆) alkyl group;
(c9) a halo (C₂-C₆) alkenyl group;
(c10) a halo (C₂-C₆) alkynyl group;
(c11) a halo (C₃-C₆) cycloalkyl group;
(c12) a halo (C₁-C₆) alkoxy group;
(c13) a substituted (C₁-C₆) alkyl group having 1 to 3 substituents, each independently selected from a set S of substituents;
(c14) a substituted (C₃-C₆) cycloalkyl group having 1 to 3 substituents, each independently selected from a set T of substituents;
(c15) a (C₁-C₆) alkylsulfanyl group;
(c16) a (C₁-C₆) alkylsulfinyl group;
(c17) a (C₁-C₆) alkylsulfonyl group;
(c18) a (C₁-C₆) alkoxycarbonyl group;
(c19) a furanyl group;
(c20) a substituted furanyl group having 1 to 3 substituents on the ring, each independently selected from a set U of substituents;
(c21) an oxazolyl group;
(c22) a substituted oxazolyl group having 1 to 3 substituents on the ring, each independently selected from the set U of substituents;
(c23) a thienyl group;
(c24) a substituted thienyl group having 1 to 3 substituents on the ring, each independently selected from the set U of substituents;
(c25) a thiazolyl group;
(c26) a substituted thiazolyl group having 1 or 2 substituents on the ring, each independently selected from the set U of substituents;
(c27) a naphthyl group;
(c28) a substituted naphthyl group having 1 to 7 substituents on the ring, each independently selected from the set U of substituents;
(c29) a phenyl group;
(c30) a substituted phenyl group having 1 to 5 substituents on the ring, each independently selected from the set U of substituents;
(c31) a pyridyl group;
(c32) a substituted pyridyl group having 1 to 4 substituents on the ring, each independently selected from the set U of substituents;
(c33) a pyridazinyl group;
(c34) a substituted pyridazinyl group having 1 to 3 substituents on the ring, each independently selected from the set U of substituents;
(c35) a pyrimidinyl group;
(c36) a substituted pyrimidinyl group having 1 to 3 substituents on the ring, each independently selected from the set U of substituents;
(c37) a pyrazinyl group;
(c38) a substituted pyrazinyl group having 1 to 3 substituents on the ring, each independently selected from the set U of substituents;
(c39) a phenyl (C₁-C₆) alkyl group; or
(c40) a substituted phenyl (C₁-C₆) alkyl group having 1 to 5 substituents on the ring, each independently selected from the set U of substituents,
Y represents a nitrogen atom or CR⁴(wherein R⁴ represents a hydrogen atom, a halogen atom or a (C₁-C₆) alkyl group),
R^{5a} represents
(d1) a hydrogen atom;
(d2) a (C₁-C₆) alkyl group; or
(d3) a halo (C₁-C₆) alkyl group,
R^{5b} represents
(e1) a hydrogen atom;
(e2) a halogen atom;
(e3) a (C₁-C₆) alkyl group; or
(e4) a halo (C₁-C₆) alkyl group,
the set S of substituents consists of
(f1) a cyano group;
(f2) a (C₃-C₆) cycloalkyl group;
(f3) a (C₁-C₆) alkoxy group;
(f4) a (C₁-C₆) alkylsulfanyl group;
(f5) a (C₁-C₆) alkylsulfinyl group;
(f6) a (C₁-C₆) alkylsulfonyl group;
(f7) a halo (C₃-C₆) cycloalkyl group;
(f8) a halo (C₁-C₆) alkoxy group;
(f9) a halo (C₁-C₆) alkylsulfanyl group;
(f10) a halo (C₁-C₆) alkylsulfinyl group; and
(f11) a halo (C₁-C₆) alkylsulfonyl group,
the set T of substituents consists of
(g1) a cyano group;
(g2) a (C₁-C₆) alkyl group;
(g3) a (C₃-C₆) cycloalkyl group;
(g4) a (C₁-C₆) alkoxy group;
(g5) a (C₁-C₆) alkylsulfanyl group;
(g6) a (C₁-C₆) alkylsulfinyl group;
(g7) a (C₁-C₆) alkylsulfonyl group;
(g8) a halo (C₁-C₆) alkyl group;
(g9) a halo (C₃-C₆) cycloalkyl group;
(g10) a halo (C₁-C₆) alkoxy group;
(g11) a halo (C₁-C₆) alkylsulfanyl group;
(g12) a halo (C₁-C₆) alkylsulfinyl group;
(g13) a halo (C₁-C₆) alkylsulfonyl group; and
(g14) a phenyl group,
the set U of substituents consists of
(h1) a halogen atom;
(h2) a cyano group;
(h3) a nitro group;
(h4) an amino group;
(h5) a hydroxy group;
(h6) a hydroxy (C₁-C₆) alkyl group;
(h7) a (C₁-C₆) alkyl group;
(h8) a (C₂-C₆) alkenyl group;
(h9) a (C₂-C₆) alkynyl group;
(h10) a (C₃-C₆) cycloalkyl group;
(h11) a halo (C₁-C₆) alkyl group;
(h12) a halo (C₂-C₆) alkenyl group;
(h13) a halo (C₂-C₆) alkynyl group;
(h14) a halo (C₃-C₆) cycloalkyl group;
(h15) a (C₁-C₆) alkoxy group;
(h16) a (C₁-C₆) alkylsulfanyl group;
(h17) a (C₁-C₆) alkylsulfinyl group;
(h18) a (C₁-C₆) alkylsulfonyl group;
(h19) a halo (C₁-C₆) alkoxy group;
(h20) a halo (C₁-C₆) alkylsulfanyl group;
(h21) a halo (C₁-C₆) alkylsulfinyl group;
(h22) a halo (C₁-C₆) alkylsulfonyl group;
(h23) an N-((C₁-C₆) alkylcarbonyl) amino group; and
(h24) an N-((C₁-C₆) alkylsulfonyl) amino group, and
each solid circle represents the position of binding),
A represents
(i1) an oxazolinyl group;
(i2) a substituted oxazolinyl group having 1 to 4 substituents on the ring, each independently selected from a set V of substituents;
(i3) an imidazolinyl group;
(i4) a substituted imidazolinyl group having 1 to 4 substituents on the ring, each independently selected from the set V of substituents;
(i5) an imidazolidinonyl group;
(i6) a substituted imidazolidinonyl group having 1 to 4 substituents on the ring, each independently selected from the set V of substituents;
(i7) a triazolinonyl group;
(i8) a substituted triazolinonyl group having 1 or 2 substituents on the ring, each independently selected from the set V of substituents;
(i9) a pyrazolinonyl group;
(i10) a substituted pyrazolinonyl group having 1 to 3 substituents on the ring, each independently selected from the set V of substituents;
(i11) a pyrazolyl group;
(i12) a substituted pyrazolyl group having 1 to 3 substituents on the ring, each independently selected from the set V of substituents;
(i13) a triazolyl group;
(i14) a substituted triazolyl group having 1 or 2 substituents on the ring, each independently selected from the set V of substituents;
(i15) a tetrazolyl group;
(i16) a substituted tetrazolyl group having 1 substituent on the ring, selected from the set V of substituents;
(i17) an imidazolyl group;
(i18) a substituted imidazolyl group having 1 to 3 substituents on the ring, each independently selected from the set V of substituents;
(i19) a pyrrolyl group;
(i20) a substituted pyrrolyl group having 1 to 4 substituents on the ring, each independently selected from the set V of substituents;
(i21) a thiazolyl group;
(i22) a substituted thiazolyl group having 1 or 2 substituents on the ring, each independently selected from the set V of substituents;
(i23) an oxazolyl group;
(i24) a substituted oxazolyl group having 1 or 2 substituents on the ring, each independently selected from the set V of substituents;
(i25) a tetrahydrofuranyl (C₁-C₆) alkoxy group;
(i26) a substituted tetrahydrofuranyl (C₁-C₆) alkoxy group having 1 to 4 substituents on the ring, each independently selected from the set V of substituents;
(i27) a dioxolanyl (C₁-C₆) alkoxy group;
(i28) a substituted dioxolanyl (C₁-C₆) alkoxy group having 1 to 4 substituents on the ring, each independently selected from the set V of substituents;
(i29) an isoxazolinyl (C₁-C₆) alkoxy group;
(i30) a substituted isoxazolinyl (C₁-C₆) alkoxy group having 1 to 4 substituents on the ring, each independently selected from the set V of substituents;
(i31) a pyrazolyl (C₁-C₆) alkoxy group;
(i32) a substituted pyrazolyl (C₁-C₆) alkoxy group having 1 to 3 substituents on the ring, each independently selected from the set V of substituents;
(i33) a triazolyl (C₁-C₆) alkoxy group;
(i34) a substituted triazolyl (C₁-C₆) alkoxy group having 1 or 2 substituents on the ring, each independently selected from the set V of substituents;
(i35) a tetrazolyl (C₁-C₆) alkoxy group;
(i36) a substituted tetrazolyl (C₁-C₆) alkoxy group having 1 substituent on the ring, selected from the set V of substituents;
(i37) an imidazolyl (C₁-C₆) alkoxy group;
(i38) a substituted imidazolyl (C₁-C₆) alkoxy group having 1 to 3 substituents on the ring, each independently selected from the set V of substituents;
(i39) a pyrrolyl (C₁-C₆) alkoxy group;
(i40) a substituted pyrrolyl (C₁-C₆) alkoxy group having 1 to 4 substituents on the ring, each independently selected from the set V of substituents;
(i41) a thiazolyl (C₁-C₆) alkoxy group;
(i42) a substituted thiazolyl (C₁-C₆) alkoxy group having 1 or 2 substituents on the ring, each independently selected from the set V of substituents;
(i43) an oxazolyl (C₁-C₆) alkoxy group; or
(i44) a substituted oxazolyl (C₁-C₆) alkoxy group having 1 or 2 substituents on the ring, each independently selected from the set V of substituents, and
the set V of substituents consists of
(j1) a halogen atom;
(j2) a cyano group;
(j3) a (C₁-C₆) alkyl group;
(j4) a (C₂-C₆) alkenyl group;
(j5) a (C₂-C₆) alkynyl group;
(j6) a (C₃-C₆) cycloalkyl group;
(j7) a halo (C₁-C₆) alkyl group;
(j8) a halo (C₂-C₆) alkenyl group;
(j9) a halo (C₂-C₆) alkynyl group;
(j10) a halo (C₃-C₆) cycloalkyl group;
(j11) a (C₁-C₆) alkoxy group;
(j12) a (C₁-C₆) alkylsulfanyl group;
(j13) a (C₁-C₆) alkylsulfinyl group;
(j14) a (C₁-C₆) alkylsulfonyl group;
(j15) a halo (C₁-C₆) alkoxy group;
(j16) a halo (C₁-C₆) alkylsulfanyl group;
(j17) a halo (C₁-C₆) alkylsulfinyl group;
(j18) a halo (C₁-C₆) alkylsulfonyl group;
(j19) a (C₁-C₆) alkoxy (C₁-C₆) alkyl group;
(j20) a (C₁-C₆) alkylsulfanyl (C₁-C₆) alkyl group;
(j21) a (C₁-C₆) alkoxycarbonyl group;
(j22) a phenyl group;
(j23) a substituted phenyl group having 1 to 5 substituents on the ring, each independently selected from the group consisting of a halogen atom, a (C₁-C₆) alkyl, and a halo (C₁-C₆) alkyl;
(j24) a thienyl group; and
(j25) a substituted thienyl group having 1 to 3 substituents on the ring, each independently selected from the group consisting of a halogen atom, a (C₁-C₆) alkyl, and a halo (C₁-C₆) alkyl}, or
a salt thereof.

2. The compound or the salt thereof according to claim 1, wherein
R¹ is
(a1) a (C₁-C₆) alkyl group; or
(a3') an N(R^{2a})R^{2b} group
(wherein R^{2a} and R^{2b} represent
(b1) a hydrogen atom,
(b2) a (C₁-C₆) alkyl group,
(b5) a (C₃-C₆) cycloalkyl group,
(b6) a halo (C₁-C₆) alkyl group,
(b7) a (C₁-C₇) alkylcarbonyl group, or
(b8) a (C₁-C₆) alkoxycarbonyl group, and
R^{2a} and R^{2b} may be the same or different),
Het is the formula (Het-1) or the formula (Het-2),
R³ is
(c1) a halogen atom;
(c3) a (C₁-C₆) alkyl group;
(c4) a (C₂-C₆) alkenyl group;
(c6) a (C₃-C₆) cycloalkyl group;
(c7) a (C₁-C₆) alkoxy group;
(c8) a halo (C₁-C₆) alkyl group;
(c9) a halo (C₂-C₆) alkenyl group;
(c11) a halo (C₃-C₆) cycloalkyl group;
(c13) a substituted (C₁-C₆) alkyl group having 1 to 3 substituents, each independently selected from a set S of substituents;
(c14) a substituted (C₃-C₆) cycloalkyl group having 1 to 3 substituents, each independently selected from a set T of substituents;
(c15) a (C₁-C₆) alkylsulfanyl group;
(c16) a (C₁-C₆) alkylsulfinyl group;
(c17) a (C₁-C₆) alkylsulfonyl group;
(c18) a (C₁-C₆) alkoxycarbonyl group;
(c19) a furanyl group;
(c20) a substituted furanyl group having 1 to 3 substituents on the ring, each independently selected from a set U of substituents;
(c23) a thienyl group;
(c24) a substituted thienyl group having 1 to 3 substituents on the ring, each independently selected from the set U of substituents;
(c25) a thiazolyl group;
(c26) a substituted thiazolyl group having 1 or 2 substituents on the ring, each independently selected from the set U of substituents;
(c27) a naphthyl group;
(c28) a substituted naphthyl group having 1 to 7 substituents on the ring, each independently selected from the set U of substituents;
(c29) a phenyl group;
(c30) a substituted phenyl group having 1 to 5 substituents on the ring, each independently selected from the set U of substituents;
(c31) a pyridyl group;
(c32) a substituted pyridyl group having 1 to 4 substituents on the ring, each independently selected from the set U of substituents;
(c39) a phenyl (C₁-C₆) alkyl group; or
(c40) a substituted phenyl (C₁-C₆) alkyl group having 1 to 5 substituents on the ring, each independently selected from the set U of substituents,
Y is a nitrogen atom or CR⁴ (wherein R⁴ represents a hydrogen atom),
R^{5a} is
(d1) a hydrogen atom; or
(d2) a (C₁-C₆) alkyl group,
R^{5b} is
(e1) a hydrogen atom; or
(e3) a (C₁-C₆) alkyl group,
the set S of substituents consists of
(f1) a cyano group;
(f2) a (C₃-C₆) cycloalkyl group;
(f3) a (C₁-C₆) alkoxy group;
(f4) a (C₁-C₆) alkylsulfanyl group;
(f5) a (C₁-C₆) alkylsulfinyl group; and
(f6) a (C₁-C₆) alkylsulfonyl group,
the set T of substituents consists of
(g1) a cyano group;
(g2) a (C₁-C₆) alkyl group;
(g8) a halo (C₁-C₆) alkyl group; and
(g14) a phenyl group,
the set U of substituents consists of
(h1) a halogen atom;
(h2) a cyano group;
(h5) a hydroxy group;
(h6) a hydroxy (C₁-C₆) alkyl group;
(h7) a (C₁-C₆) alkyl group;
(h10) a (C₃-C₆) cycloalkyl group;
(h11) a halo (C₁-C₆) alkyl group;
(h14) a halo (C₃-C₆) cycloalkyl group;
(h15) a (C₁-C₆) alkoxy group;
(h16) a (C₁-C₆) alkylsulfanyl group;
(h17) a (C₁-C₆) alkylsulfinyl group;
(h18) a (C₁-C₆) alkylsulfonyl group;
(h19) a halo (C₁-C₆) alkoxy group;
(h20) a halo (C₁-C₆) alkylsulfanyl group;
(h21) a halo (C₁-C₆) alkylsulfinyl group;
(h22) a halo (C₁-C₆) alkylsulfonyl group; and
(h23) an N-((C₁-C₆) alkylcarbonyl) amino group,
A is
(i1) an oxazolinyl group;
(i2) a substituted oxazolinyl group having 1 to 4 substituents on the ring, each independently selected from a set V of substituents;
(i3) an imidazolinyl group;
(i4) a substituted imidazolinyl group having 1 to 4 substituents on the ring, each independently selected from the set V of substituents;
(i5) an imidazolidinonyl group;
(i6) a substituted imidazolidinonyl group having 1 to 4 substituents on the ring, each independently selected from the set V of substituents;
(i7) a triazolinonyl group;
(i8) a substituted triazolinonyl group having 1 or 2 substituents on the ring, each independently selected from the set V of substituents;
(i9) a pyrazolinonyl group;
(i10) a substituted pyrazolinonyl group having 1 to 3 substituents on the ring, each independently selected from the set V of substituents;
(i11) a pyrazolyl group;
(i12) a substituted pyrazolyl group having 1 to 3 substituents on the ring, each independently selected from the set V of substituents;
(i13) a triazolyl group;
(i14) a substituted triazolyl group having 1 or 2 substituents on the ring, each independently selected from the set V of substituents;
(i21) a thiazolyl group;
(i22) a substituted thiazolyl group having 1 or 2 substituents on the ring, each independently selected from the set V of substituents;
(i23) an oxazolyl group;
(i24) a substituted oxazolyl group having 1 or 2 substituents on the ring, each independently selected from the set V of substituents;
(i25) a tetrahydrofuranyl (C₁-C₆) alkoxy group;
(i26) a substituted tetrahydrofuranyl (C₁-C₆) alkoxy group having 1 to 4 substituents on the ring, each independently selected from the set V of substituents;
(i27) a dioxolanyl (C₁-C₆) alkoxy group;
(i28) a substituted dioxolanyl (C₁-C₆) alkoxy group having 1 to 4 substituents on the ring, each independently selected from the set V of substituents;
(i29) an isoxazolinyl (C₁-C₆) alkoxy group;
(i30) a substituted isoxazolinyl (C₁-C₆) alkoxy group having 1 to 4 substituents on the ring, each independently selected from the set V of substituents;
(i31) a pyrazolyl (C₁-C₆) alkoxy group;
(i32) a substituted pyrazolyl (C₁-C₆) alkoxy group having 1 to 3 substituents on the ring, each independently selected from the set V of substituents;
(i33) a triazolyl (C₁-C₆) alkoxy group;
(i34) a substituted triazolyl (C₁-C₆) alkoxy group having 1 or 2 substituents on the ring, each independently selected from the set V of substituents;
(i41) a thiazolyl (C₁-C₆) alkoxy group;
(i42) a substituted thiazolyl (C₁-C₆) alkoxy group having 1 or 2 substituents on the ring, each independently selected from the set V of substituents;
(i43) an oxazolyl (C₁-C₆) alkoxy group; or
(i44) a substituted oxazolyl (C₁-C₆) alkoxy group having 1 or 2 substituents on the ring, each independently selected from the set V of substituents, and
the set V of substituents consists of
(j1) a halogen atom;
(j2) a cyano group;
(j3) a (C₁-C₆) alkyl group;
(j4) a (C₂-C₆) alkenyl group;
(j6) a (C₃-C₆) cycloalkyl group;
(j7) a halo (C₁-C₆) alkyl group;
(j8) a halo (C₂-C₆) alkenyl group;
(j10) a halo (C₃-C₆) cycloalkyl group;
(j11) a (C₁-C₆) alkoxy group;
(j12) a (C₁-C₆) alkylsulfanyl group;
(j13) a (C₁-C₆) alkylsulfinyl group;
(j14) a (C₁-C₆) alkylsulfonyl group;
(j15) a halo (C₁-C₆) alkoxy group;
(j16) a halo (C₁-C₆) alkylsulfanyl group;
(j20) a (C₁-C₆) alkylsulfanyl (C₁-C₆) alkyl group;
(j21) a (C₁-C₆) alkoxycarbonyl group;
(j22) a phenyl group;
(j23) a substituted phenyl group having 1 to 5 substituents on the ring, each independently selected from the group consisting of a halogen atom, a (C₁-C₆) alkyl, and a halo (C₁-C₆) alkyl;
(j24) a thienyl group; and
(j25) a substituted thienyl group having 1 to 3 substituents on the ring, each independently selected from the group consisting of a halogen atom, a (C₁-C₆) alkyl, and a halo (C₁-C₆) alkyl.

3. The compound or the salt thereof according to claim 1, wherein
R¹ is
(a1) a (C₁-C₆) alkyl group; or
(a3") an N(R^{2a})R^{2b} group
(wherein R^{2a} and R^{2b} represent
(b1) a hydrogen atom,
(b2) a (C₁-C₆) alkyl group,
(b7) a (C₁-C₇) alkylcarbonyl group, or
(b8) a (C₁-C₆) alkoxycarbonyl group, and
R^{2a} and R^{2b} may be the same or different),
Het is the formula (Het-1) or the formula (Het-2),
R³ is
(c1) a halogen atom;
(c3) a (C₁-C₆) alkyl group;
(c4) a (C₂-C₆) alkenyl group;
(c6) a (C₃-C₆) cycloalkyl group;
(c8) a halo (C₁-C₆) alkyl group;
(c13) a substituted (C₁-C₆) alkyl group having 1 to 3 substituents, each independently selected from a set S of substituents;
(c14) a substituted (C₃-C₆) cycloalkyl group having 1 to 3 substituents, each independently selected from a set T of substituents;
(c18) a (C₁-C₆) alkoxycarbonyl group;
(c19) a furanyl group;
(c23) a thienyl group;
(c25) a thiazolyl group;
(c26) a substituted thiazolyl group having 1 or 2 substituents on the ring, each independently selected from a set U of substituents;
(c27) a naphthyl group;
(c29) a phenyl group;
(c30) a substituted phenyl group having 1 to 5 substituents on the ring, each independently selected from the set U of substituents;
(c31) a pyridyl group;
(c32) a substituted pyridyl group having 1 to 4 substituents on the ring, each independently selected from the set U of substituents; or
(c40) a substituted phenyl (C₁-C₆) alkyl group having 1 to 5 substituents on the ring, each independently selected from the set U of substituents,
Y is a nitrogen atom or CR⁴ (wherein R⁴ represents a hydrogen atom),
R^{5a} is
(d2) a (C₁-C₆) alkyl group,
R^{5b} is
(e1) a hydrogen atom; or
(e3) a (C₁-C₆) alkyl group,
the set S of substituents is
(f3) a (C₁-C₆) alkoxy group,
the set T of substituents is
(g14) a phenyl group,
the set U of substituents consists of
(h1) a halogen atom;
(h5) a hydroxy group;
(h6) a hydroxy (C₁-C₆) alkyl group;
(h7) a (C₁-C₆) alkyl group;
(h11) a halo (C₁-C₆) alkyl group; and
(h15) a (C₁-C₆) alkoxy group,
A is
(i2) a substituted oxazolinyl group having 1 to 4 substituents on the ring, each independently selected from a set V of substituents;
(i4) a substituted imidazolinyl group having 1 to 4 substituents on the ring, each independently selected from the set V of substituents;
(i6) a substituted imidazolidinonyl group having 1 to 4 substituents on the ring, each independently selected from the set V of substituents;
(i8) a substituted triazolinonyl group having 1 or 2 substituents on the ring, each independently selected from the set V of substituents;
(i10) a substituted pyrazolinonyl group having 1 to 3 substituents on the ring, each independently selected from the set V of substituents;
(i11) a pyrazolyl group;
(i12) a substituted pyrazolyl group having 1 to 3 substituents on the ring, each independently selected from the set V of substituents;
(i13) a triazolyl group;
(i14) a substituted triazolyl group having 1 or 2 substituents on the ring, each independently selected from the set V of substituents;
(i22) a substituted thiazolyl group having 1 or 2 substituents on the ring, each independently selected from the set V of substituents;
(i26) a substituted tetrahydrofuranyl (C₁-C₆) alkoxy group having 1 to 4 substituents on the ring, each independently selected from the set V of substituents;
(i28) a substituted dioxolanyl (C₁-C₆) alkoxy group having 1 to 4 substituents on the ring, each independently selected from the set V of substituents;
(i30) a substituted isoxazolinyl (C₁-C₆) alkoxy group having 1 to 4 substituents on the ring, each independently selected from the set V of substituents;
(i31) a pyrazolyl (C₁-C₆) alkoxy group;
(i32) a substituted pyrazolyl (C₁-C₆) alkoxy group having 1 to 3 substituents on the ring, each independently selected from the set V of substituents;
(i34) a substituted triazolyl (C₁-C₆) alkoxy group having 1 or 2 substituents on the ring, each independently selected from the set V of substituents; or
(i42) a substituted thiazolyl (C₁-C₆) alkoxy group having 1 or 2 substituents on the ring, each independently selected from the set V of substituents, and
the set V of substituents consists of
(j1) a halogen atom;
(j3) a (C₁-C₆) alkyl group;
(j4) a (C₂-C₆) alkenyl group;
(j6) a (C₃-C₆) cycloalkyl group;
(j7) a halo (C₁-C₆) alkyl group;
(j11) a (C₁-C₆) alkoxy group;
(j12) a (C₁-C₆) alkylsulfanyl group;
(j20) a (C₁-C₆) alkylsulfanyl (C₁-C₆) alkyl group;
(j21) a (C₁-C₆) alkoxycarbonyl group;
(j23') a substituted phenyl group having 1 to 5 substituents on the ring, selected independently from a halogen atom; and
(j24) a thienyl group.

4. An agricultural or horticultural herbicide comprising the compound or the salt thereof according to any one of claims 1 to 3 as an active ingredient.

5. A method for using an agricultural or horticultural herbicide, comprising treating weeds, soil, paddy fields, or growing media with an effective amount of the agricultural or horticultural herbicide according to claim 4.

6. A method for controlling weeds, comprising treating weeds, soil, paddy fields, or growing media with an effective amount of the agricultural or horticultural herbicide according to claim 4.
